# EUROPÄISCHE PATENTSCHRIFT

(11) **EP 2 740 476 B1**
(45) Veröffentlichungstag und Bekanntmachung des Hinweises auf die Patenterteilung: **05.12.2018**
(21) Anmeldenummer: 14000496.1
(22) Anmeldetag: 27.07.2011
(51) Int. Cl.: A61K 31/438, C07D 471/10, A61P 25/04

(54) **Cis-Tetrahydro-Spiro(Cyclohexan-1,1'-Pyrido[3,4-b]Indol)-4-Amin-Derivate**
Cis-tetrahydro-spiro(cyclohexan-1,1'-pyrido[3,4-b]indol)-4-amine derivatives
Dérivés de cis-tetrahydro-spiro(cyclohexan-1,1'-pyrido[3,4-b]indol)-4-amine

(30) Priorität: 28.07.2010 EP 10007822
(43) Veröffentlichungstag der Anmeldung: 11.06.2014
(62) Teilanmeldung aus: 11737904.0
(73) Patentinhaber: Grünenthal GmbH, 52078 Aachen (DE)
(72) Erfinder: Linz, Klaus, 53359 Rheinbach (DE); Zemolka, Saskia, 52066 Aachen (DE); Nolte, Bert, 53902 Bad Münstereifel (DE); Schunk, Stefan, 52080 Aachen (DE); Schick, Hans, 13086 Berlin-Weißensee (DE)

(56) Entgegenhaltungen:
- WO-A1-2006/108565
- WO-A1-2007/124903
- WO-A1-2009/118173

## Beschreibung

Die Erfindung verwendet Verbindungen, welche auf das Nociceptin/ORL-1-Rezeptorsystem sowie auf das µ-Opioid-Rezeptorsystem wirken und sich insbesondere durch eine selektive Wirksamkeit bei der Behandlung von chronischem Schmerz (u.a. Entzündungsschmerz, viszeralem Schmerz, Tumorschmerz, bevorzugt neuropathischem Schmerz) auszeichnen, ohne gleichzeitig eine ausgeprägte Wirksamkeit bei akutem, nociceptivem Schmerz zu entfalten. Bei den erfindungsgemäß verwendeten Verbindungen handelt es sich um cis-Tetrahydro-spiro(cyclohexan-1,1'-pyrido[3,4-b]indol)-4-amin-Derivate.

Chronische Schmerzen können in zwei große Gruppen unterteilt werden. Der pathophysiologische Nociceptorschmerz wird nach Gewebetraumen durch die Erregung von intakten Nociceptoren hervorgerufen. Hierzu gehören insbesondere chronische Entzündungsschmerzen. Schmerzen hingegen, die durch eine mechanische, metabolische oder entzündliche Schädigung von Nerven selbst entstehen, werden als neuropathische Schmerzen bezeichnet. Die Behandlung des chronischen Schmerzes stellt eine große medizinische Herausforderung dar, da die am Markt befindlichen Arzneimittel zwar zum Teil im Akutschmerz hochwirksam sind, im chronischen und besonders im neuropathischen Schmerz jedoch in vielen Fällen zu keiner zufrieden stellenden Schmerzbehandlung führen.

Entzündungsvorgänge gehören zu den wichtigsten Mechanismen der Schmerzentstehung. Die typischen Entzündungsschmerzen werden durch Freisetzung von Bradykinin, Histamin und Prostaglandinen mit einer Ansäuerung des Gewebes und dem Druck des Exsudats auf die Nociceptoren ausgelöst. In der Folge treten häufig Sensibilisierungsphänomene im zentralen Nervensystem auf, die sich in der Zunahme der neuronalen Spontanaktivität und in stärkeren Reizantworten zentraler Neurone äußern (Coderre et al., Pain 1993, 52, 259-285). Diese Veränderungen im Antwortverhalten zentraler Neurone können zum Spontanschmerz und zur Hyperalgesie (gesteigerte Schmerzempfindung auf einen noxischen Reiz) beitragen, die für entzündetes Gewebe typisch sind (Yaksh et al., PNAS 1999, 96, 7680-7686).

Zur Behandlung von Entzündungsschmerzen haben sich insbesondere nicht-steroidale Antiphlogistika (NSAIDs) bewährt, die neben der analgetischen Wirkung auch eine antientzündliche Komponente haben (Dickensen, A., International Congress and Symposium Series - Royal Society of Medicine (2000), 246, 47-54). Ihre Anwendung bei der Langzeittherapie chronischer Schmerzen ist jedoch durch zum Teil erhebliche unerwünschte Wirkungen, wie gastroenterale Ulzera oder toxische Nierenschädigungen, limitiert. Bei starken bis sehr starken Entzündungsschmerzen (beispw. im Rahmen einer chronischen Pankreatitis) reduzieren NSAIDs den Schmerz möglicherweise nur leicht, führen jedoch aufgrund der erhöhten Blutungsgefahr zu einem zu hohen Risiko. Der nächste Schritt ist im Allgemeinen die Behandlung mit µ-Opioiden, wobei unter den betroffenen Personen die Abhängigkeit von Narkotika weit verbreitet ist (Vercauteren et al., Acta Anaesthesiologica Belgica 1994, 45, 99-105). Es besteht daher dringender Bedarf an Verbindungen, die im Entzündungsschmerz gut wirksam sind und ein vermindertes Abhängigkeitspotential besitzen.

Neuropathische Schmerzen entstehen, wenn periphere Nerven auf mechanische, metabolische oder entzündliche Weise geschädigt werden. Die dabei auftretenden Schmerzbilder sind vorwiegend durch das Auftreten von Spontanschmerz, Hyperalgesie und Allodynie (Schmerz wird bereits durch nicht-noxische Reize ausgelöst) gekennzeichnet. (vgl. Baron, Clin. J. Pain 2000;16 (2 Suppl), 12-20). Die Ursachen und Ausprägungen und daher auch die Behandlungserfordernisse von neuropathischem Schmerz sind vielfältig. Sie entstehen als Folge von Verletzungen oder Erkrankungen von Gehirn, Rückenmark oder peripheren Nerven. Ursachen können Operationen (z B. Phantomschmerz nach Amputation), Rückenmarksverletzungen, Schlaganfälle, Multiple Sklerose, Alkohol- oder Medikamentenmissbrauch bzw. weitere Giftstoffe, Krebserkrankungen aber auch Stoffwechselkrankheiten wie Diabetes, Gicht, Niereninsuffizienz oder Leberzirrhose, sowie Infektionserkrankungen (u.a. Herpes zoster, Pfeiffer-Drüsenfieber, Ehrlichiose, Typhus, Diphtherie, HIV, Lues oder Borreliose) sein. Das Schmerzerleben hat sehr unterschiedliche Zeichen und Symptome (z.B. Kribbeln, Brennen, einschießende, elektrisierende oder ausstrahlende Schmerzen), die sich in Anzahl und Intensität über die Zeit verändern können.

Zur pharmakologischen Basistherapie neuropathischer Schmerzen gehören trizyklische Antidepressiva und Antikonvulsiva, die als Monotherapie oder auch in Kombination mit Opioiden eingesetzt werden. Diese Medikamente erbringen meistens nur eine gewisse Schmerzerleichterung, während eine Schmerzfreiheit oftmals nicht erreicht wird. Die sich häufig einstellenden Nebenwirkungen stehen dabei Dosiserhöhungen der Arzneimittel zur Erzielung einer ausreichenden Schmerzlinderung häufig im Wege. Tatsächlich wird zur zufriedenstellenden Behandlung von neuropathischem Schmerz häufig eine höhere Dosierung eines µ-Opioids benötigt als zur Behandlung von Akutschmerz, wodurch die Nebenwirkungen eine noch größere Bedeutung erlangen. Daher ist neuropathischer Schmerz heute schwer zu behandeln. Er wird selbst durch hohe Dosen von Stufe-3 Opioiden nur teilweise gelindert (Saudi Pharm. J. 2002, 10 (3), 73-85).

Opioide, welche bei der Behandlung von neuropathischem Schmerz eingesetzt werden, wirken üblicherweise gleichzeitig auch gegen Akutschmerz. Eine Trennung der Behandlung von neuropathischem Schmerz einerseits und von Akutschmerz andererseits ist bisher nicht möglich. Je nach Dosierung der Opioide wird daher jegliches Schmerzempfinden des Patienten unterdrückt, was durchaus von Nachteil sein kann. So erfüllt Akutschmerz eine Schutzfunktion des Körpers, welche verloren geht, wenn das Empfinden von Akutschmerz beeinträchtigt oder unterdrückt ist. Es besteht also ein Bedarf an der Erhaltung des allgemeinen Schmerzempfindens bei gleichzeitiger Bekämpfung von neuropathischem Schmerz.
Spirocyclische Cyclohexan-Derivate mit Wirkung auf das Nociceptin/ORL-1- und das µ-opioide Rezeptorsystem sind im Stand der Technik bekannt. Diese Verbindungen zeichnen sich u.a. durch eine außerordentlich große strukturelle Variabilität aus und eignen sich u.a. zur Behandlung von Entzündungs- und Neuropathie-Schmerz. In diesem Zusammenhang kann beispielsweise vollumfänglich verwiesen werden auf WO 2004/043967, WO2005/063769, WO2005/066183 und WO2006/108565.
Es besteht ein Bedarf an Arzneimitteln, welche bei der Behandlung von chronischem, insbesondere neuropathischem, Schmerz wirksam sind und welche gleichzeitig das Empfinden von Akutschmerz in möglichst geringem Maße beeinflussen. Nach Möglichkeit sollten diese Arzneimittel eine so geringe Wirkstoffdosis enthalten, dass eine zufrieden stellende Schmerztherapie gewährleistet werden kann, ohne dass nicht tolerierbare Nebenwirkungen auftreten.
Der Erfindung liegt die Aufgabe zugrunde, neue Verbindungen zur Verfügung zu stellen, welche sich als Arzneistoffe eignen und Vorteile gegenüber dem Stand der Technik haben.
Diese Aufgabe wird durch den Gegenstand der Patentansprüche gelöst.

Die Erfindung betrifft Verbindungen der allgemeinen Formel (III), 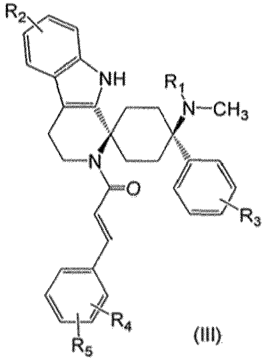 worin
R₁ -H oder CH₃ ist;
R₂ -H oder -Halogen ist;
R₃ -H oder -Halogen ist;
R₄ -H, -Halogen oder -OC₁₋₃-Alkyl ist und
R₅ -H, -Halogen oder -OC₁₋₃-Alkyl ist; und
wobei die Verbindung als Hydrochlorid-, Citrat- oder Hemicitratsalz vorliegt.

Es wurde überraschend gefunden, dass die Verbindungen gemäß der allgemeinen Formel (III) auf das Nociceptin/ORL-1- und das µ-opioide Rezeptorsystem wirken und bei der Behandlung von chronischem Schmerz, insbesondere von neuropathischem Schmerz, besonders wirksam sind, ohne gleichzeitig das Empfinden für Akutschmerz zu unterdrücken. Darüber hinaus zeigen diese Verbindungen überraschenderweise - wenn überhaupt - nur sehr geringe opioid-typische Nebenwirkungen im analgetisch effektiven Dosisbereich.

Die Verbindungen gemäß der allgemeinen Formel (III) zeigen eine sehr hohe analgetische Wirksamkeit bei der Behandlung chronischer Schmerzen, insbesondere neuropathischer Schmerzen, bevorzugt infolge von poly- oder mononeuropathischen Erkrankungen.

Es wurde überraschend gefunden, dass die Verbindungen bei Dosierungen, die zu einer praktisch vollständigen Aufhebung der neuropathischen Schmerzen in Mono- bzw. Polyneuropathie-Modellen führen, bei gesunden Tieren bzw. im gesunden Gewebe mononeuropathischer Tiere keinen Effekt auf die normale Nozizeption haben. Dies bedeutet, dass diese Verbindungen den pathologischen Zustand (Allodynie bzw. Hyperalgesie) aufheben, gleichzeitig jedoch - wenn überhaupt - allenfalls nur geringfügig das normale Schmerzempfinden beeinträchtigen. Die antinociceptive Wirkung der Verbindungen im Akutschmerz ist daher vernachlässigbar.

Die Verbindungen gemäß der allgemeinen Formel (III) ermöglichen somit eine selektive Wirksamkeit gegen chronischen Schmerz, bevorzugt gegen neuropathischen Schmerz, bevorzugter gegen mononeuropathischen/neuralgischen oder polyneuropathischen Schmerz, noch bevorzugter gegen Schmerz bei post-herpetischer Neuralgie oder bei diabetischer Polyneuropathie, vorzugsweise bei vernachlässigbarer antinociceptiver Wirksamkeit im Akutschmerz. Diese ungewöhnliche Eigenschaft der erfindungsgemäßen Verbindungen ist von grundsätzlicher Bedeutung für die gesamte Schmerztherapie.

Die Verbindungen gemäß der allgemeinen Formel (III) stellen eine Auswahl aus den in der WO2004/ 043967, WO2005/066183 und WO2006/108565 offenbarten Verbindungen dar. Es wurde überraschend gefunden, dass die erfindungsgemäßen Spiroamine, welche am Cyclohexanring in Bezug auf die beiden Stickstoffe die cis-Konfiguration haben (cis-Tetrahydro-spiro(cyclohexan-1,1'-pyrido[3,4-b]indol)-4-amin Derivate), Vorteile gegenüber den anderen Heterocyclen haben.

So zeigen die erfindungsgemäßen cis-Spiroamide im Unterschied zu den anderen Verbindungen gemäß WO2004/ 043967, WO2005/066183 und WO2006/108565 im Tiermodell eine hervorragende Wirkung gegen chronischen, bevorzugt neuropathischen Schmerz, bevorzugter Schmerz bei diabetischer Polyneuropathie, ohne bei der dazu erforderlichen therapeutischen Dosis eine signifikante Wirkung gegen Akutschmerz zu zeigen. Da zahlreiche Nebenwirkungen herkömmlicher Analgetika mit dem Wirkmechanismus gegen Akutschmerz in Verbindung gebracht werden, zeichen sich die erfindungsgemäßen spirocyclischen cis-substituierten Cyclohexanderivate durch ein besonders vorteilhaftes Nebenwirkungsprofil aus, insbesondere im Hinblick auf opioid-typische Nebenwirkungen.

Die Verbindungen gemäß der allgemeinen Formel (III) sind bevorzugt achiral; der Grundkörper der allgemeinen Formel (III) enthält kein Chiralitätselement (Zentrum, Achse oder Ebene).

Bei den Verbindungen gemäß der allgemeinen Formel (III) handelt es sich in Bezug auf das spiro-Ringsystem um Isomere, bei denen das Substitutionsmuster am spiro-Cyclohexanringsystem (nicht am Indol) auch mit cis/trans, Z/E oder syn/anti bezeichnet werden kann. "Cis-trans-Isomere" sind eine Untergruppe der Stereoisomere (Konfigurationsisomere).

Bei den Verbindungen gemäß der allgemeinen Formel (III) stehen die beiden Stickstoffatome des Spiroamins jeweils syn bzw. cis bzw. Z zueinander: 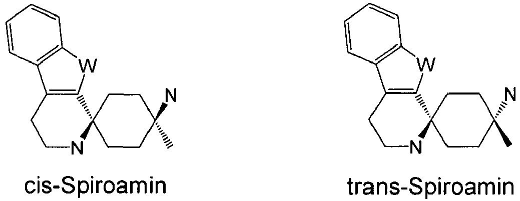 In einer bevorzugten Ausführungsform der Erfindung beträgt der Überschuss des so bezeichneten cis-Isomers wenigstens 50 %de, bevorzugter wenigstens 75 %de, noch bevorzugter wenigstens 90%de, am bevorzugtesten wenigstens 95%de und insbesondere wenigstens 99%de.
Geeignete Methoden zur Trennung der Isomere (Diastereomere) sind dem Fachmann bekannt. Als Beispiele können Säulenchromatographie, präparative HPLC und Kristallisationsverfahren genannt werden. Auch gezielte Syntheseverfahren, bei denen das eine Isomer im Überschuss gebildet wird, sind dem Fachmann grundsätzlich bekannt.
Die Vorteile des cis-Isomers sind ferner insofern besonders überraschend, als dass bei den strukturell verwandten Spiroethern üblicherweise nicht das cis-Isomer, sondern das trans-Isomer aus pharmakologischer Sicht vorteilhafte Eigenschaften aufweist (welche allerdings mitunter anders geartet sind als die Vorteile der erfindungsgemäßen cis-Spiroamine):

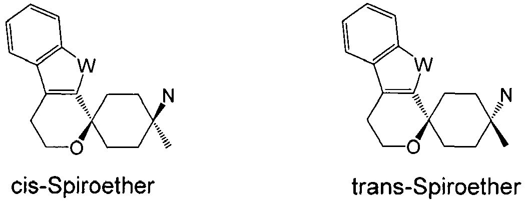

Zum Zwecke der Beschreibung bedeutet "-Halogen" vorzugsweise -F, -Cl, -Br oder -I, bevorzugter -F oder -Cl, insbesondere -F.

Zum Zwecke der Beschreibung ist "C₁₋₃-Alkyl" jeweils unabhängig linear oder verzweigt, gesättigt oder einfach oder mehrfach ungesättigt. So umfasst "C₁₋₃-Alkyl" acyclische gesättigte oder ungesättigte Kohlenwasserstoffreste, die verzweigt oder geradkettig sein können, d.h. C₁₋₃-Alkanyle, C₁₋₃-Alkenyle und C₁₋₃-Alkinyle.

Bevorzugt sind Verhindungen der allgemeinen Formel (III): 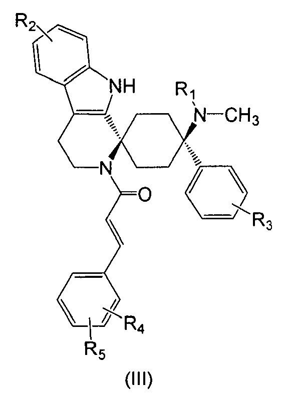 wobei die Verbindung als Hydrochlorid-, Citrat- oder Hemicitratsalz vorliegt.

Bevorzugt ist R₂ -H und/oder R₃ -F.

Bevorzugt sind R₄ und R₅ entweder beide -H oder beide -OCH₃.

In einer besonder bevorzugten Ausführungsform betrifft die Erfindung die Verbindung der allgemeinen Formel (VI) 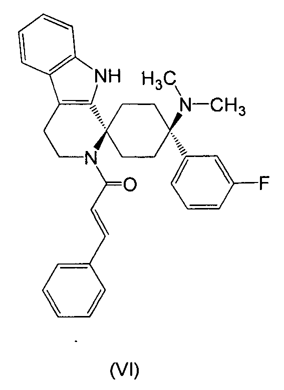 wobei die Verbindung als Hydrochlorid-, Citrat- oder Hemicitratsalz vorliegt.

Die freie Base der Verbindung der allgemeinen Formel (VI) kann systematisch bezeichnet werden als (E)-2',3',4',9'-Tetrahydro-N,N-dimethyl-4-(3-fluorphenyl)-2'-(2-phenylvinyl)carbonyl-spiro[cyclohexan-1,1'(1'H)-pyrido[3,4-b]indol]-4-amin (Cis-Diastereomer) oder auch als (E)-1-((1s,4s)-4-(dimethylamino)-4-(3-fluorophenyl)-3'-4'-dihydrospiro[cyclohexan 1,1'pyrido-[3,4-b]indol]-2'(9'H)-yl)-3-phenylprop-2-en-1-on. Diese Verbindung liegt als Hydrochlorid, als Citrat oder als Hemicitrat vor.

Erfindungsgemäß werden insbesondere die Verbindungen ausgewählt aus der Gruppe bestehend aus

| | |
|---|---|
| (E)-2',3',4',9'-tetrahydro-N,N-dimethyl-4-phenyl-2'-(2-phenylvinyl)carbonyl-spiro[cyclohexan-1,1'(1'H)-pyrido[3,4-b]indol]-4-amin (Cis-Diastereomer) oder (E)-1-((1s,4s)-4-(Dimethylamino)-4-phenyl-3',4'-dihydrospiro[cyclohexan-1,1'-pyrido[3,4-b]indol]-2'(9'H)-yl)-3-phenylprop-2-en-1-on | AMD-1^{cis} |
| (E)-2',3',4',9'-Tetrahydro-N,N-dimethyl-4-(3-fluorphenyl)-2'-(2-phenylvinyl)-carbonyl-spiro[cyclohexan-1,1'(1'H)-pyrido[3,4-b]indol]-4-amin (Cis-Diastereomer) oder (E)-1-((1s,4s)-4-(Dimethylamino)-4-(3-fluorophenyl)-3',4'-dihydro-spiro[cyclohexan-1,1'-pyrido[3.4-b]indol]-2'(9'H)-yl)-3-phenylprop-2-en-1-on | AMD-5^{cis} |
| | AMD-6^{cis} |
| (E)-2',3',4',9'-Tetrahydro-N,N-dimethyl-6'-fluor-4-(3-fluorphenyl)-2'-(2-phenyl-vinyl)carbonyl-spiro[cyclohexan-1,1'(1'H)-pyrido[3,4-b]indol]-4-amin (Cis-Diastereomer) oder (E)-1-((1s,4s)-4-(Dimethylamino)-6'-fluoro-4-(3-fluorophenyl)-3',4'-3',4'-dihydrospiro[cyclohexan-1,1'-pyrido[3,4-b]indol]-2'(9'H)-yl)-3-phenylprop-2-en-1-on | AMD-8^{cis} |
| (E)-2',3',4',9'-Tetrahydro-N,N-dimethyl-6'-fluor-4-phenyl-2'-(2-phenylvinyl)carbonyl-bonyl-spiro[cyclohexan-1,1'(1'H)-pyrido[3,4-b]indol]-4-amin (Cis-Diastereomer) oder (E)-1-((1s,4s)-4-(Dimethylamino)-6'-fluoro-4-phenyl-3',4'-dihydrospiro-[cyclohexan-1,1'-pyrido[3,4-b]indol]-2'(9'H)-yl)-3-phenylprop-2-en-1-on | AMD-10^{cis} |
| (E)-2',3',4',9'-Tetrahydro-N,N-dimethyl-4-(4-fluorphenyl)-2'-(2-phenylvinyl)carbonyl-spiro[cyclohexan-1,1'(1'H)-pyrido[3,4-b]indol]-4-amin (Cis-Diastereomer) oder (E)-1-((1s,4s)-4-(Dimethylamino)-4-(4-fluorophenyl)-3',4'-dihydrospiro-[cyclohexan-1,1'-pyrido[3,4-b]indol]-2'(9'H)-yl)-3-phenylprop-2-en-1-on | AMD-12^{cis} |

jeweils in der Form als Hydrochlorid-, Citrat- oder Hemicitratsalz.

In einer bevorzugten Ausführungsform der Erfindung kommen die Verbindungen gemäß der allgemeinen Formel (III) zweimal täglich, einmal täglich oder seltener zur Anwendung, besonders bevorzugt höchstens einmal täglich.

In einer weiteren Ausführungsform kommen Verbindungen gemäß der allgemeinen Formel (III) zur Anwendung bei der Behandlung von chronischem Schmerz. Bevorzugt ist der chronische Schmerz ausgewählt aus der Gruppe bestehend aus Entzündungsschmerz, viszeralem Schmerz, Tumorschmerz und neuropathischem Schmerz. Der neuropathische Schmerz kann mononeuropathischen/neuralgischen oder polyneuropathischen Ursprungs sein.

In einer weiteren bevorzugten Ausführungsform kommen die Verbindungen gemäß der allgemeinen Formel (III) zur Anwendung bei der Behandlung von Schmerz bei diabetischer Polyneuropathie.

In einer weiteren bevorzugten Ausführungsform kommen die Verbindungen gemäß der allgemeinen Formel (III) zur Anwendung bei der Behandlung von Schmerz infolge post-herpetischer Neuralgie.

Die Verbindungen gemäß der allgemeinen Formel (III) eignen sich zur Verwendung zur Behandlung von neuropathischem Schmerz, bevorzugt von mononeuropathischem/neuralgischem oder polyneuropathischem Schmerz. Bevorzugt ist der Schmerz peripherer polyneuropathischer Schmerz oder zentraler polyneuropathischer Schmerz.
Bevorzugt ist die Polyneuropathie bzw. der polyneuropathische Schmerz akut (bis vier Wochen), subakut (vier bis acht Wochen) oder chronisch (mehr als acht Wochen).

Bevorzugt ist bei der Polyneuropathie das motorische, sensible, autonome, sensomotorische oder zentrale Nervensystem betroffen. Vorzugsweise sind die Symptome symmetrisch oder asymmetrisch verteilt. Der Schmerz kann leicht, mäßig, mittelstark, stark oder sehr stark sein. Als Maß kann die neuropathische Schmerzskala (NPS) dienen (vgl. B.S. Galer et al., Neurology 1997, 48, 332-8).

Beispiele für Ursachen von peripherem neuropathischem Schmerz sind diabetische Polyneuropathie, postherpetische Neuralgie, Radioculopathie, posttraumatische Neuralgie, durch chemische Substanzen, z.B. durch Chemotherapie induzierte Polyneuropathie, Phantomschmerzen an den Gliedmaßen, komplexes regionales Syndrom, HIV-induzierte sensorische Polyneuropathie und alkoholische Polyneuropathie. Beispiele für Ursachen von zentralem polyneuropathischem Schmerz sind kompressive Myelopathie infolge von verengter Kanalstenose, posstraumatischer Spinalschmerz, Schlaganfallschmerz, postischemische Myelopathie, strahleninduzierte Myelopathie, durch Multiple Sklerose induzierte Myelopahthie und HIV-induzierte Myelopathie.

In einer bevorzugten Ausführungsform ist die den neuropathischen Schmerz verursachende Neuropathie assoziiert mit einer Erkrankung ausgewählt aus der Gruppe bestehend aus Diabetes mellitus, Vaskulitis, Urämie, Hypotyrodismus, Alkoholmissbrauch, postherpetischer Neuralgie, idiopathischer Neuropathie, chronisch entzündlicher demyelinisierender Neuropathie, multifokaler motorischer Neuropathie, hereditärer Polyneuropathie, Guillain-Barré-Syndrom, Intoxikation [z.B. durch Alkohol, Schwermetalle {insbesondere Pb, Hg, As}, Hydrocarbone, infolge einer Chemotherapie mit Cytostatika], Porphyrie, Infektionskrankheiten, Krebserkrankungen [z.B. Myelom, Amyloid, Leukämie, Lymphome], perniziöse Anämie, Vitamin-E-Mangel, Morbus Refsum, Bassen-Kornzweig-Syndrom, Morbus-Fabry, Vaskulitis und Amyloidose. Diabetische Polyneuropathie und postherpetische Neuralgie sind besonders bevorzugt. Handelt es sich um eine Infektionskrankheit, so ist diese vorzugsweise ausgewählt aus der Gruppe bestehend aus Mononukleose, Ehrlichiose, Typhus, Diphterie, Lepra, HIV, Lues und Borreliose.

Bevorzugt handelt es sich bei dem polyneuropathischen Schmerz um Schmerz, welcher als Ursache eine Polyneuropathie im Sinne der ICD-10 hat (Internationale statistische Klassifikation der Krankheiten und verwandter Gesundheitsprobleme, WHO Ausgabe, vorzugsweise Stand 2008).

Ein weiterer Gegenstand der Erfindung betrifft die erfindungsgemäßen Verbindungen zur Anwendung bei der Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmissbrauch und/- oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Ein weiterer Gegenstand der Erfindung betrifft die erfindungsgemäßen Verbindungen zur Anwendung bei der Behandlung, insbesondere in einer der vorgenannten Indikationen, eines nichthumanen Säugetieres oder Menschen, das oder der eine Behandlung von chronischen Schmerzen, bevorzugt neuropathischen Schmerzen, bevorzugter Schmerzen bei diabetischer Polyneuropathie oder postherpetischer Neuralgie benötigt, durch Verabreichung einer individuell therapeutisch erforderlichen täglichen Dosis einer erfindungsgemäßen Verbindung, oder einer erfindungsgemäßen Darreichungsform, wobei vorzugsweise gleichzeitig keine signifikante Unterdrückung des Empfindens von akutem Nociceptorschmerz erfolgt und/oder keine signifikanten opioid-typischen Nebenwirkungen auftreten, insbesondere im wesentlichen keine Atemdepression und/oder keine Obstipation und/oder keine Harnretention und/oder keine Übelkeit und/oder kein Erbrechen und/oder keine Hypotonie und/oder keine Bradykardie und/oder keine Sucht und/oder keine Abhängigkeit und/oder keine Euphorisierung und/oder keine Depression und/oder keine Sedation und/oder kein Schwindel.

Ein weiterer Gegenstand der Erfindung betrifft die erfindungsgemäßen Verbindungen zur Anwendung bei der Behandlung, insbesondere in einer der vorgenannten Indikationen, eines nichthumanen Säugetieres oder Menschen, das oder der eine Behandlung von chronischen Schmerzen, bevorzugt neuropathischen Schmerzen, bevorzugter Schmerzen bei diabetischer Polyneuropathie oder postherpetischer Neuralgie benötigt, durch Verabreichung einer täglichen Dosis X einer erfindungsgemäßen Verbindung, oder einer erfindungsgemäßen Darreichungsform, wobei vorzugsweise keine signifikante gleichzeitige Unterdrückung des Empfindens von akutem Nociceptorschmerz erfolgt und/oder keine signifikanten opioid-typischen Nebenwirkungen auftreten, insbesonere im wesentlichen keine Atemdepression und/oder keine Obstipation und/oder keine Harnretention und/oder keine Übelkeit und/oder kein Erbrechen und/oder keine Hypotonie und/oder keine Bradykardie und/oder keine Sucht und/oder keine Abhängigkeit und/oder keine Euphorisierung und/oder keine Depression und/oder keine Sedation und/oder kein Schwindel; wobei die tägliche Dosis X ausgewählt ist aus der Gruppe bestehend aus 0,001, 0,002, 0,003, 0,004, 0,005, 0,006, 0,007, 0,008, 0,009, 0,01, 0,02, 0,03, 0,04, 0,05, 0,06, 0,07, 0,08, 0,09, 0,1, 0,2, 0,3, 0,4, 0,5, 0,6, 0,7, 0,8, 0,9, 1, 2, 3, 4, 5, 6, 7, 8, 9, 10 mg.

Ein weiterer Gegenstand der Erfindung betrifft die erfindungsgemäßen Verbindungen der allgemeinen Formel (III) mit Affinität zum µ-Opioid-Rezeptor und zum ORL-1-Rezeptor, zur Anwendung
- bei der Behandlung von neuropathischem Schmerz, bevorzugt in der Ratte, bevorzugter als mononeuropathischer Schmerz im Modell nach Chung, signifikant wirksam und dabei durch eine halbmaximal wirksame Dosierung ED₅₀ⁿ gekennzeichnet sind, und
- bei der Behandlung von akutem Schmerz, bevorzugt in der Ratte, bevorzugter im *Tail-Flick-*Test*,* in einer Dosierung, welche um den Faktor 5 höher als ED₅₀ⁿ ist, im wesentlichen nicht signifikant wirksam sind.

Somit weisen die Verbindungen gemäß der allgemeinen Formel (III) bei Verabreichung in dieser halbmaximal wirksamen Dosierung ED₅₀ⁿ, welche im Hinblick auf die Wirksamkeit der Verbindung gegen neuropathischen Schmerz definiert ist, und selbst in einer Dosierung, welche um den Faktor 5 höher als ED₅₀ⁿ ist, - wenn überhaupt - allenfalls eine vernachlässigbare antinociceptive Wirkung im Akutschmerz auf, bevorzugt in der Ratte, bevorzugter im Tail-Flick-Test.
In einer bevorzugten Ausführungsform handelt es sich bei dem neuropathischen Schmerz um mononeuropathischen oder neuralgischen Schmerz, vorzugsweise um Schmerz infolge einer post-herpetischen Neuralgie. In einer anderen bevorzugten Ausführungsform handelt es sich bei dem Schmerz um polyneuropathischen Schmerz, vorzugsweise um Schmerz bei diabetischer Polyneuropathie.

Bevorzugt werden die Verbindungen gemäß der allgemeinen Formel (III) in einer Dosierung verwendet, welche um Faktor 10, 20, 30, 40 oder 50, bevorzugter um Faktor 75, 100, 125, 150 oder 175, noch bevorzugter um Faktor 200, 300, 400 oder 500, am bevorzugtesten um Faktor 600, 700, 800 oder 900, und insbesondere um Faktor 1000 höher als die halbmaximal wirksame Dosierung ED₅₀ⁿ ist, bei der Behandlung von akutem bzw. nociceptivem Schmerz im wesentlichen nicht signifikant wirksam.

Die halbmaximal wirksame Dosierung ED₅₀ⁿ ist dem Fachmann bekannt. Sie ist vorzugsweise definiert als diejenige Dosierung, bei der im Hinblick auf die Behandlung von neuropathischem Schmerz 50% der maximalen therapeutischen Wirkung erzielt wird. Entsprechend kann eine halbmaximal wirksame Dosierung ED₅₀^{a} als diejenige Dosierung definiert werden, bei der im Hinblick auf die Behandlung von akutem Schmerz 50% der maximalen therapeutischen Wirkung erzielt wird. Die erfindungsgemäßen Verbindungen sind allerdings über ED₅₀ⁿ definiert, nicht jedoch über ED₅₀^{a}.

Geeignete Methoden zur Untersuchung der Wirksamkeit eines Wirkstoffs bei der Behandlung von neuropathischem Schmerz und die Ermittlung der halbmaximal wirksamen Dosierung ED₅₀ⁿ bei der Behandlung von neuropathischem Schmerz sind dem Fachmann bekannt. Dies gilt ebenso für die Untersuchung der Wirksamkeit eines Wirkstoffs gegen Akutschmerz.

Beispielsweise kann die Bestimmung im Tiermodell erfolgen (z.B. Maus oder Ratte), wobei
- mononeuropathischer Schmerz gemäß Chung (S.H. Kim, J.M. Chung, Pain. 1992, 50(3), 355-63) oder Bennett (G.J. Bennett, Y.K. Xie, Pain. 1988, 33(1), 87-107),
- Schmerz bei diabetischer Polyneuropathie nach Streptozotocin- (STZ-) induzierter Diabetes (E.K. Joseph, J.D. Levine, Neuroscience. 2003;120(4):907-13) und
- Akutschmerz im sog. *Tail-Flick*-Test (D'Amour and Smith, J. Pharm. Exp. Ther. 72, 1941, 74-9)
untersucht werden können.

Bevorzugt erfolgt die Bestimmung im Tiermodell, und zwar im Hinblick auf die Wirksamkeit gegen neuropathischen Schmerz als Wirksamkeit gegen mononeuropathischen Schmerz in der Ratte im Modell nach Chung und im Hinblick auf die Wirksamkeit gegen Akutschmerz in der Ratte im *Tail-Flick* Test, vorzugsweise jeweils wie im experimentellen Teil beschrieben.

Somit weisen die erfindungsgemäßen Verbindungen bevorzugt eine Affinität zum µ-Opioid-Rezeptor und zum ORL-1-Rezeptorauf, welche in der Ratte
- bei der Behandlung von mononeuropathischem Schmerz im Modell nach Chung signifikant wirksam und dabei durch eine halbmaximal wirksame Dosierung ED₅₀ⁿ gekennzeichnet sind, und
- bei der Behandlung von akutem Schmerz im *Tail-Flick*-Test in einer Dosierung, welche um den Faktor 5 höher als ED₅₀ⁿ ist, nicht signifikant wirksam sind.

Die Auswertung der experimentellen Befunde im Hinblick auf statistisch signifikante Unterschiede zwischen den jeweiligen Dosisgruppen und der Vehikel-Kontrollgruppen erfolgt bevorzugt mittels Varianzanalyse mit Messwiederholung (repeated measures ANOVA) sowie einer post hoc Analyse nach Bonferroni, bevorzugt wie im experimentellen Teil beschrieben. Dabei wird das Signifikanzniveau auf p < 0.05 gesetzt. Die Gruppengrößen betragen üblicherweise n=10.
Grundsätzlich kann die vergleichende Bestimmung analgetischen Wirksamkeit gegen neuropathischen Schmerz und akuten, nociceptiven Schmerz auch beim Menschen erfolgen, was jedoch u.a. aus ethischen Erwägungen heraus weniger bevorzugt ist. Die Untersuchung der Wirksamkeit gegen neuropathischen Schmerz, d.h. bei Patienten, die an neuropathischen Schmerzen leiden, kann dann erfolgen gemäß Hansson P, Backonja M, Bouhassira D. (2007). Usefulness and limitations of quantitative sensory testing: clinical and research application in neuropathic pain states. Pain. 129(3): 256-9. Die Untersuchung der Wirksamkeit gegen Akutschmerz kann dann erfolgen gemäß Posner J, Telekes A, Crowley D, Phillipson R, Peck AW. (1985). Effects of an opiate on cold-induced pain and the CNS in healthy volunteers. Pain. 23(1):73-82.
Es wurde überraschend gefunden, dass sich die Verbindungen gemäß der allgemeinen Formel (III) durch ein im Vergleich zu gebräuchlichen Stufe-3 Opioiden sehr günstiges Nebenwirkungsprofil auszeichnen. So wurde selbst bei Verabreichung therapeutisch wirksamer Dosierungen, wie sie insbesondere zur Behandlung von neuropathischem Schmerz erforderlich sind, keine oder allenfalls nur geringfügig ausgeprägte opioid-typische Nebenwirkungen beobachtet, wie z.B. Atemdepression, Obstipation, Harnretention, Übelkeit, Erbrechen, Hypotonie, Bradykardie, Sucht, Abhängigkeit, Euphorisierung, Depression, Sedation und Schwindel. Bisher wurde das stark verminderte Auftreten der opioid-typischen Nebenwirkungen Atemdepression, Obstipation, Hypotonie, Bradykardie, Störung der motorischen Koordinationsfähigkeit (als Maß für zentral-nervöse Nebenwirkungen), körperliche sowie psychische Abhängigkeit in Tiermodellen experimentell gezeigt.
In einer bevorzugten Ausführungsform der erfindungsgemäßen Verwendung zeigen die Verbindungen gemäß der allgemeinen Formel (III) bei Verabreichung in der halbmaximal wirksamen Dosierung ED₅₀ⁿ, welche im Hinblick auf die Wirksamkeit der Verbindung gegen neuropathischen Schmerz definiert ist, und bevorzugt selbst in einer Dosierung, welche um den Faktor 5 höher als ED₅₀ⁿ ist, keine signifikante Atemdepression als Nebenwirkung, bevorzugt in der Ratte, bevorzugter im Blutgasanalyse-Modell. Bevorzugt weisen die Verbindungen gemäß der allgemeinen Formel (III) selbst in der Verwendung mit einer Dosierung, welche um Faktor 10, 20, 30, 40 oder 50, bevorzugter um Faktor 75, 100, 125, 150 oder 175, noch bevorzugter um Faktor 200 höher als die halbmaximal wirksame Dosierung ED₅₀ⁿ ist, keine signifikante Atemdepression als Nebenwirkung auf.
Geeignete Methoden zur Untersuchung einer Wirkstoff-induzierten Atemdepression sind dem Fachmann bekannt. Bevorzugt erfolgt die Untersuchung in einem Blutgasanalysemodell bei der Ratte als Änderung der arteriellen O₂ und CO₂ Partialdrücke. Die Auswertung der experimentellen Befunde im Hinblick auf statistisch signifikante Unterschiede zwischen den jeweiligen Dosisgruppen und der Vehikel-Kontrollgruppen erfolgt dabei bevorzugt mittels Ein-Faktor Varianzanalyse (one-way ANOVA) sowie einer post hoc Analyse nach Dunnett, bevorzugt wie im experimentellen Teil beschrieben. Dabei wird das Signifikanzniveau auf p < 0.05 festgelegt. Die Gruppengrößen betragen üblicherweise n=6. Bezüglich weiterer Einzelheiten dieses Tiermodells wird ferner auf den experimentellen Teil verwiesen.
In einer bevorzugten Ausführungsform der erfindungsgemäßem Verwendung zeigen die Verbindungen gemäß der allgemeinen Formel (III) bei Verabreichung in der halbmaximal wirksamen Dosierung ED₅₀ⁿ, welche im Hinblick auf die Wirksamkeit der Verbindung gegen neuropathischen Schmerz definiert ist, und bevorzugt selbst in einer Dosierung, welche um den Faktor 5 höher als ED₅₀ⁿ ist, keine signifikante Obstipation als Nebenwirkung, bevorzugt in der Maus, bevorzugter im Kohlepassage-Test. Bevorzugt weisen Verbindungen selbst in einer Dosierung, welche um Faktor 10, 20, 30, 40 oder 50, bevorzugter um Faktor 75, 100, 125, 150 oder 175, noch bevorzugter um Faktor 200, 300, 400 oder 500, am bevorzugtesten um Faktor 600 höher als die halbmaximal wirksame Dosierung ED₅₀ⁿ ist, keine signifikante Obstipation als Nebenwirkung auf.
Geeignete Methoden zur Untersuchung einer Wirkstoff-induzierten Obstipation sind dem Fachmann bekannt. Bevorzugt erfolgt die Untersuchung in einem Kohlepassage-Modell bei der Maus als Änderung der gastrointestinalen Transitgeschwindigkeit. Die Auswertung der experimentellen Befunde im Hinblick auf statistisch signifikante Unterschiede zwischen den jeweiligen Dosisgruppen und der Vehikel-Kontrollgruppen erfolgt dabei bevorzugt mittels Ein-Faktor Varianzanalyse (one-way ANOVA) sowie einer post hoc Analyse nach Dunnett, bevorzugt wie im experimentellen Teil beschrieben. Dabei wird das Signifikanzniveau auf p < 0.05 festgelegt. Die Gruppengrößen betragen üblicherweise n=10. Bezüglich weiterer Einzelheiten dieses Tiermodells wird ferner auf den experimentellen Teil verwiesen.
In einer bevorzugten Ausführungsform der erfindungsgemäßen Verwendung zeigen die Verbindungen gemäß der allgemeinen Formel (III) bei Verabreichung in der halbmaximal wirksamen Dosierung ED₅₀ⁿ, welche im Hinblick auf die Wirksamkeit der Verbindung gegen neuropathischen Schmerz definiert ist, und bevorzugt selbst in einer Dosierung, welche um den Faktor 5 höher als ED₅₀ⁿ ist, keine signifikante Hypotonie als Nebenwirkung, bevorzugt im wachen Kaninchen, bevorzugter im Kreislaufmodell an wachen, telemetrierten Kaninchen. Bevorzugt weisen die Verbindungen selbst in einer Dosierung, welche um Faktor 10, 20, 30, 40 oder 50, bevorzugter um Faktor 75, 100, 125, 150 oder 175, noch bevorzugter um Faktor 200 höher als die halbmaximal wirksame Dosierung ED₅₀ⁿ ist, keine signifikante Hypotonie als Nebenwirkung auf.
Geeignete Methoden zur Untersuchung einer Wirkstoff-induzierten Hypotonie sind dem Fachmann bekannt. Bevorzugt erfolgt die Untersuchung in einem Kreislaufmodell beim wachen, telemetrierten Kaninchen als Änderung des arteriellen Blutdrucks (systolischer, diastolischer und mittlerer Wert). Die Auswertung der experimentellen Befunde im Hinblick auf statistisch signifikante Unterschiede zwischen den jeweiligen Dosisgruppen und der Vehikel-Kontrollgruppen erfolgt dabei bevorzugt mittels Ein-Faktor Varianzanalyse (one-way ANOVA) sowie einer post hoc Analyse nach Dunnett, bevorzugt wie im experimentellen Teil beschrieben. Dabei wird das Signifikanzniveau auf p < 0.05 festgelegt. Die Gruppengrößen betragen üblicherweise n=6. Bezüglich weiterer Einzelheiten dieses Tiermodells wird ferner auf den experimentellen Teil verwiesen.
In einer bevorzugten Ausführungsform der erfindungsgemäßen Verwendung zeigen die Verbindungen gemäß der allgemeinen Formel (III) bei Verabreichung in der halbmaximal wirksamen Dosierung ED₅₀ⁿ, welche im Hinblick auf die Wirksamkeit der Verbindung gegen neuropathischen Schmerz definiert ist, und bevorzugt selbst in einer Dosierung, welche um den Faktor 5 höher als ED₅₀ⁿ ist, keine signifikante Bradykardie als Nebenwirkung, bevorzugt im wachen Kaninchen, bevorzugter im Kreislaufmodell an wachen, telemetrierten Kaninchen. Bevorzugt weisen die Verbindungen selbst in einer Dosierung, welche um Faktor 10, 20, 30, 40 oder 50, bevorzugter um Faktor 75, 100, 125, 150 oder 175, noch bevorzugter um Faktor 200 höher als die halbmaximal wirksame Dosierung ED₅₀ⁿ ist, keine signifikante Bradykardie als Nebenwirkung auf.
Geeignete Methoden zur Untersuchung einer Wirkstoff-induzierten Bradykakrdie sind dem Fachmann bekannt. Bevorzugt erfolgt die Untersuchung in einem Kreislaufmodell beim wachen, telemetrierten Kaninchen als Änderung der Herzfrequenz. Die Auswertung der experimentellen Befunde im Hinblick auf statistisch signifikante Unterschiede zwischen den jeweiligen Dosisgruppen und der Vehikel-Kontrollgruppen erfolgt dabei bevorzugt mittels Ein-Faktor Varianzanalyse (one-way ANOVA) sowie einer post hoc Analyse nach Dunnett, bevorzugt wie im experimentellen Teil beschrieben. Dabei wird das Signifikanzniveau auf p < 0.05 festgelegt. Die Gruppengrößen betragen üblicherweise n=6. Bezüglich weiterer Einzelheiten dieses Tiermodells wird ferner auf den experimentellen Teil verwiesen.
In einer bevorzugten Ausführungsform der erfindungsgemäßen Verwendung zeigen die Verbindungen gemäß der allgmeinen Formel (III) bei Verabreichung in der halbmaximal wirksamen Dosierung ED₅₀ⁿ, welche im Hinblick auf die Wirksamkeit der Verbindung gegen neuropathischen Schmerz definiert ist, und bevorzugt selbst in einer Dosierung, welche um den Faktor 5 höher als ED₅₀ⁿ ist, keine signifikante Störung der motorischen Koordinationsfähigkeit (als Maß für zentralnervöse Nebenwirkungen) als Nebenwirkung, bevorzugt in der Maus, bevorzugter im RotaRod-Test. Bevorzugt weisen die Verbindungen selbst in einer Dosierung, welche um Faktor 10, 20, 30, 40 oder 50, bevorzugter um Faktor 75, 100, 125, 150 oder 175, noch bevorzugter um Faktor 200, 300, 400 oder 500, am bevorzugtesten um Faktor 600, 700, 800 oder 900, und insbesondere um Faktor 1000 höher als die halbmaximal wirksame Dosierung ED₅₀ⁿ ist, keine signifikante Störung der motorischen Koordinationsfähigkeit (als Maß für zentralnervöse Nebenwirkungen) als Nebenwirkung auf.
Geeignete Methoden zur Untersuchung einer Wirkstoff-induzierten Störung der motorischen Koordinationsfähigkeit sind dem Fachmann bekannt. Bevorzugt erfolgt die Untersuchung in einem RotaRod-Modell an der Maus (analog zu Kuribara H., Higuchi Y., Tadokoro S. (1977), Effects of central depressants on Rota-Rod and traction performance in mice. Japan. J. Pharmacol. 27, 117-126.) als Änderung der Fähigkeit auf einem rotierenden Stab zu laufen. Die Auswertung der experimentellen Befunde im Hinblick auf statistisch signifikante Unterschiede zwischen den jeweiligen Dosisgruppen und der Vehikel-Kontrollgruppen erfolgt dabei bevorzugt mittels Ein-Faktor Varianzanalyse (one-way ANOVA) sowie einer post hoc Analyse nach Dunnett, bevorzugt wie im experimentellen Teil beschrieben. Dabei wird das Signifikanzniveau auf p < 0.05 festgelegt. Die Gruppengrößen betragen üblicherweise n=10. Bezüglich weiterer Einzelheiten dieses Tiermodells wird ferner auf den experimentellen Teil verwiesen.
In einer bevorzugten Ausführungsform des erfindungsgemäßen Verfahrens zeigen die Verbindungen gemäß der allgemeinen Formel (III) bei Verabreichung in der halbmaximal wirksamen Dosierung ED₅₀ⁿ, welche im Hinblick auf die Wirksamkeit der Verbindung gegen neuropathischen Schmerz definiert ist, und bevorzugt selbst in einer Dosierung, welche um den Faktor 5 höher als ED₅₀ⁿ ist, keine signifikante körperliche Abhängigkeit bzw. Entzugssymptome als Nebenwirkung, bevorzugt in der Maus, bevorzugter im Jumping-Test.

Bevorzugt weisen die Verbindungen selbst in einer Dosierung, welche um Faktor 10, 20, 30, 40 oder 50, bevorzugter um Faktor 75, 100, 125, 150 oder 175, noch bevorzugter um Faktor 200, 300, 400 oder 500, am bevorzugtesten um Faktor 600, 700, 800 oder 900, und insbesondere um Faktor 1000 höher als die halbmaximal wirksame Dosierung ED₅₀ⁿ ist, keine signifikante körperliche Abhängigkeit bzw. Entzugssymptome als Nebenwirkung auf.
Geeignete Methoden zur Untersuchung einer Wirkstoff-induzierten körperlichen Abhängigkeit sind dem Fachmann bekannt. Bevorzugt erfolgt die Untersuchung im Jumping-Modell an der Maus (analog zu Saelens JK, Arch Int Pharmacodyn 190: 213-218, 1971) als Nalxoninduzierter Entzug. Die Auswertung der experimentellen Befunde im Hinblick auf statistisch signifikante Unterschiede zwischen den jeweiligen Dosisgruppen und der Vehikel-Kontrollgruppen erfolgt bevorzugt mittels Fisher's exact Test für den Paramter "Anzahl der Tiere mit Entzugssymptomatik" sowie mittels Kruskal-Wallis Test für den Parameter "Sprungfrequenz" bevorzugt wie im experimentellen Teil beschrieben. Dabei wird das Signifikanzniveau jeweils auf p < 0.05 festgelegt. Die Gruppengrößen betragen üblicherweise n=12. Bezüglich weiterer Einzelheiten dieses Tiermodells wird ferner auf den experimentellen Teil verwiesen.
In einer bevorzugten Ausführungsform der erfindungsgemäßen Verwendung zeigen die Verbindungen gemäß der allgemeinen Formel (III) bei Verabreichung in der halbmaximal wirksamen Dosierung ED₅₀ⁿ, welche im Hinblick auf die Wirksamkeit der Verbindung gegen neuropathischen Schmerz definiert ist, und bevorzugt selbst in einer Dosierung, welche um den Faktor 5 höher als ED₅₀ⁿ ist, keine signifikante psychische Abhängigkeit bzw.Sucht als Nebenwirkung, bevorzugt in der Ratte, bevorzugter mittels konditionierter Platzpräferenz. Bevorzugt weisen die Verbindungen selbst in einer Dosierung, welche um Faktor 10, 20, 30, 40 oder 50, bevorzugter um Faktor 75, 100, 125, 150 oder 175, noch bevorzugter um Faktor 200, 300, 400 oder 500, am bevorzugtesten um Faktor 600, 700, 800 oder 900, und insbesondere um Faktor 1000 höher als die halbmaximal wirksame Dosierung ED₅₀ⁿ ist, keine signifikante psychische Abhängigkeit bzw. Sucht als Nebenwirkung auf.
Geeignete Methoden zur Untersuchung einer Wirkstoff-induzierten psychischen Abhängigkeit bzw. Sucht sind dem Fachmann bekannt. Bevorzugt erfolgt die Untersuchung mittels konditionierter Platzpräferenz bei Ratten, bevorzugt wie in Tzschentke, T.M., Bruckmann, W. and Friderichs, F. (2002) Lack of sensitization during place conditioning in rats is consistent with the low abuse potential of tramadol. Neuroscience Letters 329, 25-28 beschrieben. Die Auswertung der experimentellen Befunde im Hinblick auf statistisch signifikante Unterschiede in der Präferenz der Tiere für den Wirkstoff bzw. das Vehikel erfolgt bevorzugt mittels gepaartem t-Test Dabei wird das Signifikanzniveau auf p < 0.05 festgelegt. Die Gruppengrößen betragen üblicherweise n=8. Bezüglich weiterer Einzelheiten dieses Tiermodells wird auf die Methodenbeschreibung in Tzschentke, T.M., Bruckmann, W. and Friderichs, F. (2002) Neuroscience Letters 329, 25-28 verwiesen.

Die Verbindungen gemäß der allgemeinen Formel (III) eignen sich zur Verwendung zur Behandlung von chronischem Schmerz, bevorzugt neuropathischem Schmerz, bevorzugter gegen mononeuropathischen/ neuralgischen oder polyneuropathischen Schmerz, noch bevorzugter gegen Schmerz bei post-herpetischer Neuralgie oder bei diabetischer Polyneuropathie.
Dem Fachmann sind die Definitionen der unterschiedlichen Formen von chronischem Schmerz bekannt. In diesem Zusammenhang kann beispielsweise verwiesen werden auf Merskey H., Bogduk N. Classification of chronic pain. Seattle: IASP Press 1994, Bennett G.J., Anesth Analg. 2003, 97, 619-20, sowie Backonja M.M., Anesth Analg. 2003, 97, 785-90.
Zum Zweck der Beschreibung ist chronischer Schmerz vorzugsweise definiert als eine über einen längeren Zeitraum (üblicherweise wenigstens 3, 4, 5 oder 6 Monate) bestehende Schmerzsymptomatik, die über die normale Heilungszeit hinaus anhält. Vorzugsweise ist neuropathischer Schmerz definiert als Schmerz oder sensorisches Phänomen, das durch Läsion, Erkrankung oder Dysfunktion des zentralen oder peripheren Nervensystems verursacht wird. Zum Zweck der Beschreibung ist Akutschmerz vorzugsweise definiert als eine unangenehme sensorische und gefühlsmäßige Erfahrung, die mit akuter oder potentieller Gewebeschädigung einhergeht oder in Form solcher Schädigungen beschrieben wird (vgl. Definition der International Association for the Study of Pain® (IASP)).
Die erfindungsgemäß zur Anwendung kommenden Verbindungen gemäß der allgemeinen Formel (III) weisen bevorzugt einen Kᵢ-Wert am µ-Opioid-Rezeptor von höchstens 1000 nM, bevorzugter höchstens 500 nM, noch bevorzugter 100 nM, am bevorzugtesten höchstens 50 nM und insbesondere höchstens 25 nM auf.
Methoden zur Bestimmung des Kᵢ-Werts am µ-Opioid-Rezeptor sind dem Fachmann bekannt. Bevorzugt erfolgt die Bestimmung in einem homogenen Ansatz in Mikrotiterplatten. Hierzu werden vorzugseise Verdünnungsreihen der jeweils zu prüfenden Substanzen mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wird bevorzugt 50 mmol/l Tris-HCI supplementiert mit 0,05 Gew.-% Natriumazid und mit 0,06 Gew.-% bovinem Serumalbumin verwendet. Zur Bestimmung der unspezifischen Bindung wird bevorzugt zusätzlich 25 µmol/l Naloxon zugegeben. Nach Beendigung der neunzigminütigen Inkubationszeit werden die Mikrotiterplatten vorzugsweise für 20 Minuten bei 1000 g abzentrifugiert und die Radioaktivität in einem β-Counter (Microbeta-Trilux, Firma PerkinElmer Wallac, Freiburg, Deutschland) vermessen. Es wirde die prozentuale Verdrängung des radioaktiven Liganden aus seiner Bindung zum humanen µ-Opiatrezeptor bei einer Konzentration der Prüfsubstanzen von bevorzugt 1 µmol/l bestimmt und als prozentuale Hemmung (%Hemmung) der spezifischen Bindung angegeben. Ausgehend von der prozentualen Verdrängung durch unterschiedliche Konzentrationen der zu prüfenden Verbindungen können IC₅₀ Hemmkonzentrationen berechnet werden, die eine 50-prozentige Verdrängung des radioaktiven Liganden bewirken. Durch Umrechnung mittels der Cheng-Prusoff-Beziehung können daraus die Kᵢ-Werte für die Prüfsubstanzen berechnet werden.

Die erfindungsgemäße verwendeten Verbindungen gemäß der allgemeinen Formel (III) weisen bevorzugt einen Kᵢ-Wert am ORL1-Rezeptor von höchstens 500 nM, bevorzugter höchstens 100 nM, am bevorzugtesten höchstens 50 nM und insbesondere höchstens 10 nM auf.
Methoden zur Bestimmung des Kᵢ-Werts am ORL1-Rezeptor sind dem Fachmann bekannt. Bevorzugt erfolgt die Bestimmung in einem Rezeptorbindungsassay mit ³H-Nociceptin/ Orphanin FQ mit Membranen von rekombinanten CHO-ORL1 Zellen. Dieses Testsystem wird vorzugsweise gemäß der von Ardati et al. (Mol. Pharmacol., 51, 1997, S. 816-824) vorgestellten Methode durchgeführt. Die Konzentration von ³H-Nociceptin/Orphanin FQ beträgt bei diesen Versuchen bevorzugt 0.5 nM. Die Bindungsassays werden bevorzugt mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wird bevorzugt unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt.

Ein weiterer Gegenstand der Erfindung betrifft ein Verfahren zur Herstellung der erfindungsgemäßen Verbindungen. Geeignete Verfahren zur Synthese der erfindungsgemäßen verbindungen sind dem Fachmann grundsätzlich bekannt.

Nachfolgend werden bevorzugte Syntheserouten beschrieben:

### Synthese der Ketonbausteine E:

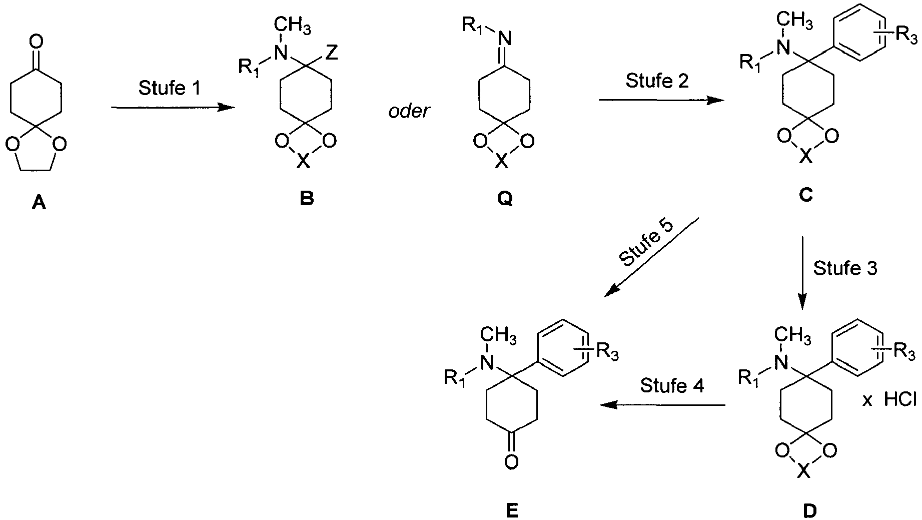

### Stufe 1 (Via B)

Strukturen der Formel B lassen sich durch Reaktion von Ketonen A mit Aminen und aciden Reaktanden Z-H herstellen. Geeignete Reaktanden Z-H sind z. B. Cyanwasserstoff, 1,2,3-Triazol, Benzotriazol oder Pyrazol. Ein besonders bevorzugter Weg zu Verbindungen der Struktur B ist die Umsetzung von Ketonen mit Metallcyaniden und dem entsprechenden Amin in Gegenwart von Säure, vorzugsweise in einem Alkohol, bei Temperaturen von - 40 bis 60 °C, vorzugsweise bei Raumtemperatur mit Alkalimetallcyaniden in Methanol. Ein weiterer besonders bevorzugter Weg zu Verbindungen der Struktur B ist die Umsetzung von Ketonen mit 1,2,3-Triazol und dem entsprechenden Amin in Gegenwart unter wasserentziehenden Bedingungen, vorzugsweise unter Verwendung eines Wasserabscheiders bei erhöhter Temperatur in einem inerten Löungsmittel oder unter Verwendung von Molsieb oder einem anderen Trockenmittel. Analog lassen sich B analoge Strukturen mit Benzotriazol- oder Pyrazol- statt Triazolgruppen einführen.

### Stufe 1 (Via Q)

Die Herstellung von Iminen der allgemeinen Formel Q aus Ketonen A ergibt sich aus dem allgemeinen Stand der Technik.

### Stufe 2 (via B)

Allgemein können Acetale C durch Substitution von geeigneten Abgangsgruppen Z in Strukturen der Formel B erhalten werden. Geeignete Abgangsgruppen sind bevorzugt Cyanogruppen; 1,2,3-Triazol-1-yl-Gruppen. Weitere geeignete Abgangsgruppen sind 1H-Benzo[d][1,2,3]triazol-1-yl-Gruppen und Pyrazol-1-yl-Gruppen (Katritzky et al., Synthesis 1989, 66-69). Ein besonders bevorzugter Weg zu Verbindungen der Struktur C ist die Umsetzung von Aminonitrilen B (Z= CN) mit entsprechenden Organometallverbindungen, vorzugsweise Grignardverbindungen, vorzugsweise in Ethern, vorzugsweise bei RT. Die Organometallverbindungen sind entweder käuflich erhältlich oder können Herstellung nach allg. Stand der Technik hergestellt werden. Ein weiterer besonders bevorzugter Weg zu Verbindungen der Struktur C ist die Umsetzung von Aminotriazolen B (Z = Triazol) mit entsprechenden Organometallverbindungen, vorzugsweise Grignardverbindungen, vorzugsweise in Ethern, vorzugsweise bei RT. Die Organometallverbindungen sind entweder käuflich erhältlich oder können Herstellung nach allg. SdT hergestellt werden.

### Stufe 2 (via Q)

Aminoacetale C mit maximal einem Substituenten am Stickstoffatom können nach dem Fachmann prinzipiell bekannte Verfahren durch Addition von Kohlenstoff-Nucleophilen an Imine Q erhalten werden, vorzugsweise Organometallverbindungen in inerten Lösungsmitteln, besonders bevorzugt mit Grignardreagenzien oder Organolithiumverbindungen, bevorzugt in Ethern, bevorzugt bei Temperaturen von 100 bis RT.

### Stufe 4/5:

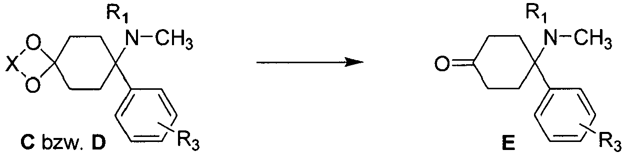

Verbindungen der Formel E können aus entsprechenden Acetalen C, oder aus deren Salzen D, nach allgemein bekanntem Stand der Technik durch Entschützen mittels Säuren freigesetzt werden. Dabei ist X aus der Gruppe Alkyl, Alkyl/ Alkyliden/ mit Aryl oder Alkyl (gesättigt/ungesättigt) subst. Alkyliden ausgewählt
Herstellung von C (R₁ ≠ -H) aus Ca (R₁ = -H)

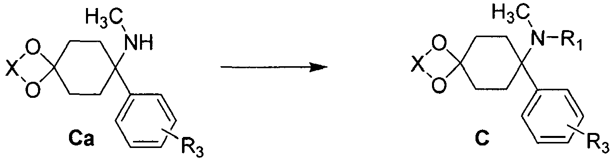

Aminoacetale Ca mit maximal einem Substituenten am Stickstoffatom können nach dem Fachmann prinzipiell bekannte Verfahren, z.B. durch reduktive Aminierung, in entsprechende Amino-acetale C mit einem oder zwei weiteren Substituenten am Stickstoff übergeführt werden.

### Aminonitril-Route, Imin-Route und Triazol-Route

Die benötigten Keton Intermediate E können beispielsweise nach den folgenden drei unterschiedliche Routen hergestellt werden: *(1) Aminonitril-Route (2) Imin-Route und (3) Triazol-Route.*

### (1) Aminonitril-Route:

In der Aminonitril-Route wird, wie im folgenden Syntheseschema beschrieben, aus einem Ketonvorläufer A das Aminonitril Ba synthetisiert, welches unter Verwendung eines Nukleophils MR3 in die Bausteine C bzw. D und weiter in E überführt wird. Dieser Syntheseweg wurde bereits in WO 2004/043967 beschrieben und angewendet.

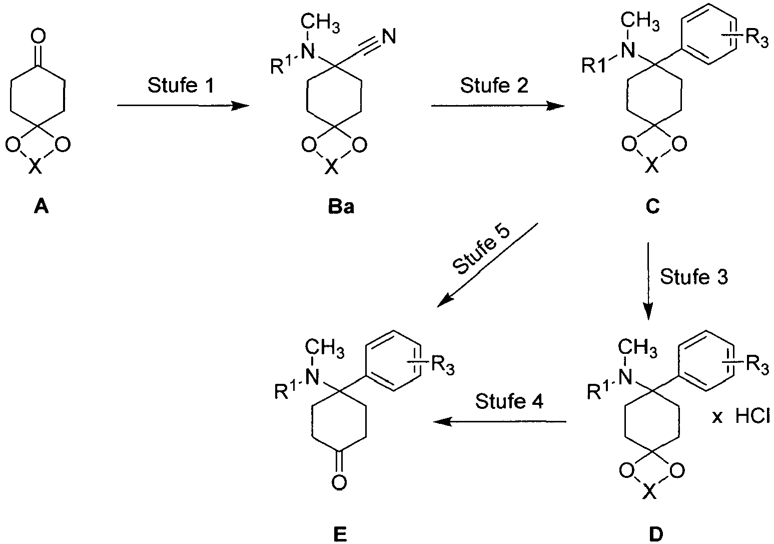

### (2) Imin-Route:

In der Imin-Route wird, wie im folgenden Schema beschrieben, aus einem Ketonvorläufer A das Imin Q synthetisiert, welches unter Verwendung eines Nukleophils MR3 in die Bausteine C bzw. D und weiter in E überführt wird. Die benötigten Iminbausteine Q können nach einer dem Fachmann bekannten Methode hergestellt werden (Layer, Chem. Rev., 1963, 8, 489-510). Für Addition der Organometallspezies MR3 an das Imin Q wurde auf literaturbekannte Verfahren (z.B. Maddox et al., J. Med. Chem., 1965, 8, 230-235. Kudzma et al., J. Med. Chem., 1989, 32, 2534-2542.) zurückgegriffen. Stufen 3, 4 und 5 erfolgen analog der Aminonitril-Route.

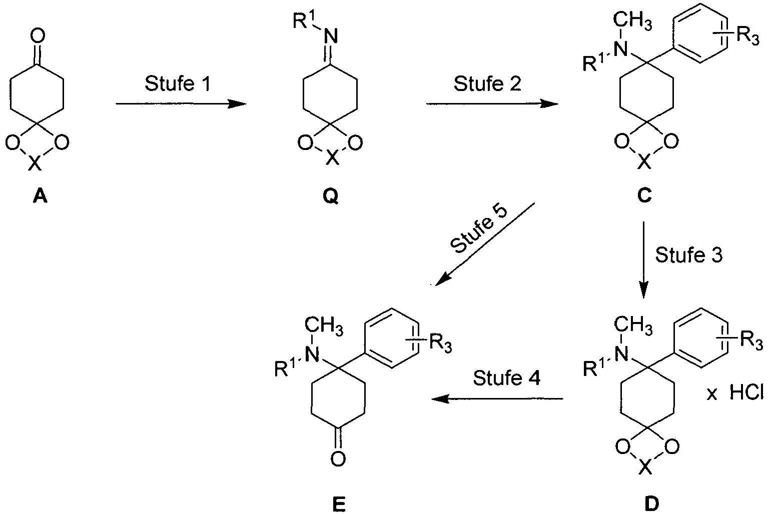

### (3) Triazol-Route:

In der Triazol-Route wurde, wie im folgenden Schema beschrieben, aus einem Ketonvorläufer A das Triazol Bb synthetisiert, welches unter Verwendung eines Nukleophils MR3 in die Bausteine C bzw. D und weiter in E überführt wird. Die Bedingungen können den angegebenen Literaturstellen entnommen werden: (a) Katritzky et al. Synthesis, 1992, 1295-1298. (b) Prashad, et al., Tetrahedron Lett. 2005, 46, 5455-5458.

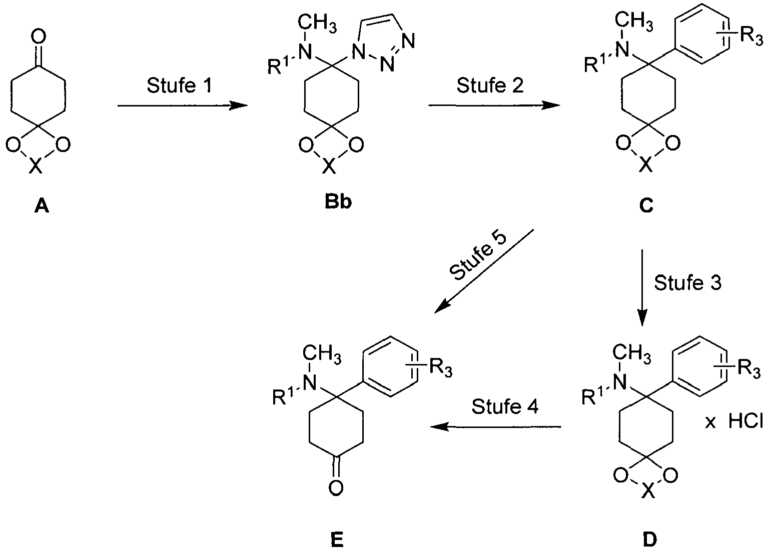

### Synthese der Spiroamine (AMN)

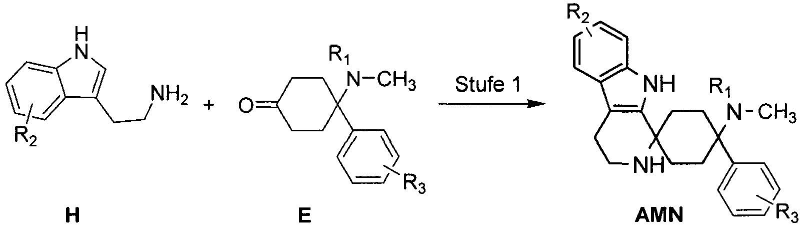

Tryptamine des Typs H können in Reaktionen von Typ der Pictet-Spengler-Reaktion, mit Ketonen E unter Zugabe von mindestens einem Reagens aus der Gruppe der Säuren, Säureanhydride, Ester, schwach sauer reagierende Salze oder Lewissäuren unter Bildung von Produkten der Formel AMN zur Reaktion gebracht werden.

Bevorzugt kommt dabei mind. ein Reagens aus der Gruppe Carbonsäuren, Phosphorsäuren oder Sulfonsäuren oder deren jeweiligen Anhydride, Carbonsäuretrialkylsilylester, sauer reagierende Salze, Mineralsäuren oder Lewissäuren ausgewählt aus der Gruppe bestehend aus Bortrifluorid, Indium(III)chlorid, Titantetrachlorid, Aluminium(III)chlorid, oder unter Zusatz wenigstens eines Übergangsmetallsalzes, vorzugsweise unter Zusatz wenigstens eines Übergangsmetalltriflats (Übergangsmetalltrifluormethansulfonats), besonders bevorzugt unter Zusatz wenigstens eines Übergangsmetalltrifluormethansulfonats ausgewählt aus der Gruppe bestehend aus Scandium(III)trifluormethansulfonat, Ytterbium(III)trifluormethansulfonat und Indium(III) trifluormethansulfonat, ggf. unter Zusatz von Celite, mit festphasengebundenen Reaktanden oder Reagenzien, bei erhöhter oder erniedrigter Temperatur, mit oder ohne Mikrowelleneinstrahlung, ggf. in einem geeigneten Lösungsmittel oder Lösungsmittelgemisch wie beispielsweise, chlorierten oder unchlorierten, dann vorzugsweise aromatischen, Kohlenwasserstoffen, Acetonitril; in etherischen Lösungsmitteln, vorzugsweise in Diethylether oder THF;oder in Nitromethan, in geeigneten Fällen auch in Alkoholen oder Wasser, zum Einsatz. Besonders bevorzugt werden dabei Pyridinium-para-Toluolsulfonat, Phosphorpentoxid in Gegenwart von Celite, Bortrifluorid-etherat, Trifluoressigsäure, Orthotitansäure-tetraisopropylester zusammen mit Trifluoressigsäure, Trifluormethansulfonsäuretrimethylsilylester, Trifluormethansulfonsäure, Methansulfonsäure, Trifluoressigsäure, Essigsäure, Phosphorsäure, Polyphosphorsäure, Polyphosphatester, p Toluolsulfonsäure, Salzsäure HCl-Gas, Schwefelsäure zusammen mit Acetatpuffer, Zinntetrachlorid, eingesetzt.

Wiederum bevorzugt werden die in den nachfolgenden Beispielen aufgeführten Bedingungen angewandt.

Verbindungen der allgemeinen Formeln H und E sind entweder kommerziell erhältlich, oder ihre Herstellung ist aus dem Stand der Technik bekannt oder in für den Fachmann offensichtlicher Weise aus dem Stand der Technik ableitbar. Insbesondere relevant sind hierfür die folgenden Zitate: Jirkovsky et al., J. Heterocycl. Chem., 12, 1975, 937-940; Beck et al., J. Chem. Soc. Perkin 1, 1992, 813-822; Shinada et al., Tetrahedron Lett., 39, 1996, 7099-7102; Garden et al., Tetrahedron, 58, 2002, 8399-8412; Lednicer et al., J. Med. Chem., 23, 1980, 424-430; Bandini et al. J. Org. Chem. 67, 15; 2002, 5386 - 5389; Davis et al., J.Med.Chem. 35, 1, 1992, 177-184; Yamagishi et al., J.Med.Chem. 35, 11, 1992, 2085-2094; Gleave et al.; Bioorg.Med.Chem.Lett. 8, 10, 1998, 1231-1236; Sandmeyer, Helv.Chim.Acta; 2; 1919; 239; Katz et al.; J. Med. Chem. 31, 6, 1988; 1244-1250; Bac et al. Tetrahedron Lett. 1988, 29, 2819; Ma et al. J. Org. Chem. 2001, 66, 4525; Kato et al. J. Fluorine Chem. 99, 1, 1999, 5-8.

### Synthese der Spiroamide (AMD)

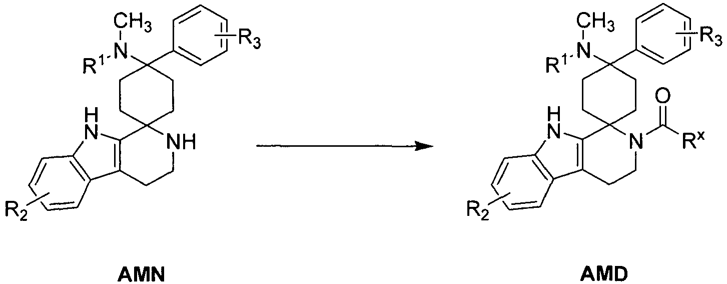

Verbindungen der allgemeinen Formel AMN können mit Carbonsäuren in wenigstens einem Lösungsmittel, vorzugsweise ausgewählt aus der Gruppe bestehend aus Dichlormethan, Acetonitril, Dimethylformamid, Diethylether, Dioxan und Tetrahydrofuran, unter Zusatz wenigstens eines Kupplungsreagenzes, vorzugsweise ausgewählt aus der Gruppe bestehend aus Carbonyldiimidazol (CDI), 2-Chloro-1-methylpyridinium iodide (Mukaiyama Reagenz), *N*-(3-Dimethylaminopropyl)-*N'*-ethylcarbodiimid (EDCI), *O*-(Benzotriazol-1-yl)-*N,N,N',N'*-tetramethyluronium tetrafluoroborat (TBTU), *N,N'*-Dicyclohexylcarbodiimid (DCC) und 1-Benzotriazolyloxy-tris-(dimethylamino)-phosphonium hexafluorophosphat (BOP) ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Diisopropylethylamin und Pyridin und ggf. unter Zusatz von 4-(Dimethylamino)pyridin oder 1-Hydroxybenzotriazol bei Temperaturen von vorzugsweise 25°C bis 150°C ggf. unter Mikrowelleneinstrahlung zu Verbindungen der allgemeinen Formel AMD umgesetzt werden.

Verbindungen der allgemeinen Formel AMN können mit Säureanhydriden und Carbonsäurechloriden in wenigstens einem Lösungsmittel, vorzugsweise ausgewählt aus der Gruppe bestehend aus Dichlormethan, Acetonitril, Dimethylformamid, Diethylether, Dioxan und Tetrahydrofuran ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Diisopropylethylamin und Pyridin und ggf. unter Zusatz von 4-(Dimethylamino)pyridin oder 1-Hydroxybenzotriazol bei Temperaturen von vorzugsweise 25°C bis 150°C ggf. unter Mikrowelleneinstrahlung zu Verbindungen der allgemeinen Formel AMD umgesetzt werden.

Bezüglich weiterer Einzelheiten zur Synthese der erfindungsgemäß verwendeten Verbindungen, insbesondere im Hinblick auf die Synthese geeigneter Eduktbausteine, wird ferner vollumfänglich verwiesen auf WO2004/043967, WO2005/063769, WO2005/066183, WO2006/018184, WO2006/108565, WO2007/124903 und WO2008/009416. Ein Fachmann erkennt, dass geeignete Eduktbausteine für die Synthese der erfindungsgemäßen Verbindungen in Analogie zu den in diesen Druckschriften offenbarten Syntheseschemata und Ausführungsbeispielen hergestellt werden können.
Die erfindungsgemäß verwendeten Verbindungen wirken beispielsweise auf die im Zusammenhang mit verschiedenen Erkrankungen relevanten ORL1- und µ-Opioid-Rezeptoren, sodass sie sich als Wirkstoff (Arzneistoff) in einer pharmazeutischen Zusammensetzung eignen.
Ein weiterer Gegenstand der Erfindung betrifft die Verwendung einer pharmazeutischen Zusammensetzung zur Anwendung bei der Behandlung von neuropathischem und/oder chronischem Schmerz, wobei die Zusammensetzung einen physiologisch verträglichen Träger und wenigstens eine Verbindung gemäß der allgemeinen Formel (III) enthält.
Bevorzugt kommt eine Zusammensetzung zur Anwendung,die
- fest, flüssig oder pastös ist; und/oder
- enthält die erfindungsgemäße Verbindung in einer Menge von 0,001 bis 99 Gew.-%, vorzugsweise 1,0 bis 70 Gew.-%, bezogen auf das Gesamtgewicht der Zusammensetzung.
Die erfindungsgemäß verwendete pharmazeutische Zusammensetzung kann ggf. geeignete Zusatz- und/oder Hilfsstoffe und/oder ggf. weitere Wirkstoffe enthalten.
Beispiele für geeignete physiologisch verträgliche Träger, Zusatz- und/oder Hilfsstoffe sind Füllstoffe, Lösungsmittel, Verdünnungsmittel, Farbstoffe und/oder Bindemittel. Diese Stoffe sind dem Fachmann bekannt (vgl. H.P. Fiedler, Lexikon der Hilfsstoffe für Pharmazie, Kosmetik und angrenzende Gebiete, Editio Cantor Aulendoff).
Bevorzugt enthält die erfindungsgemäß verwendete Zusammensetzung die erfindungsgemäße Verbindung in einer Menge von 0,001 bis 99 Gew.-%, bevorzugter 0,1 bis 90 Gew.-%, noch bevorzugter 0,5 bis 80 Gew.-%, am bevorzugtesten 1,0 bis 70 Gew.-% und insbesondere 2,5 bis 60 Gew.-%, bezogen auf das Gesamtgewicht der Zusammensetzung.
Bevorzugt ist die Zusammensetzung zur erfindungsgemäßen Verwendung zur systemischen, topischen oder lokalen Verabreichung konfektioniert, vorzugsweise zur oralen Verabreichung.

In einer weiteren Ausführungsform der erfindungsgemäßen Verwendung kommt eine pharmazeutische Darreichungsform zur Anwendung, welche die erfindungsgemäße pharmazeutische Zusammensetzung enthält.
In einer bevorzugten Ausführungsform ist die erfindunsggemäß verwendete Darreichungsform zur zweimal täglichen Verabreichung, zur einmal täglichen Verabreichung oder zur seltener als einmal täglichen Verabreichung konfektioniert, vorzugsweise zur höchstens einmal täglichen Verabreichung.
Vorzugsweise handelt es sich dabei um eine systemische, insbesondere orale Verabreichung.
In einer bevorzugten Ausführungsform enthält die erfindungsgemäß verwendete Darreichungsform die Verbindung gemäß der allgemeinen Formel (III) in einer so geringen Dosis enthält, dass sie bei der Behandlung von akutem Schmerz nicht signifikant wirksam ist. Bevorzugt liegt diese Dosis im Bereich von 1,0 µg bis 10 mg, bezogen auf das Molekulargewicht der freien Base.
Bevorzugt beträgt die Dosis 0,001 mg±50%, 0,002 mg±50%, 0,003 mg±50%, 0,004 mg±50%, 0,005 mg±50%, 0,006 mg±50%, 0,007 mg±50%, 0,008 mg±50%, 0,009 mg±50%, 0,01 mg±50%, 0,02 mg±50%, 0,03 mg±50%, 0,04 mg±50%, 0,05 mg±50%, 0,06 mg±50%, 0,07 mg±50%, 0,08 mg±50%, 0,09 mg±50%, 0,1 mg±50%, 0,15 mg±50%, 0,2 mg±50%, 0,25 mg±50%, 0,3 mg±50%, 0,35 mg±50%, 0,4 mg±50%, 0,45 mg±50%, 0,5 mg±50%, 0,55 mg±50%, 0,6 mg±50%, 0,65 mg±50%, 0,7 mg±50%, 0,75 mg±50%, 0,8 mg±50%, 0,85 mg±50%, 0,9 mg±50%, 0,95 mg±50%, 1 mg±50%, 1,5 mg±50%, 2 mg±50%, 2,5 mg±50%, 3 mg±50%, 3,5 mg±50%, 4 mg±50%, 4,5 mg±50%, 5 mg±50%, 5,5 mg±50%, 6 mg±50%, 6,5 mg±50%, 7 mg±50%, 7,5 mg±50%, 8 mg±50%, 8,5 mg±50%, 9 mg±50%, 9,5 mg±50% oder 10 mg±50%, bezogen auf das Molekulargewicht der freien Base.
Bevorzugter beträgt die Dosis 0,001 mg±25%, 0,002 mg±25%, 0,003 mg±25%, 0,004 mg±25%, 0,005 mg±25%, 0,006 mg±25%, 0,007 mg±25%, 0,008 mg±25%, 0,009 mg±25%, 0,01 mg±25%, 0,02 mg±25%, 0,03 mg±25%, 0,04 mg±25%, 0,05 mg±25%, 0,06 mg±25%, 0,07 mg±25%, 0,08 mg±25%, 0,09 mg±25%, 0,1 mg±25%, 0,15 mg±25%, 0,2 mg±25%, 0,25 mg±25%, 0,3 mg±25%, 0,35 mg±25%, 0,4 mg±25%, 0,45 mg±25%, 0,5 mg±25%, 0,55 mg±25%, 0,6 mg±25%, 0,65 mg±25%, 0,7 mg±25%, 0,75 mg±25%, 0,8 mg±25%, 0,85 mg±25%, 0,9 mg±25%, 0,95 mg±25%, 1 mg±25%, 1,5 mg±25%, 2 mg±25%, 2,5 mg±25%, 3 mg±25%, 3,5 mg±25%, 4 mg±25%, 4,5 mg±25%, 5 mg±25%, 5,5 mg±25%, 6 mg±25%, 6,5 mg±25%, 7 mg±25%, 7,5 mg±25%, 8 mg±25%, 8,5 mg±25%, 9 mg±25%, 9,5 mg±25% oder 10 mg±25%, bezogen auf das Molekulargewicht der freien Base.
Besonders bevorzugt beträgt die Dosis 0,001 mg, 0,002 mg, 0,003 mg, 0,004 mg, 0,005 mg, 0,006 mg, 0,007 mg, 0,008 mg, 0,009 mg, 0,01 mg, 0,02 mg, 0,03 mg, 0,04 mg, 0,05 mg, 0,06 mg, 0,07 mg, 0,08 mg, 0,09 mg, 0,1 mg, 0,15 mg, 0,2 mg, 0,25 mg, 0,3 mg, 0,35 mg, 0,4 mg, 0,45 mg, 0,5 mg, 0,55 mg, 0,6 mg, 0,65 mg, 0,7 mg, 0,75 mg, 0,8 mg, 0,85 mg, 0,9 mg, 0,95 mg, 1 mg, 1,5 mg, 2 mg, 2,5 mg, 3 mg, 3,5 mg, 4 mg, 4,5 mg, 5 mg, 5,5 mg, 6 mg, 6,5 mg, 7 mg, 7,5 mg, 8 mg, 8,5 mg, 9 mg, 9,5 mg oder 10 mg, bezogen auf das Molekulargewicht der freien Base.
In einer bevorzugten Ausführungsform enthält die erfindungsgemäß verwendete Darreichungsform eine Verbindung gemäß der allgemeinen Fomel (III) in einer Menge von 10 µg±90%, bevorzugter 10 µg±75%, noch bevorzugter 10 µg±50%, am bevorzugtesten 10 µg±25%, und insbesondere 10 µg±10%, bezogen auf das Molekulargewicht der freien Base.
In einer anderen bevorzugten Ausführungsform enthält die erfindungsgemäß verwendete Darreichungsform eine Verbindung gemäß der allgemeinen Fomel (III) in einer Menge von 100 µg±90%, bevorzugter 100 µg±75%, noch bevorzugter 100 µg±50%, am bevorzugtesten 100 µg±25%, und insbesondere 100 µg±10%, bezogen auf das Molekulargewicht der freien Base.
In einer weiteren bevorzugten Ausführungsform enthält die erfindungsgemäß verwendete Darreichungsform eine Verbindung gemäß der allgemeinen Fomel (III) in einer Menge von 250 µg±90%, bevorzugter 250 µg±75%, noch bevorzugter 250 µg±50%, am bevorzugtesten 250 µg±25%, und insbesondere 250 µg±10%, bezogen auf das Molekulargewicht der freien Base.
In einer weiteren bevorzugten Ausführungsform enthält die erfindungsgemäß verwendete Darreichungsform eine Verbindung gemäß der allgemeinen Fomel (III) in einer Menge von 500 µg±90%, bevorzugter 500 µg±75%, noch bevorzugter 500 µg±50%, am bevorzugtesten 500 µg±25%, und insbesondere 500 µg±10%, bezogen auf das Molekulargewicht der freien Base.
In einer anderen bevorzugten Ausführungsform enthält die erfindungsgemäß verwendete Darreichungsform eine Verbindung gemäß der allgemeinen Fomel (III) in einer Menge von 750 µg±90%, bevorzugter 750 µg±75%, noch bevorzugter 750 µg±50%, am bevorzugtesten 750 µg±25%, und insbesondere 750 µg±10%, bezogen auf das Molekulargewicht der freien Base.
In einer weiteren bevorzugten Ausführungsform enthält die erfindungsgemäß verwendete Darreichungsform eine Verbindung gemäß der allgemeinen Fomel (III) in einer Menge von 1000 µg±90%, bevorzugter 1000 µg±75%, noch bevorzugter 1000 µg±50%, am bevorzugtesten 1000 µg±25%, und insbesondere 1000 µg±10%, bezogen auf das Molekulargewicht der freien Base.
Die erfindungsgemäß verwendete Darreichungsform kann z.B. als flüssige Arzneiform in Form von Injektionslösungen, Tropfen oder Säften, oder als halbfeste Arzneiform in Form von Granulaten, Tabletten, Pellets, Patches, Kapseln, Pflastern/Sprühpflastern oder Aerosolen verabreicht werden. Die Auswahl der Hilfsstoffe etc. sowie die einzusetzenden Mengen derselben hängen davon ab, ob die Darreichungsform oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll.
Für die orale Applikation eignen sich Darreichungsformen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Verbindungen in einem Depot, in gelöster Form oder in einem Pflaster, ggf. unter Zusatz von die Hautpenetration fördernden Mitteln, sind geeignete perkutane Applikationszubereitungen zur erfindungsgemäßen Anwendung.
Oral oder perkutan anwendbare Darreichungsformen können die erfindungsgemäß verwendeten Verbindungen verzögert freisetzen. Die erfindungsgemäß verwendeten Verbindungen können auch in parenteralen Langzeitdepotformen wie z. B. Implantaten oder implantierten Pumpen angewendet werden. Prinzipiell können den erfindungsgemäß verwendeten Darreichungsformen andere dem Fachmann bekannte weitere Wirkstoffe zugesetzt werden.
In einer bevorzugten Ausführungsform werden die erfindungsgemäß verwendeten Verbindungen aus der Darreichungsform sofort freigesetzt (*immediate release,* IR), d.h. vorzugsweise werden unter *in vitro* Bedingungen, bevorzugt gemäß Ph. Eur., nach 20 Minuten mindestens 80% des ursprünglich enthaltenen Wirkstoffs freigesetzt.

Es wurde überraschend gefunden, dass sich die erfindungsgemäß verwendeten Verbindungen gemäß der allgemeinen Formel (III) durch eine ungewöhnlich lange Halbwertszeit (t_{1/2}) bzw. pharmakodynamische Wirkdauer auszeichnen, so dass eine vergleichsweise seltene Verabreichung ausreicht, um eine vergleichsweise lang anhaltende pharmakologische Wirksamkeit und damit Schmerzlinderung zu erzielen.
Darreichungsformen mit retardierter Freisetzung der erfindungsgemäß verwendeten Verbindungen sind dazu nicht zwingend erforderlich, selbst bei sofortiger Freisetzung (*immediate release,* IR) wird infolge der langen Halbwertszeit eine lang anhaltende Wirkung erreicht. Wegen der IR-Eigenschaft solcher Darreichungsformen hat dies den zusätzlichen Vorteil, dass bei lang anhaltender Wirksamkeit dennoch ein schnelles Anfluten des Wirkstoffs (rapid onset) und damit ein schnelles Einsetzen der pharmakologischen Wirksamkeit nach der ersten Verabreichung erzielt werden. Somit werden Eigenschaften von IR-Darreichungsformen mit Eigenschaften von PR-Darreichungsformen vereint (PR, *prologed release,* verzögerte Freisetzung).
In einer bevorzugten Ausführungsform handelt es sich bei der erfindungsgemäß verwendeten Darreichungsform um eine Darreichungsform mit sofortiger Wirkstofffreisetzung (IR), welche eine erfindungsgemäße Verbindung, vorzugsweise der allgemeinen Formel (VI) als freie Base oder ein physiologisch verträgliches Salz, vorzugsweise das Hydrochlorid, Citrat oder Hemicitrat enthält, und zur höchstens einmal täglichen, bevorzugt genau einmal täglichen, vorzugsweise oralen Verabreichung konfektioniert ist. In diesem Zusammenhang bedeutet "sofortige Wirkstofffrisetzung", dass unter *in vitro* Bedingungen, bevorzugt gemäß Ph. Eur., nach 20 Minuten mindestens 80% des ursprünglich enthaltenen Wirkstoffs freigesetzt werden.
Die an den Patienten zu verabreichende Menge der erfindungsgemäß verwendeten Verbindungen variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg, bevorzugter 1 bis 10 µg/kg wenigstens einer Verbindung gemäß der allgemeinen Formel (III) appliziert.
Für alle vorstehenden Ausführungen der erfindungsgemäß verwendeten Darreichungsformen ist es besonders bevorzugt, wenn die Darreichungsform neben wenigstens einer Verbindung gemäß der allgemeinen Formel (III) noch einen weiteren Wirkstoff enthält.

Der ORL1-Rezeptor und der µ-Opioidrezeptor werden insbesondere mit dem Schmerzgeschehen in Zusammenhang gebracht. Entsprechend können die erfindungsgemäß verwendeten Verbindungen zur Herstellung eines Medikaments zur Behandlung von chronischem Schmerz, bevorzugt von neuropathischem Schmerz, bevorzugter von mononeuropathischem/ neuralgischem oder polyneuropathischem Schmerz, noch bevorzugter von Schmerz bei post-herpetischer Neuralgie oder bei diabetischer Polyneuropathie verwendet werden.

Die folgenden Beispiele dienen der Erläuterung der Erfindung, sind jedoch nicht einschränkend auszulegen:
Bei der nachfolgenden Nomenklatur der Stereochemie der Beispielverbindungen bezieht sich die Angabe "(E)" auf die Substitution an einer Doppelbindung, z.B. an einem Zimtsäurederivat, und die Bezeichnung "cis" bzw. "trans" auf die Substitution am Cyclohexylring.

### Synthese der Indol Bausteine (H)

### Baustein H-1:

### 2-(1H-indol-3-yl)ethanamin (H-1)

Zur Zeit der Synthese kommerziell erhältlich bei Aldrich.

### Baustein H-2:

### 2-(5-Fluoro-1H-indol-3-yl)ethanamin (H-2)

Zur Zeit der Synthese kommerziell erhältlich bei Fluorochem.

### Synthese der Ketonbausteine (E)

### Baustein E-1:

### Dimethyl-(8-phenyl-1,4-dioxaspiro[4.5]dec-8-yl)amin-hydrochlorid (D-1)

Zu einer 1,82M Phenylmagnesiumchlorid-Lösung in THF (109 ml, 0,198 mol) wurde unter Argon und Eiskühlung innerhalb von 15 min das Aminonitril B-1 (21 g, 0,1 mol), gelöst in THF (210 ml), hinzugefügt und dann 16 h bei Raumtemperatur gerührt. Zur Aufarbeitung des Reaktionsgemisches wurde unter Eiskühlung gesättigte Ammoniumchloridlösung (150 ml) zugegeben und mit Diethylether (3 × 100 ml) extrahiert. Die organische Phase wurde mit Wasser (100 ml) und gesättigter NaCI-Lösung (100 ml) ausgeschüttelt und eingeengt. Es blieb ein gelbes Öl (25,2 g) zurück. Das Rohprodukt wurde in Ethylmethylketon (280 ml) gelöst und unter Eiskühlung mit ClSiMe₃ (18,8 ml, 0,15 mol) versetzt. Nach einer Reaktionszeit von 6 h konnte das Hydrochlorid D-1 in einer Ausbeute von 35 % (10,5 g) als weißer Feststoff isoliert werden.

### 4-Dimethylamino-4-phenylcyclohexanon (E-1)

Das Hydrochlorid D-1 (10,5 g, 35,2 mmol) wurde in 7,5N Salzsäure (36 ml) gelöst und 96 h bei Raumtemperatur gerührt. Nach beendeter Hydrolyse wurde die Reaktionsmischung mit Diethylether extrahiert (2 × 50 ml). Die wäßrige Phase wurde unter Eiskühlung mit 5N Natronlauge alkalisch gemacht, mit Dichlormethan (3 × 50 ml) extrahiert und eingeengt. Das Keton 6 konnte so als gelber Feststoff mit einem Schmelzpunkt von 104-108 °C in einer Ausbeute von 97 % (7,4 g) isoliert werden.

### Baustein E-2:

### Variante 1:

### [8-(3-Fluorphenyl)-1,4-dioxaspiro[4.5]dec-8-yl]dimethylamin-hydrochlorid (D-2)

Zu einer Lösung des Aminonitrils B-1 (19,8 g, 94 mmol) in THF (100 ml) wurde unter Argon und Eiskühlung innerhalb von 15 min 0,5M 3-Fluorphenylmagnesiumbromid-Lösung in THF (3, 750 ml, 375 mmol) hinzugefügt und dann 16 h bei Raumtemperatur gerührt. Zur Aufarbeitung des Reaktionsgemisches wurde unter Eiskühlung gesättigte Ammoniumchloridlösung (150 ml) und Wasser (60 ml) zugegeben und mit Diethylether (3 × 100 ml) extrahiert. Die organische Phase wurde mit Wasser (50 ml) und gesättigter NaCI-Lösung (50 ml) ausgeschüttelt und eingeengt. Es blieb ein braunes Öl (26,5 g) zurück, das außer der Phenylverbindung 4 noch das Ketal 2 enthielt. Das Rohprodukt wurde in Ethylmethylketon (156 ml) gelöst und unter Eiskühlung mit ClSiMe₃ (17,8 ml, 141 mmol) versetzt. Nach einer Reaktionszeit von 6 h konnte das Hydrochlorid D-2 in einer Ausbeute von 55 % (16,3 g) als weißer Feststoff mit einem Schmelzpunkt von 275-278 °C isoliert werden.

### Variante 2:

### [8-(3-Fluor-phenyl)-1,4-dioxa-spiro[4.5]dec-8-yl]-dimethyl-amin Hydrochlorid (D-2)

Eine Lösung von 1-Brom-3-fluorbenzol (5.00 g, 28.6 mmol) in abs. Ether (15 mL) wurde zu einer Suspension von Magnesium (694 mg, 28.6 mmol) in abs. Ether (10 mL) so getropft, dass der Ether siedete. Nach beendeter Zugabe wurde 10 min bei RT nachgerührt, das Magnesium war danach vollständig gelöst. Die Reaktionslösung wurde im Eisbad abgekühlt und bei 10 °C tropfenweise das Aminonitril B-1 (3.00 g, 14.3 mmol) in abs. THF (30 mL) zugegeben. Der Ansatz rührte bei Raumtemperatur über Nacht, das Reaktionsgemisch wurde unter Eiskühlung mit 20 %iger NH₄Cl-Lösung (20 mL) und Wasser (30 mL) versetzt und mit Ether (3 x 50 mL) extrahiert. Die org. Phase wurde mit Wasser (50 mL) und anschließend mit gesättigter NaCI-Lösung (50 mL) gewaschen, über Na₂SO₄ getrocknet und i. Vak. eingeengt. Das Rohprodukt wurde in Ethylmethylketon (25 mL) gelöst, unter Eiskühlung mit CISiMe₃ (3.2 mL, 25 mmol) versetzt und bei Raumtemperatur 5 h gerührt. Der entstandene Niederschlag wurde abfiltriert und i. Vak. getrocknet.
Ausbeute D-2: 2.8 g (62 %)
¹H-NMR (DMSO-d₆): 1.91 (8 H, m); 2.54 (6 H, s); 3.91 (4 H, d); 7.37 (1 H, m); 7.61 (3 H, m).

### Variante 1:

### 4-Dimethylamino-4-(3-fluor-phenyl)-cyclohexanon (E-2)

Das Hydrochlorid D-2 (7,2 g, 22,75 mmol) wurde in Wasser (9,6 ml) gelöst, mit konzentrierter Salzsäure (14 ml, 455 mmol) versetzt und 4 d bei Raumtemperatur gerührt. Nach beendeter Hydrolyse wurde die Reaktionsmischung mit Diethylether extrahiert (2 × 50 ml), die wäßrige Phase unter Eiskühlung mit 5N Natronlauge alkalisch gemacht, wobei das Produkt ausfiel. Das Keton E-2 konnte als gelber Feststoff mit einem Schmelzpunkt von 83-88 °C und einer Ausbeute von 50 % (6,05 g) isoliert werden.

### Variante 2:

### 4-Dimethylamino-4-(3-fluor-phenyl)-cyclohexanon (E-2)

Das Hydrochlorid D-2 (2.80 g, 8.86 mmol) wurde in Wasser (3.7 mL) gelöst, mit konzentrierter Salzsäure (5.5 mL) versetzt und bei RT 4 d gerührt. Nach beendeter Hydrolyse wurde die Reaktionsmischung mit Ether (2 x 10 mL) extrahiert, die wässrige Lösung unter Eiskühlung mit 5N Natronlauge alkalisch gestellt, die Reaktionsmischung mit Dichlormethan (3 x 50 mL) extrahiert, die organische Phase über Natriumsulfat getrocknet und i. Vak. eingeengt. Das Rohprodukt wurde durch Flash-Chromatographie mit CHCl₃/MeOH (20:1) gereinigt.
Ausbeute E-2: 676 mg (32 %), farbloser Feststoff
Schmelzpunkt: 62-67 °C
¹H-NMR (DMSO-d₆): 2.02 (6 H, s); 2.12 (5 H, m); 2.45 (3 H, m); 7.24 (3 H, m); 7.43 (1 H, m).

### Baustein E-3:

### [8-(4-Fluorphenyl)-1,4-dioxaspiro[4.5]dec-8-yl]dimethylamin-hydrochlorid (D-3)

Zu einer Lösung des Aminonitrils B-1 (10,5 g, 50 mmol) in THF (150 ml) wurde unter Argon und Eiskühlung innerhalb von 15 min 1M 4-Fluorphenylmagnesiumbromid-Lösung in THF (3, 125 ml, 125 mmol) hinzugefügt und dann 16 h bei Raumtemperatur gerührt. Zur Aufarbeitung des Reaktionsgemisches wurde unter Eiskühlung gesättigte Ammoniumchloridlösung (37 ml) und Wasser (50 ml) zugegeben und mit Diethylether (3 × 100 ml) extrahiert. Die organische Phase wurde mit Wasser (50 ml) und gesättigter NaCI-Lösung (50 ml) ausgeschüttelt und eingeengt. Es blieb ein braunes Öl (12,55 g) zurück, das außer der Phenylverbindung C-3 noch das Ketal B-1 enthielt. Das Rohprodukt wurde in Ethylmethylketon (75 ml) gelöst und unter Eiskühlung mit CISiMe₃ (9,5 ml, 75 mmol) versetzt. Nach einer Reaktionszeit von 6 h konnte das Hydrochlorid D-3 in einer Ausbeute von 47 % (7,48 g) als weißer Feststoff isoliert werden.

### 4-Dimethylamino-4-(4-fluorphenyl)cyclohexanon (E-3)

Das Hydrochlorid D-3 (7,2 g, 22,75 mmol) wurde in Wasser (9,6 ml) gelöst, mit konzentrierter Salzsäure (14 ml, 455 mmol) versetzt und 4 d bei Raumtemperatur gerührt. Nach beendeter Hydrolyse wurde die Reaktionsmischung mit Diethylether extrahiert (2 × 50 ml), die wäßrige Phase unter Eiskühlung mit 5N Natronlauge alkalisch gemacht, mit Dichlormethan (3 × 50 ml) extrahiert und eingeengt. Das Keton E-3 konnte als gelber Feststoff mit einem Schmelzpunkt von 128-133 °C und einer Ausbeute von 76 % (4,05 g) isoliert werden.

### Baustein E-4:

### Dimethyl-(8-thiophen-2-yl-1,4-dioxaspiro[4.5]dec-8-yl)amin-hydrochlorid (D-4)

2-lodthiophen (1, 22,9 g, 109 mmol) wurde unter Argon in THF (80 ml) gelöst und innerhalb von 30 min bei 0 °C mit 2M Isopropylmagnesiumchlorid (2, 35,7 ml, 72 mmol) in THF versetzt. Nach einer Reaktionszeit von 1 h bei 3-5 °C wurde das Aminonitril B-1 (10 g, 47,6 mmol), gelöst in Tetrahydrofuran (20 ml), hinzugefügt und 20 h bei Raumtemperatur gerührt. Die Aufarbeitung des Ansatzes erfolgte durch Zugabe von gesättigter NH₄Cl-Lösung (85 ml) und Extraktion mit Diethylether (3×100 ml). Die organische Phase wurde mit Wasser (50 ml) und gesättigter NaCl-Lösung (50 ml) ausgeschüttelt und eingeengt. Es konnte ein dunkelbraunes Öl (21,3 g) erhalten werden, das außer dem gewünschten Ketal das Aminonitril B-1 und 2-lodthiophen enthielt. Das Rohprodukt wurde in Ethylmethylketon (140 ml) gelöst und mit CISiMe₃ (9,1 ml, 71,4 mmol) versetzt. Nach einer Reaktionszeit von 6 h wurde das Hydrochlorid D-4 als weiße kristalline Verbindung in einer Ausbeute von 60 % (8,74 g) isoliert.

### 4-Dimethylamino-4-thiophen-2-ylcyclohexanon (E-4)

Das Hydrochlorid D-4 (8,68 g, 28,6 mmol) wurde in 7,5N Salzsäure (29 ml) gelöst und 48 h bei Raumtemperatur gerührt. Nach beendeter Hydrolyse wurde die Reaktionsmischung mit Diethylether extrahiert (2 × 50 ml). Die wäßrige Phase wurde unter Eiskühlung mit 5N Natronlauge alkalisch gemacht, mit Dichlormethan (3 × 50 ml) extrahiert und eingeengt. Das Keton E-4 wurde so als gelber Feststoff mit einem Schmelzpunkt von 108-110 °C in einer Ausbeute von 89 % (5,66 g) erhalten.

### Baustein E-5:

### N,N-dimethyl-8-(thiophen-3-yl)-1,4-dioxaspiro[4.5]decan-8-amin (D-5)

3-lodthiophen (1, 5 g, 23,8 mmol) wurde unter Argon in THF (18 ml) gelöst und innerhalb von 8 min bei 0 °C mit 2M Isopropylmagnesiumchlorid (2, 7,8 ml, 15,5 mmol) in THF versetzt. Nach einer Reaktionszeit von 1 h bei 3-5 °C wurde das Aminonitril B-1 (2,16 g, 10,3 mmol), gelöst in Tetrahydrofuran (20 ml), hinzugefügt. Anschließend wurde 20 h bei Raumtemperatur gerührt. Die Aufarbeitung des Ansatzes erfolgte durch Zugabe von gesättigter NH₄Cl-Lösung (20 ml) und Extraktion mit Diethylether (3 × 50 ml). Die organische Phase wurde mit Wasser (20 ml) und gesättigter NaCI-Lösung (20 ml) ausgeschüttelt und eingeengt. Es wurde ein hellbraunes Öl (3,95 g) erhalten. Das Rohprodukt wurde in Ethylmethylketon (40 ml) gelöst und mit ClSiMe₃ (1,95 ml, 15,5 mmol) versetzt. Nach einer Reaktionszeit von 3 h konnte das gewünschte Hydrochlorid als weiße kristalline Verbindung in einer Ausbeute von 60 % (1,86 g) mit einem Schmelzpunkt von 250-251 °C isoliert werden.

### 4-(Dimethylamino)-4-(thiophen-3-yl)cyclohexanon (E-5)

Das Hydrochlorid D-5 (1,8 g, 5,9 mmol) wurde in 7,5N Salzsäure (7 ml) gelöst und 48 h bei Raumtemperatur gerührt. Nach beendeter Hydrolyse wurde die Reaktionsmischung mit Diethylether extrahiert (2 × 30 ml), unter Eiskühlung die wäßrige Phase mit 5N Natronlauge alkalisch gemacht, mit Dichlormethan (3 × 30 ml) extrahiert und eingeengt. Das Keton E-5 konnte als gelber Feststoff mit einem Schmelzpunkt von 147-150 °C und einer Ausbeute von 98 % (1,27 g) isoliert werden.

### Synthese der Spiroamin Bausteine (AMN^{cis} / AMN^{trans})

### Vergleichsbeispiel AMN-1^{cis.}

### 2',3',4',9'-Tetrahydro-N,N-dimethyl-4-(phenyl)-spiro[cyclohexan-1,1'(1'H)-pyrido[3,4-b]indol]-4-amin (Cis-Diastereomer)

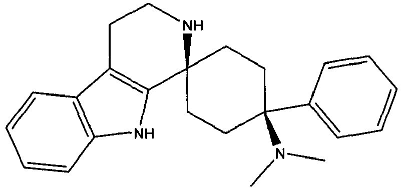

*Anmerkung:* Nach dieser Vorschrift wird vorwiegend das cis-Produkt AMN-1*^{cis}* erhalten. Das trans-Produkt AMN-1*^{trans}* fällt nur als Nebenprodukt bzw. verunreinigt an.

Das Keton E-1 (3,26 g, 15 mmol) und Tryptamin H-1 (2,4 g, 15 mmol) wurden unter Ausschluß von Sauerstoff in trockenem MeOH (100 ml) gelöst. Zu diesem Gemisch wurde Natriumsulfat (3 g) hinzugefügt. Nach einer Reaktionszeit von 17 h wurde das Lösungsmittel am Rotationsverdampfer abdestilliert und der Rückstand in 1,2-Dichlorethan (100 ml) aufgenommen. Die Reaktionsmischung wurde mit Trifluoressigsäure (15 ml) versetzt und 1 h bei Raumtemperatur gerührt. Der Verlauf der Reaktion wurde durch DC verfolgt. Zur Aufarbeitung wurde der Ansatz mit H₂O (40 ml) versetzt und mit NaOH (5 mol/l) auf pH 11 eingestellt. Dabei fiel ein weißer Feststoff aus, der über eine Fritte abgesaugt wurde. Der Feststoff wurde mit H₂O (3 × 5 ml) gewaschen und getrocknet. Es handelte sich dabei um das cis-Produkt AMN-1*^{cis}*, welches als weißer Feststoff mit einem Schmelzpunkt von 214-218 °C in einer Ausbeute von 4 g (74 %) erhalten wurde. Die Mutterlauge (wäßrige Phase) wurde mit 1,2-Dichlorethan (3 × 25 ml) extrahiert. Die organische Phase wurde mit Na₂SO₄ getrocknet und eingeengt. Der feste braune Rückstand wurde aus MeOH (10 ml) umkristallisiert und ergab ein Gemisch aus cis-AMN-1*^{cis}* und trans- AMN-1*^{trans}* Spiroamin (1 : 1). Das Gemisch wurde als weißer Feststoff in einer Ausbeute von 940 mg (17 %) erhalten werden.
¹H NMR (600 MHz, DMSO-*d*₆): 1,61 (m, 2 H) 1,63 (m, 2 H) 1,92 (s, 6 H) 2,12 (m, 2 H) 2,39 (m, 2 H) 2,53 (t, *J* = 5,36 Hz, 2 H) 2,99 (t, *J* = 5,35 Hz, 2 H) 6,86 (m, 1 H) 6,91 (m, 1 H) 7,16 (d, *J* = 7,52 Hz, 1 H) 7,28 (d, *J* = 7,52 Hz, 1 H) 7,31 (m, 1 H) 7,43 (m, 4 H) 10,21 (s, 1 H)

### Vergleichsbeispiel AMN-2^{cis.}

### 2',3',4',9'-Tetrahydro-N,N-dimethyl-4-(3-fluorophenyl)-spiro[cyclohexan-1,1'(1'H)-pyrido[3,4-b]indol]-4-amin (Cis-Diastereomer)

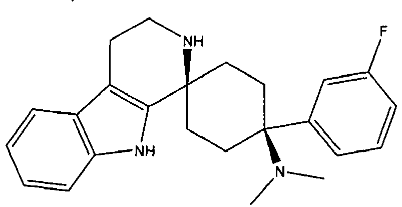

Das Keton E-2 (4,71 g, 20 mmol) und Tryptamin H-1 (3,2 g, 20 mmol) wurden unter Argon in trockenem MeOH (200 ml) gelöst. Nach einer Reaktionszeit von 24 h wurde MeOH abdestilliert und der gelbe, ölige Rückstand in 1,2-Dichlorethan (200 ml) suspendiert. Die Reaktionsmischung wurde mit Trifluoressigsäure (20 ml) versetzt und 2 h bei Raumtemperatur gerührt. Der Verlauf der Reaktion wurde durch DC verfolgt. Zur Aufarbeitung wurde der Ansatz mit H₂O (100 ml) verdünnt und mit NaOH (5 mol/l) auf pH 11 eingestellt. Nach Zugabe von Ethylacetat (50 ml) fiel beim Rühren ein weißer Feststoff aus, der über eine Fritte abgesaugt wurde. Der Feststoff wurde mit H₂O (3 × 25 ml) gewaschen und anschließend getrocknet. Es handelte sich dabei um das cis-Diastereomere AMN-2*^{cis}*, welches als weißer Feststoff mit einem Schmelzpunkt von 220-225 °C in einer Ausbeute von 5,5 g (73 %) erhalten wurde.
¹H NMR (600 MHz, DMSO-*d*₆): 1,61 (m, 2 H) 1,62 (m, 2 H) 1,93 (s, 6 H) 2,11 (m, 2 H) 2,38 (m, 2 H) 2,53 (t, *J* = 5,56 Hz, 2 H) 2,99 (t, *J* = 5,56 Hz, 2 H) 6,87 (m, 1 H) 6,92 (m, 1 H) 7,14 (m, 1 H) 7,17 (d, *J* = 8,34 Hz, 1 H) 7,20 (m, 1 H) 7,25 (d, *J* = 7,82 Hz, 1 H) 7,28 (d, *J* = 7,47 Hz, 1 H) 7,47 (m, 1 H) 10,26 (s, 1 H)

### Vergleichsbeispiel AMN-2^{trans.}

### 2',3',4',9'-Tetrahydro-N,N-dimethyl-4-(3-fluorophenyl)-spiro[cyclohexan-1,1'(1'H)-pyrido[3,4-b]indol]-4-amin (Trans-Diastereomer)

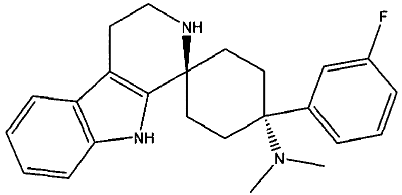

Tryptamin H-1 (2,03 g, 12,7 mmol) und das Keton (E-2, 3,0 g, 12,7 mmol) wurden in abs. Methanol (130 ml) gelöst und 16 h bei Raumtemperatur unter Argon gerührt. Die Reaktionsmischung wurde anschließend eingeengt. Der Rückstand wurde in abs. 1,2-Dichlorethan (130 ml) gelöst, schnell mit Trifluoressigsäure (12,7 ml) versetzt und 2 h bei Raumtemperatur gerührt. Unter Eiskühlung wurden Wasser (120 ml) und 5N Natronlauge (40 ml) hinzugefügt und 1 h gerührt. Der dabei entstandene farblose Feststoff, wurde durch Filtration abgetrennt und mit 1,2-Dichlorethan (30 ml) und Wasser (4 × 25 ml) gewaschen. Das cis-Spiroamin AMN-2*^{cis}* wurde in einer Ausbeute von 77 % (3,7 g) mit Spuren des trans-Spiroamins AMN-2*^{trans}* erhalten. Die Phasen des Filtrats wurden getrennt. Die organische Phase wurde mit Natriumsulfat getrocknet, eingeengt, mit Methanol (3 ml) versetzt und 1 h bei Raumtemperatur gerührt. Dabei fiel ein weißer Feststoff aus, der durch Filtration abgetrennt und mit Methanol (4 × 3 ml) gewaschenwurde. Das trans-Spiroamin AMN-2*^{trans}* wurde dabei in einer Ausbeute von 5 % (250 mg) mit Spuren des cis-Spiroamins AMN-2*^{cis}* erhalten. Nach chromatographischer Reinigung [Kieselgel 60 (20 g); Methanol (200 ml)] wurde das trans-Spiroamin AMN-2*^{trans}* (170 mg) mit einem Schmelzpunkt von 296-299 °C erhalten .
¹H NMR (600 MHz, DMSO-*d*₆): 1,55 (m, 2 H) 1,62 (m, 2 H) 1,88 (s, 6 H) 2,26 (m, 2 H) 2,43 (m, 2 H) 2,55 (t, *J* = 5,49 Hz, 2 H) 2,96 (t, *J* = 5,25 Hz, 2 H) 6,91 (m, 1 H) 6,99 (m, 1 H) 7,08 (m, 1 H) 7,14 (m, 1 H) 7,20 (d, *J* = 7,64 Hz, 1 H) 7,32 (m, 2 H) 7,40 (m, 1 H) 10,63 (s, 1 H)

### Vergleichsbeispiel AMN-3^{cis}.

### 6'-Fluoro-2',3',4',9'-Tetrahydro-N,N-dimethyl-4-(3-fluorphenyl)-spiro[cyclohexan-1,1'(1'H)-pyrido[3,4-b]indol]-4-amin (Cis-Diastereomer)

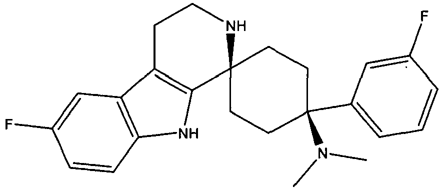

Das Keton E-2 (9.6 g, 41.2 mmol) und Fluortryptamin H-2 (7.3 g, 41.2 mmol) wurden in Ethanol (200 ml) gelöst und für 12 Stunden zum Rückfluß erhitzt. Anschließend wurde das Ethanol abdestilliert und das Rohprodukt in 1,2-Dichlorethan (100 ml) suspendiert. Die Reaktionsmischung wurde mit Trifluoressigsäure (90 ml) versetzt und 12 h bei Raumtemperatur gerührt. Der Verlauf der Reaktion wurde durch DC verfolgt. Zur Aufarbeitung wurde der Ansatz mit 500 ml 1N NaOH-Lösung bei 0°C basisch gestellt und anschließend 3x mit 500 ml Ethylacetat extrahiert. Die vereinigten organischen Phasen wurden über Magensiumsulfat getrocknet, und unter vermindertem Druck konzentriert. Nach Zugabe von Methanol (100 ml) fiel beim Rühren ein weißer Feststoff aus, der über eine Fritte abgesaugt wurde. Der Feststoff wurde mit Methanol (2 × 25 ml) gewaschen und anschließend getrocknet. Es handelte sich dabei um das cis-Diastereomere AMN-3*^{cis}*, welches als weißer Feststoff in einer Ausbeute von 3,6 g (22 %) erhalten wurde.
¹H NMR (DMSO-d6, 400 MHz): δ 10.39(s, 1H), 7.44-7.49 (m, 1H), 7.11-7.24 (m, 4H), 7.00-7.04 (m, 1H), 6.72-6.78 (m, 1H), 2.95-2.98 (t, 2H), 2.48-2.50 (m, 1H), 2.36-2.39 (d, 2H), 1.98-2.11 (m, 2H), 1.91 (s, 6H), 1.51-1.67 (m, 5H)
MS m/z (M+1): 396.4; Purity (HPLC): 95.03%

### Vergleichsbeispiel AMN-4^{cis}.

### 6'-Fluoro-2',3',4',9'-Tetrahydro-N,N-dimethyl-4-(phenyl)-spiro[cyclohexan-1,1'(1'H)-pyrido[3,4-b]indol]-4-amin (Cis-Diastereomer)

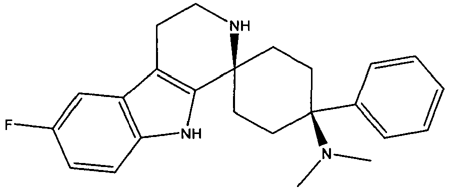

Das Keton E-1 (8.4 g, 47 mmol) und Fluortryptamin H-2 (10.2g, 47 mmol) wurden in Ethanol (200 ml) gelöst und für 12 Stunden zum Rückfluß erhitzt. Anschließend wurde das Ethanol abdestilliert und das Rohprodukt in 1,2-Dichlorethan (120 ml) suspendiert. Die Reaktionsmischung wurde mit Trifluoressigsäure (100 ml) versetzt und 12 h bei Raumtemperatur gerührt. Der Verlauf der Reaktion wurde durch DC verfolgt. Zur Aufarbeitung wurde der Ansatz mit 1N NaOH-Lösung bei 0°C basisch gestellt und anschließend 3x mit 500 ml Ethylacetat extrahiert. Die vereinigten organischen Phasen wurden über Magensiumsulfat getrocknet, und unter vermindertem Druck konzentriert. Nach Zugabe von Methanol (100 ml) fiel beim Rühren ein weißer Feststoff aus, der über eine Fritte abgesaugt wurde. Der Feststoff wurde mit Methanol (2 × 25 ml) gewaschen und anschließend getrocknet. Es handelte sich dabei um das cis-Diastereomere AMN-4*^{cis}*, welches als weißer Feststoff in einer Ausbeute von 4 g (28 %) erhalten wurde.
¹H NMR (DMSO-d₆, 400 MHz): δ 10.36(s, 1H), 7.45-7.42 (t, 4H), 7.32-7.29 (m, 1H), 7.14-7.10 (m, 1H), 7.03-7.00 (m, 1H), 6.76-6.71 (m, 1H), 2.99-2.96 (t, 2H), 2.40-2.37 (d, 2H), 2.13-2.04 (m, 2H), 1,91 (s, 6H), 1.88 (s, 1H), 1.65-1.54 (m, 4H), 1.23 (s, 1H).

### Vergleichsbeispiel AMN-5^{cis}.

### 2',3',4',9'-Tetrahydro-N,N-dimethyl-4-(4-fluorophenyl)-spiro[cyclohexan-1,1'(1'H)-pyrido[3,4-b]indol]-4-amin (Cis-Diastereomer)

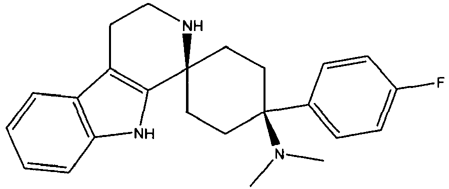

Das Keton E-3 (2800 mg, 11,90 mmol) und Tryptamin (H-1, 1910 mg, 11,90 mmol) wurden unter Argon in trockenem Methanol (119 ml) gelöst und 18 h gerührt. Anschließend wurde das Methanol im Vakuum abdestilliert und der Rückstand in 1,2-Dichlorethan (119 ml) suspendiert. Die Reaktionsmischung wurde mit Trifluoressigsäure (11,9 ml) versetzt und 2 h bei Raumtemperatur gerührt. Dann wurde die Reaktionsmischung mit 1,2-Dichlorethan (119 ml) verdünnt und unter Eiskühlung mit 1N Natriumhydroxid-Lösung auf pH 11 eingestellt. Es entstand ein heller Niederschlag. Die Mischung wurde über Nacht bei Raumtemperatur gerührt. Der Niederschlag wurde abgesaugt, mit Wasser gewaschen und im Vakuum getrocknet. Das cis-Diastereomer AMN-5*^{cis}* (Smp. 249-250 °C, teilweise schon bei 225-230 °C) konnte in einer Ausbeute von 80 % (3610 mg, 9,56 mmol) isoliert werden. - Die Phasen wurden getrennt. Die organische Phase wurde mit Natriumsulfat getrocknet, filtriert und im Vakuum von flüchtigen Bestandteilen befreit. Der helle Rückstand (trans-Diastereoisomer AMN-5*^{trans}*) wurde in Methanol (5 ml) aufgenommen und 48 h gerührt. Der Niederschlag wurde abfiltriert und im Vakuum getrocknet. Das trans-Diastereoisomer AMN-5*^{trans}* (268-271 °C) konnte in einer Ausbeute von 6 % (279 mg, 0,74 mmol) isoliert werden.
¹³C{¹H}-NMR (101 MHz, DMSO-*D*₆) δ ppm: 22.8 (1 C), 27.3 (2 C), 32.6 (2 C), 37.8 (2 C), 38.6 (1 C), 51.2 (1 C), 60.5 (1 C), 106.7 (1 C), 110.8 (1 C), 114.2 (2 C, d, J = 21 Hz), 117.2 (1 C), 117.9 (1 C), 120.0 (1 C), 126.9 (1 C), 129.7 (2 C, d, J = 8 Hz), 132.8 (1 C, d, J = 3 Hz), 135.4 (1 C), 141.4 (1 C), 160.7 (1 C, d, J = 242 Hz)

### Synthese der Cis Spiroamid Beispiele (AMD^{cis})

### Beispiel AMD-1^{cis}:

### (E)-2',3',4',9'-tetrahydro-N,N-dimethyl-4-phenyl-2'-(2-phenylvinyl)carbonyl-spiro[cyclohexan-1,1'(1'H)-pyrido[3,4-b]indol]-4-amin Methansulfonat (1:1) (Cis-Diastereomer)

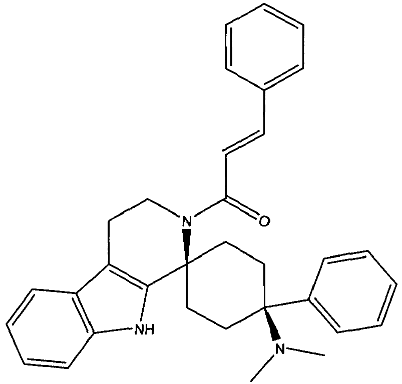

AMN-1*^{cis}* wurde in THF (8 ml) gelöst. Danach wurde Zimtsäurechlorid (254 mg, 1,53 mmol) und Diisopropylethylamin (216 mg, 1,67 mmol) zugegeben und 2 d bei RT gerührt. Nach beendeter Reaktion wurde der Feststoff abfiltriert und das Filtrat mit gesättigter Na₂CO₃-Lösung versetzt. Die wässrige Phase wurde dreimal mit je 10 ml Ethylacetat extrahiert. Anschließend wurde die organische Phase über MgSO₄ getrocknet und am Rotationsverdampfer eingeengt. Das Rohprodukt wurde säulenchromatographisch aufgereinigt [Kieselgel 60; DCM/Methanol (19 : 1, 570 ml)]. Das Produkt wurde in einer Ausbeute von 174 mg (26 %) erhalten. Zur Herstellung des Methansulfonates wurde das soeben erhaltene Spiroamid (174 mg, 0,355 mmol) in DCM (6 ml) suspendiert und bei RT mit Methansulfonsäure (23,7 µl, 0,355 mmol) versetzt. Anschließend wurde Aceton (0,8 ml) zugegeben und soviel Diethylether zugegeben, dass die auftretende Trübung durch schütteln gerade aufgelöst wurde. Es wurde weitere 30 min. gerührt und der entstandene Feststoff anschließend unter Luftausschluß abgesaugt, mit Diethylether gewaschen und im Ölpumpenvakuum bei 50°C für 3h getrocknet. Das Produkt AMD-1*^{cis}* wurde in einer Ausbeute von 159 mg (76 %) erhalten.
¹H NMR (600 MHz, DMSO-*d*₆) 1,65 (t, *J* =13,22 Hz, 2 H) 2,20 (t, *J* = 12,84 Hz, 2 H) 2,51 (d, *J* = 4,53 Hz, 9 H) 2,87 - 3,16 (m, 4 H) 4,13 (br. s., 2 H) 6,92 (t, *J* = 7,55 Hz, 1 H) 6,99 (t, *J* = 7,55 Hz, 1 H) 7,20 (d, *J* = 8,31 Hz, 1 H) 7,31 (d, *J*= 7,55 Hz, 1 H) 7,36 - 7,51 (m, 5 H) 7,56 - 7,69 (m, 3 H) 7,74 (d, *J* = 7,55 Hz, 2 H) 7,82 (d, *J* = 7,55 Hz, 2 H) 9,62 (br, s, 1 H)

### Vergleichsbeispiel AMD-2^{cis}.

### 2',3',4',9'-Tetrahydro-N,N-dimethyl-4-(3-fluor-phenyl)-2'-(4-chlorbenzyl)-carbonyl-spiro-[cyclohexan-1,1'(1'H)-pyrido[3,4-b]indol]-4-amin (Cis-Diastereomer)

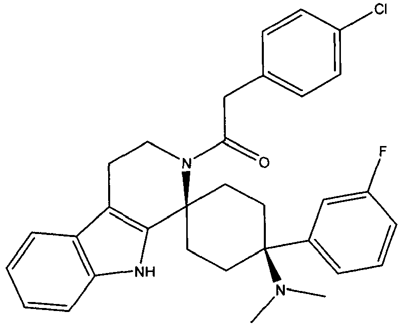

Das Spiroamin (AMN-2*^{cis}*; 396 mg, 1,05 mmol) wurde in DCM (15 ml) in einem mikrowellengeeigneten Gefäß suspendiert und mit 2-(4-Chlorophenyl)acetylchlorid (397 mg, 2,1 mmol) und und Diisopropylethylamin (269 mg, 2,1 mmol) versetzt. Der Reaktionsansatz wurde bei 120°C für 10 min. in der Mikrowelle (Initiator Eight, Firma Biotage) bestrahlt. Nach beendeter Reaktion (DC-Kontrolle) wurde der Reaktionsansatz zunächst filtriert, mit Diethylether (15 ml) versetzt und erneut filtriert. Es wurde mit gesättigter Na₂CO₃-Lösung (8 ml) versetzt. Nach trennen der Phasen wurde die wässrige Phase noch einmal mit DCM gewaschen. Die vereinigten organischen Phasen wurden über MgSO₄ getrocknet und am Rotationsverdampfer eingeengt. Das Rohprodukt wurde säulenchromatographisch aufgereinigt [Kieselgel 60; DCM/Methanol (19 : 1)]. Das Produkt AMD-2*^{cis}* wurde in einer Ausbeute von 91 mg (16 %) erhalten.
¹H NMR (600 MHz, DMSO-*d*₆) d ppm 1,55 (t, *J* = 13,60 Hz, 2 H) 1,79 (t, *J* = 12,84 Hz, 2 H) 1,92 (br. s., 6 H) 2,60 - 2,70 (m, 2 H) 2,73 - 2,87 (m, 2 H) 3,17 (d, *J* = 5,29 Hz, 2 H) 3,89 - 4,01 (m, 4 H) 6,90 (t, *J* = 7,55 Hz, 1 H) 6,97 (t, *J* = 7,55 Hz, 1 H) 7,08 - 7,16 (m, 1 H) 7,18 (d, *J* = 8,31 Hz, 1 H) 7,21 - 7,30 (m, 3 H) 7,34 (q, *J* = 8,31 Hz, 4 H) 7,46 (q, *J* = 7,30 Hz, 1 H) 10,53 (s, 1 H)

### Vergleichsbeispiel AMD-3^{cis}.

### 2',3',4',9'-Tetrahydro-N,N-dimethyl-4-(3-fluorphenyl)-2'-(benzothiophen-2-yl)carbonyl-spiro[cyclohexan-1,1'(1'H)-pyrido[3,4-b]indol]-4-amin (Cis-Diastereomer)

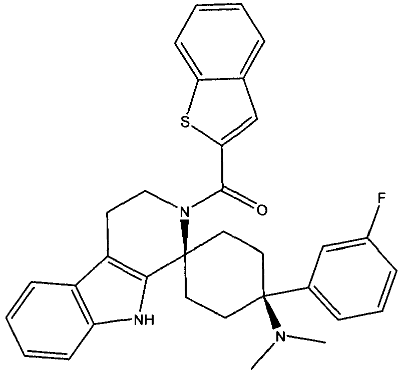

Das Spiroamin (AMN-2*^{cis}* 264 mg, 0,7 mmol) wurde in DCM (7 ml) in einem mikrowellengeeigneten Gefäß suspendiert und mit Benzo[*b*]thiophen-2-carbonylchlorid (239 mg, 1,21 mmol) und Diisopropylethylamin (180 mg, 1,4 mmol) versetzt. Der Reaktionsansatz wurde bei 100°C für 10 min. in.der Mikrowelle (Initiator Eight, Firma Biotage) bestrahlt. Nach beendeter Reaktion (DC-Kontrolle) wurde der Reaktionsansatz mit DCM (15 ml) verdünnt und filtriert. Die Mutterlauge wurde mit gesättigter Na₂CO₃-Lösung (8 ml) versetzt. Nach trennen der Phasen wurde die wässrige Phase noch zweimal mit DCM gewaschen. Die vereinigten organischen Phasen wurden über MgSO₄ getrocknet und am Rotationsverdampfer eingeengt. Das Rohprodukt wurde säulenchromatographisch aufgereinigt [Kieselgel 60; DCM/Methanol (19 : 1)]. Das Produkt AMD-3*^{cis}* wurde in einer Ausbeute von 125 mg (33 %) erhalten.
¹H NMR (600 MHz, DMSO-*d*₆) d ppm 1,63 - 1,79 (m, 2 H) 1,83 - 1,93 (m, 2 H) 1,95 (s, 6 H) 2,60 (d, *J* = 13,60 Hz, 2 H) 2,65 (t, *J* = 5,67 Hz, 2 H) 2,78 - 2,94 (m, 2 H) 4,08 - 4,22 (m, 2 H) 6,92 (t, *J* = 7,55 Hz, 1 H) 6,99 (t, *J* = 7,55 Hz, 1 H) 7,16 (t, *J* = 8,31 Hz, 1 H) 7,23 (d, *J* = 8,31 Hz, 1 H) 7,26 - 7,36 (m, 3 H) 7,44 - 7,54 (m, 3 H) 7,95 (s, 1 H) 8,03 (d, *J* = 7,55 Hz, 1 H) 8,07 (d, *J* = 8,31 Hz, 1 H) 10,66 (s, 1 H)

### Vergleichsbeispiel AMD-4^{cis}.

### 2',3',4',9'-Tetrahydro-N,N-dimethyl-4-(3-fluor-phenyl)-2'-(4-fluorbenzyl)-carbonyl-spiro[cyclohexan-1,1'(1'H)-pyrido[3,4-b]indol]-4-amin Methansulfonat (Cis-Diastereomer)

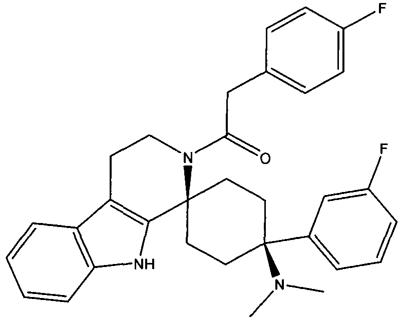

Das Spiroamin (AMN-2*^{cis}*;600 mg, 1,59 mmol) wurde in DCM (15 ml) in einem mikrowellengeeigneten Gefäß suspendiert und mit 2-(4-Fluorophenyl)acetylchlorid (548 mg, 3,18 mmol) und Diisopropylethylamin (408 mg, 3,18 mmol) versetzt. Der Reaktionsansatz wurde bei 130°C für 10 min. in der Mikrowelle (Initiator Eight, Firma Biotage) bestrahlt. Nach beendeter Reaktion (DC-Kontrolle) wurde der Reaktionsansatz zunächst filtriert, die Mutterlauge mit DCM (45 ml) verdünnt und mit gesättigter Na₂CO₃-Lösung (25 ml) versetzt. Nach trennen der Phasen wurde die organische Phase erneut mit gesättigter Na₂CO₃-Lösung gewaschen. Die organische Phase wurde über MgSO₄ getrocknet und am Rotationsverdampfer eingeengt. Das Rohprodukt wurde säulenchromatographisch aufgereinigt [Kieselgel 60; DCM/Methanol (4 : 1)]. Das Produkt wurde in einer Ausbeute von 150 mg (18 %) erhalten. Zur Herstellung des Methansulfonates wurde das Spiroamid (150 mg, 0,29 mmol) in DCM (1 ml) gelöst und bei RT mit Methansulfonsäure (18,9 µl, 0,29 mmol) versetzt. Es wurde mit Diethylether verdünnt, so dass ein rührbares Gemisch entstand. Der Feststoff wurde unter Luftausschluß abgesaugt, mit Diethylether gewaschen und bei 50°C im Ölpumpenvakuum getrocknet. Das Produkt AMD-4*^{cis}* wurde in einer Ausbeute von 148 mg (83 %) erhalten.
¹H NMR (600 MHz, DMSO-*d*₆) d ppm 1,58 (t, *J* = 12,84 Hz, 2 H) 2,16 (t, *J* = 12,09 Hz, 2 H) 2,31 (s, 3 H) 2,53 - 2,58 (m, 6 H) 2,58 - 2,68 (m, 2 H) 2,83 - 3,03 (m, 4 H) 3,98 (s, 2 H) 3,99 - 4,06 (m, 2 H) 6,92 (t, *J* = 7,18 Hz, 1 H) 6,99 (t, *J* = 7,18 Hz, 1 H) 7,14 (t, *J* = 8,31 Hz, 2 H) 7,18 (d, *J* = 8,31 Hz, 1 H) 7,29 (d, *J* = 7,55 Hz, 1 H) 7,36 (t, *J* = 6,42 Hz, 2 H) 7,45 (t, *J* = 7,93 Hz, 1 H) 7,58 - 7,75 (m, 3 H) 9,65 (br. s., 1 H)

### Beispiel AMD-5^{cis}

### (E)-2',3',4',9'-Tetrahydro-N,N-dimethyl-4-(3-fluorphenyl)-2'-(2-phenylvinyl)carbonyl-spiro[cyclohexan-1,1'(1'H)-pyrido[3,4-b]indol]-4-amin (Cis-Diastereomer)

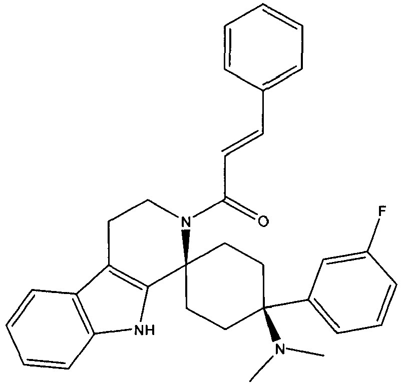

Das Spiroamin (AMN-2*^{cis}*; 378 mg, 1,0 mmol) wurde in trockenem aprotischen Lösungsmittel (6 ml) gelöst, mit Cinnamoylchlorid (183 mg, 1,1 mmol) und Diisopropylethylamin (155 mg, 1,2 mmol) versetzt und über Nacht bei RT gerührt. Nach beendeter Reaktion (DC-Kontrolle) wurde das Lösungsmittel entfernt, der Rückstand wässrig aufgearbeitet und mit halogeniertem Lösungsmittel extrahiert. Die vereinigten organischen Phasen wurden über Na₂SO₄ getrocknet und eingeengt. Das Rohprodukt wurde säulenchromatographisch aufgereinigt. Während des Einengens fiel ein Feststoff aus, der abfiltriert und anschließend getrocknet wurde. Das Produkt wurde in einer Ausbeute von 220 mg (43 %) erhalten.
¹H NMR (600 MHz, DMSO-*d*₆) d ppm 1,63 (t, *J* = 13,60 Hz, 2 H) 1,84 (t, *J* = 13,22 Hz, 2 H) 1,91 (s, 6 H) 2,54 - 2,63 (m, 2 H) 2,65 (t, *J* = 5,67 Hz, 2 H) 2,82 - 3,02 (m, 2 H) 3,17 (d, *J* = 5,29 Hz, 2 H) 4,00 - 4,22 (m, 2 H) 6,90 (t, *J* = 7,18 Hz, 1 H) 6,97 (t, *J* = 7,55 Hz, 1 H) 7,11 - 7,18 (m, 1 H) 7,20 (d, *J* = 8,31 Hz, 1 H) 7,23 - 7,33 (m, 3 H) 7,35 - 7,54 (m, 5 H) 7,72 (d, *J* = 6,80 Hz, 2 H) 10,59 (s, 1 H)

### Beispiel AMD-6^{cis}:

### (E)-2',3',4',9'-Tetrahydro-N,N-dimethyl-4-(3-fluorphenyl)-2'-(2-phenylvinyl)carbonyl-spiro[cyclohexan-1,1'(1'H)-pyrido[3,4-b]indol]-4-amin Citrat (Cis-Diastereomer)

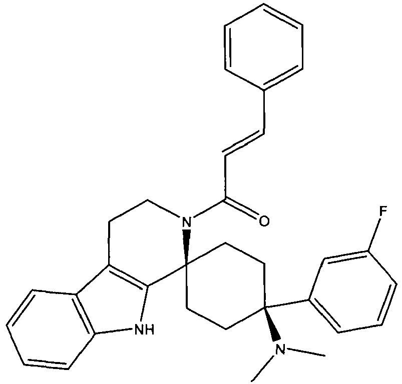

Zur Herstellung des Salzes wurde das Amid AMD-5*^{cis}* (220 mg, 0,43 mmol) in trockenem aprotischen Lösungsmittel (1,5 ml) gelöst und mit Citronensäure (83 mg, 0,43 mmol), gelöst in möglichst wenig protischem Lösungsmittel, versetzt. Zum Ausfällen des Produktes wurde tropfenweise unpolares Lösungsmittel zugegeben. Anschließend wurde der Feststoff unter Luftausschluß abgesaugt und bei 50°C im Ölpumpenvakuum getrocknet. Das Produkt AMD-6*^{cis}* wurde in einer Ausbeute von 100 mg (33 %) erhalten.

### Vergleichsbeispiel AMD-7^{cis}.

### 2',3',4',9'-Tetrahydro-N,N-dimethyl-4-(3-fluorphenyl)-2'-(3,4-dimethoxybenzyl)carbonyl-spiro[cyclohexan-1,1'(1'H)-pyrido[3,4-b]indol]-4-amin (Cis-Diastereomer)

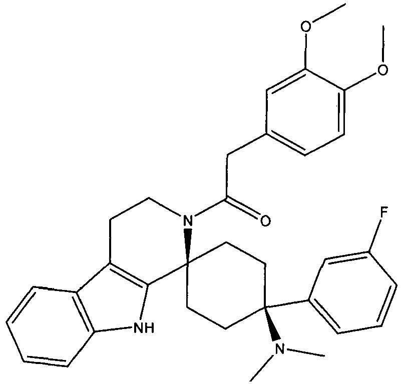

Das Spiroamin (AMN-2*^{cis}*; 200 mg, 0,54 mmol) wurde in halogeniertem Lösungsmittel (5 ml) in einem mikrowellengeeigneten Gefäß suspendiert und mit 2-(3,4-Dimethoxyphenyl)-acetylchlorid (230 mg, 1,1 mmol) und Diisopropylethylamin (138 mg, 1,1 mmol) versetzt. Der Reaktionsansatz wurde bei 120°C für 10 min. in der Mikrowelle (Initiator Eight, Firma Biotage) bestrahlt. Nach beendeter Reaktion (DC-Kontrolle) wurde zunächst filtriert und anschließend die Mutterlauge mit NaOH-Lösung (5 N, 10 ml) versetzt. Nach Trennen der Phasen wurde die wässrige Phase dreimal mit einem polaren, aprotischen Lösungsmittel (je 5 ml) extrahiert. Die vereinigten organischen Phasen wurden über MgSO₄ getrocknet und eingeengt. Das Rohprodukt wurde säulenchromatographisch aufgereinigt. Das Produkt AMD-7*^{cis}* wurde in einer Ausbeute von 140 mg (47 %) erhalten.
¹H NMR (600 MHz, DMSO-*d*₆) d ppm 1,54 (t, *J* = 12,46 Hz, 2 H) 1,79 (t, *J* = 13,22 Hz, 2 H) 1,85 - 1,96 (m, 6 H) 2,52 - 2,60 (m, 2 H) 2,62 - 2,72 (m, 2 H) 2,73 - 2,89 (m, 2 H) 3,74 (s, 3 H) 3,77 (s, 3 H) 3,82 (br. s., 2 H) 3,90 (br. s., 2 H) 6,83 - 6,93 (m, 4 H) 6,97 (t, *J* = 7,55 Hz, 1 H) 7,13 (t, *J* = 7,18 Hz, 1 H) 7,19 (d, *J* = 8,31 Hz, 1 H) 7,20 - 7,32 (m, 3 H) 7,41 - 7,54 (m, 1 H) 10,53 (s, 1 H)

### Beispiel AMD-8^{cis}

### (E)-2',3',4',9'-Tetrahydro-N,N-dimethyl-6'-fluor-4-(3-fluorphenyl)-2'-(2-phenylvinyl)carbonyl-spiro[cyclohexan-1,1'(1'H)-pyrido[3,4-b]indol]-4-amin (Cis-Diastereomer)

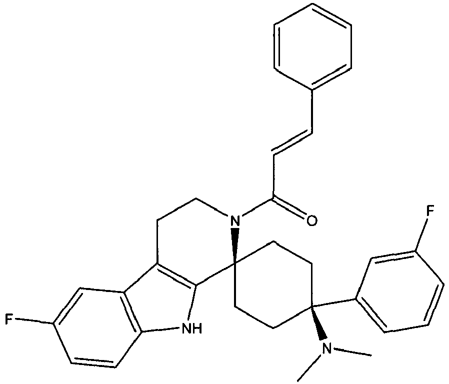

In einem Mikrowellengefäß wurde eine Suspension des Spiroamins AMN-3*^{cis}* (0.197 g; 0.5 mmol; 1 Äq.) in 15 ml abs DCM vorgelegt. Zu dieser Suspension wurden nacheinander Ethyl-diisopropylamin (0.129 g; 1 mmol; 2 Äq.) und Zimtsäurechlorid (0.166 g; 1 mmol; 2 Äq.) gegeben. Das Mikrowellengefäß wurde verschlossen und in der Mikrowelle (Initiator Eight, Firma Biotage) 10 min auf 120°C erhitzt. Zur Aufarbeitung wurde das Reaktionsgemisch mit 4 ml Wasser und 4 ml 1N Natronlauge versetzt. Diese Mischung wurde 2h bei RT gerührt. Danach wurden die Phasen getrennt und die wässrige Phase 3x mit DCM extrahiert. Die vereinigten organischen Phasen wurden mit Wasser gewaschen und über Natriumsulfat getrocknet. Nachdem das Lösungsmittel bei vermindertem Druck entfernt wurde, wurde der Rückstand säulenchromatographisch (Kieselgel; Essigester/Cyclohexan 1:2 → 1:0) gereinigt. Es wurden 0.087 g Produkt AMD-8*^{cis}* (33 %) erhalten.
HPLC/MS-Analytik: Rₜ = 4,2 min; Purity (UV 200-400 nm) 97%; *m*/*z* = 526,1

### Vergleichsbeispiel AMD-9^{cis}.

### 2',3',4',9'-Tetrahydro-N,N-dimethyl-6'-fluor-4-(3-fluorphenyl)-2'-(benzyl)carbonyl-spiro[cyclohexan-1,1'(1'H)-pyrido[3,4-b]indol]-4-amin (Cis-Diastereomer)

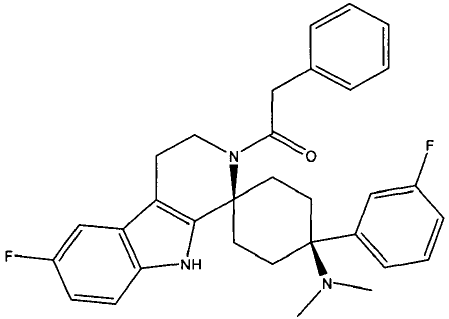

In einem Mikrowellengefäß wurde eine Suspension des Spiroamins AMN-3*^{cis}* (0.25 g; 0.63 mmol; 1 Äq.) in 19ml abs DCM vorgelegt. Zu dieser Suspension wurden nacheinander Ethyl-diisopropylamin (0.163 g; 1.26 mmol; 2 Äq.) und 2-Phenylacetyl chloride (0.195 g; 1.26 mmol; 2 Äq.) gegeben. Das Mikrowellengefäß wurde verschlossen und in der Mikrowelle (Initiator Eight, Firma Biotage) für 10 min auf 120°C erhitzt. Zur Aufarbeitung wurde das Reaktionsgemisch mit 5 ml Wasser und 5 ml 1N Natronlauge versetzt. Diese Mischung wurde 2h bei RT gerührt. Anschließend wurden die Phasen getrennt und die wässrige Phase 3x mit DCM extrahiert. Die vereinigten organischen Phasen wurden mit Wasser gewaschen und über Natriumsulfat getrocknet. Nachdem das Lösungsmittel bei vermindertem Druck entfernt wurde, wurde der Rückstand säulenchromatographisch (Kieselgel; Essigester → Essigester/Methanol 9:1) gereinigt. Es wurden 0.145 g Produkt AMD-9*^{cis}* (45%) erhalten.
HPLC/MS-Analytik: Rₜ = 3,9 min; Purity (UV 200-400 nm) 98%; *m*/*z* = 514,1

### Beispiel AMD-10^{cis}

### (E)-2',3',4',9'-Tetrahydro-N,N-dimethyl-6'-fluor-4-phenyl-2'-(2-phenylvinyl)carbonyl-spiro[cyclohexan-1,1'(1'H)-pyrido[3,4-b]indol]-4-amin (Cis-Diastereomer)

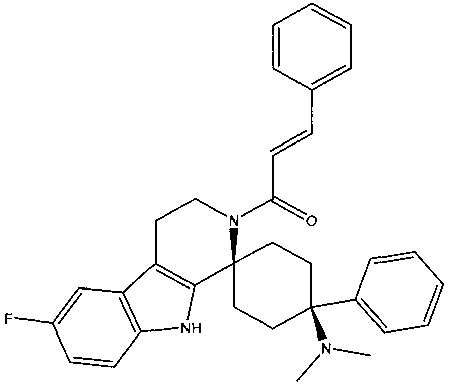

Zu einer Lösung des Zimtsäurechlorids (0.198 g; 1.192 mmol; 3 Äq.) in 4.5 ml abs THF wurde unter Stickstoff bei RT eine Lösung des Spiroamins AMN-4*^{cis}* (0.15 g; 0.397 mmol; 1 Äq.) in 9 ml abs THF gegeben. Nachdem 1h bei RT gerührt wurde, wurde die trübe Reaktionslösung erst mit 3 ml Wasser und unter Eiskühlung mit 3 ml 1N Natronlauge versetzt. Es wurde für 1.5h gerührt. Nachdem das Lösungsmittel bei vermindertem Druck entfernt wurde, wurde der ausgefallene Feststoff abfiltriert und mit Wasser gewaschen. Das Rohprodukt wurde säulenchromatographisch (Kieselgel; Essigester) gereinigt. Es wurden 0.043g Produkt AMD-10*^{cis}* (21%) erhalten.
HPLC/MS-Analytik: Rₜ = 4,2 min; Purity (UV 200-400 nm) 98%; *m*/*z* = 508,2

### Vergleichsbeispiel AMD-11^{cis}.

### 2',3',4',9'-Tetrahydro-N,N-dimethyl-4-(3-fluorphenyl)-2'-benzylcarbonyl-spiro[cyclohexan-1,1'(1'H)-pyrido[3,4-b]indol]-4-amin (Cis-Diastereomer))

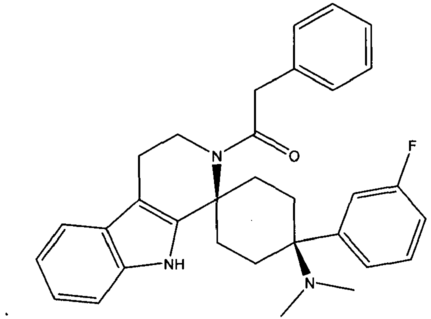

Das cis-Spiroamin AMN-2*^{cis}* (1,29 g, 3,4 mmol) wurde unter Ausschluß von Sauerstoff in absolutem Tetrahydrofuran (20 ml) und absolutem Dichlormethan (120 ml) gelöst, mit Hünig-Base (1,167 ml, 6,8 mmol) versetzt und bei Raumtemperatur mit 2-Phenylacetyl chlorid (900 µl, 6,8 mmol) versetzt. Nach einer Reaktionszeit von 30 min wurde der Ansatz mit 5N Natronlauge (100 ml) versetzt und 2 h gerührt. Die wäßrige Phase wurde abgetrennt und mit Dichlormethan (3 × 10 ml) extrahiert. Die vereinigten organischen Phasen wurden über Na₂SO₄ getrocknet und anschließend eingeengt. Es wurde ein Rohprodukt isoliert, das chromatographisch aufgetrennt wurde [Kieselgel 60 (100 g); EtOAc (1000 ml)]. Das cis-Amid AMD-11*^{cis}* wurde in einer Ausbeute von 820 mg (49 %) mit einem Schmelzpunkt von 95-100 °C als farbloser Feststoff erhalten.
¹³C-NMR (101 MHz, DMSO-D₆) δ ppm: 22.1, 29.1, 33.0, 38.0, 40.8, 43.1, 60.0, 60.3, 105.5, 111.1, 113.7, 113.2, 114.5, 114.7, 117.3, 118.4, 120.5, 123.8, 126.2, 126.5, 128.2,129.0, 129.2, 129.3, 135.3, 136.5, 139.5, 140.6, 161.1, 163.5, 173.4

### Beispiel AMD-12^{cis}

### (E)-2',3',4',9'-Tetrahydro-N,N-dimethyl-4-(4-fluorphenyl)-2'-(2-phenylvinyl)carbonyl-spiro[cyclohexan-1,1'(1'H)-pyrido[3,4-b]indol]-4-amin (Cis-Diastereomer)

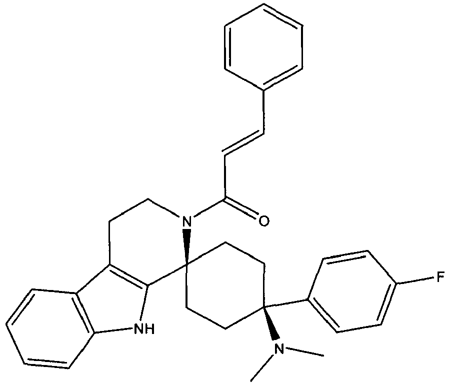

Zu einer Suspension des cis-Spiroamins AMN-5*^{cis}* (500 mg, 1,32 mmol) in absolutem Dichlormethan (40 ml) wurden unter Argon innerhalb von 10 min nacheinander Hünig-Base (0,45 ml, 342 mg, 2,64 mmol) und Zimtsäurechlorid (440 mg, 2,64 mmol), gelöst in absolutem Dichlormethan (12 ml), getropft. Die Reaktionsmischung wurde 1 h bei Raumtemperatur gerührt, anschließend mit Wasser (30 ml) sowie 1N NatriumhydroxidLösung (5 ml) versetzt und 1,5 h gerührt. Das Dichlormethan wurde dann im Vakuum entfernt. Hierbei fiel ein heller Feststoff aus, der durch Filtration abgetrennt und dann mit Wasser (3 x 30 ml) gewaschen wurde. Das so erhaltene Rohprodukt wurde chromatographisch gereinigt [Kieselgel 60 (70 g), Ethylacetat/Cyclohexan 1 : 1 (500 ml), Ethylacetat (1000 ml), Ethylacetat/Methanol 10 : 1 (330 ml), Ethylacetat/Methanol 4 : 1 (800 ml), Methanol (300 ml)]. Zum Auftragen des Rohproduktes auf die Säule mußte dieses in Ethylacetat/Cyclohexan 1 : 1 mit etwas Tetrahydrofuran gelöst werden. Das cis-Amid AMD-12*^{cis}* (Smp. 145-155 °C) wurde als farbloser Feststoff in einer Ausbeute von 31 % (204 mg, 0,40 mmol) erhalten.
¹³C{¹H}-NMR (101 MHz, DMSO-*D*₆) δ ppm: 22.5 (1 C), 29.3 (2 C), 32.6 (2 C), 37.8 (2 C), 41.3 (1 C), 59.5 (1 C), 60.3 (1 C, br), 105.4 (1 C), 111.1 (1 C), 114.3 (2 C, d, J = 20 Hz), 117.3 (1 C), 118.4 (1 C), 120.5 (1 C), 123.1 (1 C), 126.6 (1 C), 127.9 (2 C), 128.7 (2 C), 129.3 (2 C), 129.8 (2 C, d, J = 8 Hz), 132.4 (1 C, br), 135.1 (1 C), 135.4 (1 C), 139.4 (1 C), 140.4 (1 C), 160.9 (1 C, d, J = 243 Hz), 170.3 (1 C)

### Synthese der Trans-Spiroamid Vergleichsbeispiele (AMD^{trans})

### Vergleichsbeispiel AMD-3^{trans}.

### 2',3',4',9'-Tetrahydro-N,N-dimethyl-4-(3-fluorphenyl)-2'-(benzothiophen-2-yl)carbonyl-spiro[cyclohexan-1,1'(1'H)-pyrido[3,4-b]indol]-4-amin Citrat (1:1) (Trans-Diastereomer)

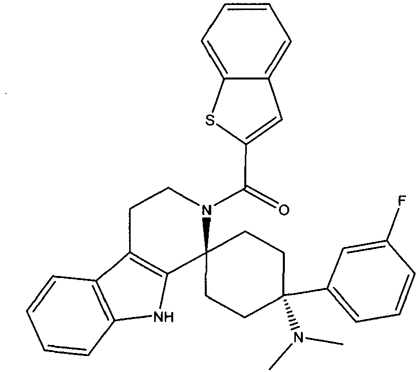

### 2',3',4',9'-Tetrahydro-N,N-dimethyl-4-(3-fluorphenyl)-2'-(benzothiophen-2-yl)carbonyl-spiro[cyclohexan-1,1'(1'H)-pyrido[3,4-b]indol]-4-amin (Trans-Diastereoisomer)

Benzo[b]thiophen-2-carbonsäurechlorid (728 mg, 3,96 mmol) wurde unter Argon in abs. Tetrahydrofuran (30 ml) gelöst und bei Raumtemperatur mit dem trans-Spiroamin AMN-2*^{trans}* (500 mg, 1,32 mmol), gelöst in abs. Tetrahydrofuran (60 ml), innerhalb von 75 min versetzt. Dabei entstand eine leichte Fällung. Nach einer Reaktionszeit von 2 h wurde die Reaktionsmischung mit Wasser (15 ml) verdünnt, unter Eiskühlung mit 1N Natronlauge (15 ml) versetzt und 2,5 h gerührt. Tetrahydrofuran wurde im Vakuum entfernt. Dabei fiel ein Feststoff aus, der durch Filtration abgetrennt und mit Wasser (3 × 20 ml) gewaschen wurde. Das Rohprodukt (587 mg) wurde chromatographisch aufgetrennt [Kieselgel 60 (80 g); Ethylacetat/Cyclohexan 1 : 1 (1 I), Ethylacetat/Methanol 4 : 1 (500 ml)]. Das trans-Amid wurde so als farbloser Feststoff in einer Ausbeute von 12 % (82 mg) mit einem Schmelzpunkt von 219-221 °C gewonnen.
¹³C-NMR (101 MHz, CDCl₃) δ ppm: 22.4, 30.0, 30.9, 38.2, 46.4, 58.3, 59.5, 106.2, 111.0, 113.5, 113.7, 114.4, 114.7, 118.0, 119.1, 121.4, 122.5, 123.1, 124.7, 125.5, 125.8, 126.4, 128.7, 136.0, 138.7, 140.1, 140.4, 141.1, 142.1, 161.2, 163.7, 167.1

### 2',3',4',9'-Tetrahydro-N,N-dimethyl-4-(3-fluorphenyl)-2'-(benzothiophen-2-yl)carbonyl-spiro-[cyclohexan-1,1'(1'H)-pyrido[3,4-b]indol]-4-amin Citrat (1:1) (Trans-Diastereomer; AMD-3^{trans})

Das soeben hergestellte trans-Amid (82 mg, 0,152 mmol) wurde bei 80 °C in Ethanol (8 ml) suspendiert und mit einer ethanolischen Lösung (3 ml) von Citronensäure (32 mg, 0,167 mmol) versetzt. Aus der klaren Lösung fiel beim Abkühlen auf Raumtemperatur ein Feststoff aus. Nach 1,5 h wurde die Mischung auf 2 ml eingeengt, mit Diethylether (20 ml) versetzt und 20 min gerührt. Durch Filtration wurde ein farbloser Feststoff abgetrennt und mit Diethylether (2 × 3 ml) gewaschen (64 mg). Nach 3 Tagen war aus dem Filtrat bei Raumtemperatur weiterer Feststoff ausgefallen, der abgesaugt und mit Diethylether (2 × 2 ml) gewaschen wurde (35 mg). Beide Fraktionen wurden vereinigt. Das trans-Citrat AMD-3*^{trans}* wurde so in einer Ausbeute von 81 % (89 mg) mit einem Schmelzpunkt von 175-185 °C erhalten.

### Vergleichsbeispiel AMD-6^{trans}.

### (E)-2',3',4',9'-Tetrahydro-N,N-dimethyl-4-(3-fluorphenyl)-2'-(2-phenylvinyl)carbonyl-spiro-[cyclohexan-1,1'(1'H)-pyrido[3,4-b]indol]-4-amin Citrat (1:1) (Trans-Diastereomer)

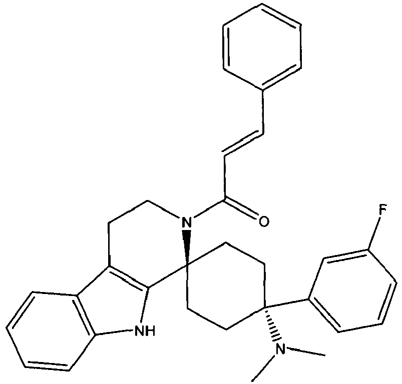

### 2',3',4',9'-Tetrahydro-N,N-dimethyl-4-(3-fluorphenyl)-2'-(2-phenylvinyl)carbonyl-spiro[cyclohexan-1,1'(1'H)-pyrido[3,4-b]indol]-4-amin (Trans-Diastereomer)

Zimtsäurechlorid (1,32 g, 7,92 mmol) wurde unter Argon in abs. Tetrahydrofuran (30 ml) gelöst und bei Raumtemperatur mit verunreinigtem Spiroamin AMN-2*^{cis}* (1,0 g, 2,64 mmol, enthielt nahezu 10 % trans-Diastereoisomer AMN-2*^{trans}*), gelöst in abs. Tetrahydrofuran (60 ml), innerhalb von 40 min versetzt. Nach einer Reaktionszeit von 1 h wurde die trübe Reaktionslösung mit Wasser (20 ml) und unter Eiskühlung mit 1N Natronlauge (20 ml) versetzt und 1,5 h gerührt. Tetrahydrofuran wurde im Vakuum entfernt. Dabei fiel ein Feststoff aus, der durch Filtration abgetrennt und mit Wasser (3 × 25 ml) gewaschen wurde. Das Rohprodukt (1,16 g) wurde chromatographisch aufgetrennt [Kieselgel 60 (200 g); Ethylacetat/Cyclohexan 1 : 1 (1,3 I), Ethylacetat (1,6 I)]. Das cis-Amid wurde als farbloser Feststoff in einer Ausbeute von 40 % (540 mg) mit einem Schmelzpunkt von 155-158 °C gewonnen. Das trans-Amid wurde in einer Ausbeute von 7 % (93 mg) mit einem Schmelzpunkt von 151-155 °C isoliert.

### 2',3',4',9'-Tetrahydro-N,N-dimethyl-4-(3-fluorphenyl)-2'-(2-phenylvinyl)carbonyl-spiro[cyclohexan-1,1'(1'H)-pyrido[3,4-b]indol]-4-amin Citrat (1:1) (Trans-Diastereomer; AMD-6^{trans})

Das soeben erhaltene trans-Amid (188 mg, 0,37 mmol) wurde bei 80 °C in Ethanol (35 ml) gelöst und mit einer ethanolischen Lösung (2 ml) von Citronensäure (77 mg, 0,4 mmol) versetzt. Es wurde 2 h bei Raumtemperatur gerührt, wobei nach und nach Kristallisation einsetzte. Die Mischung wurde 1,5 h bei 5 °C aufbewahrt, der farblose Feststoff durch Filtration abgetrennt und mit Diethylether (3 × 3 ml) gewaschen (146 mg). Das Filtrat wurde eingeengt, in Ethanol (1 ml) aufgenommen und mit Diethylether (20 ml) versetzt. Nach 16 h wurde weiteres farbloses Salz abgetrennt und mit Diethylether (2 × 2 ml) gewaschen (36 mg). Beide Fraktionen wurden vereinigt und das trans-Citrat AMD-6*^{trans}* in einer Ausbeute von 71 % (182 mg) mit einem Schmelzpunkt von 161-164 °C erhalten .
¹³C-NMR (101 MHz, DMSO-D₆) δ ppm: (trans-Diastereoisomer) 22.4, 29.2, 30.7, 37.9, 41.5, 43.1, 58.5, 59.6, 72.0, 105.5, 111.3, 113.2, 113.4, 113.5, 113.8, 117.3, 118.4, 120.5, 122.8, 123.1, 126.5, 127.7, 128.6, 129.1, 129.2, 135.0, 135.6, 139.8, 140.1, 160.7, 163.1, 169.9, 171.2, 175.2

### Vergleichsbeispiel AMD-7^{trans}.

### 2',3',4',9'-Tetrahydro-N,N-dimethyl-4-(3-fluorphenyl)-2'-(3,4-dimethoxybenzyl)carbonyl-spiro[cyclohexan-1,1'(1'H)-pyrido[3,4-b]indol]-4-amin (Trans-Diastereomer)

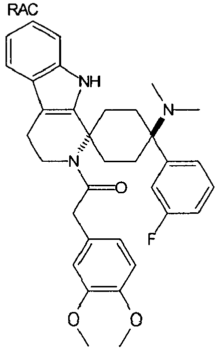

3,4-Dimethoxyphenylessigsäure (1 g, 5.1 mmol, 2.2 Äq.) wird in 25 ml abs. Toluol suspendiert und mit Thionylchlorid (0.84 ml, 11.6 mmol, 5.0 Äq.) versetzt. Es wird für 2 h unter Rückfluß erhitzt und das Lösungsmittel anschließend entfernt. Der Rückstand wurde mit abs. Toluol codestilliert (3 x 50 ml) und das Rohprodukt in Dichlormethan (37 ml) gelöst und in ein Mikrowellengefäß überführt. Es werden Spiroamin AMN-2*^{trans}* (0.875 mg, 2.32 mmol) und Hünig Base (0.78 ml, 580 mmol, 250 Äq.) hinzugefügt, das Mikrowellengefäß verschlossen und in der Mikrowelle (Initiator Eight, Firma Biotage) für 20 min auf 120°C erhitzt. Zur Aufarbeitung wurde das Reaktionsgemisch mit 17 ml Wasser und 17 ml 1N Natronlauge versetzt. Diese Mischung wurde 2h bei RT gerührt. Anschließend wurden die Phasen getrennt und die wässrige Phase 3x mit Dichlormethan extrahiert. Die vereinigten organischen Phasen wurden mit Wasser gewaschen und über Natriumsulfat getrocknet. Nachdem das Lösungsmittel bei vermindertem Druck entfernt wurde, wurde der Rückstand säulenchromatographisch (Kieselgel; Essigester/n-Hexan 2:1) gereinigt. Es wurden 0.236 g Produkt AMD-7*^{trans}* (18%) erhalten.
HPLC/MS-Analytik: Rₜ = 5.45 min; Purity (UV 200-400 nm) > 99%; *m*/*z* = 555,8

### Synthese der Cis Spiroether Vergleichsbeispiele (ETHER^{cis})

### Vergleichsbeispiel ETHER-1^{cis}

### 6'-Fluoro-4',9'-dihydro-N,N-dimethyl-4-(3-thienyl)-spiro[cyclohexan 1,1'(3'H)-pyrano[3,4-b]indol]-4-amin, Methansulfonat (2:5) (Cis-Diastereomer)

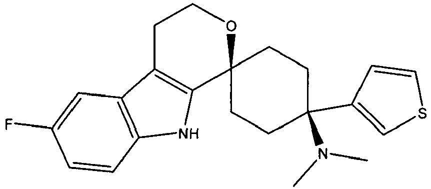

Das Keton E-5 (446,6 mg, 2 mmol) wurde zusammen mit 5-Fluortryptophol (2, 394,4 mg, 2 mmol) in absolutem 1,2-Dichlorethan (30 ml) gelöst. Die Mischung wurde anschließend mit Methansulfonsäure (0,13 ml, 2 mmol) versetzt, wobei es zum Farbumschlag der Reaktionslösung von rotbraun nach dunkelgrau kam. Nach 5 min begann ein hellgrauer Feststoff auszufallen. Der Ansatz wurde 20 h bei RT gerührt. Dann wurde das Methansulfonat des cis-Spiroethers abgesaugt und mit 1,2-Dichlorethan (2 × 10 ml) gewaschen. Der hellgraue Feststoff wurde in einer Ausbeute von 76 % (733 mg) und einem Schmelzpunkt von 143-145 °C gewonnen (ETHER-1*^{cis}*). Das Filtrat wurde anschließend mit 1N NaOH (30 ml) versetzt und 2 h bei RT gerührt. Dabei fiel der trans-Spiroether als farbloser Feststoff aus und wurde nach Filtration in einer Ausbeute von 8 % (58,5 mg) erhalten.
¹H NMR (600 MHz, DMSO-*d*₆): 1,67 (m, 2 H) 1,94 (m, 2 H) 2,24 (m, 2 H) 2,44 (s, 8 H) 2,53 (s, 3 H) 2,54 (s, 3 H) 2,66 (t, *J* = 5,27 Hz, 2 H) 2,72 (m, 2 H) 3,95 (t, *J* = 5,28 Hz, 2 H) 6,84 (m, 1 H) 7,14 (m, 1 H) 7,19 (dd, *J* = 4,50 / 8,70 Hz, 1 H) 7,47 (d, *J* = 5,10 Hz, 1 H) 7,83 (m, 1 H) 8,07 (m, 1 H) 9,67 (m, 1 H) 10,80 (s, 1 H)

### Vergleichsbeispiel ETHER-2^{cis}

### 4',9'-Dihydro-N,N-dimethyl-4-(2-thienyl)-spiro[cyclohexan 1,1'(3'H)-pyrano[3,4-b]indol]-4-amin, Methansulfonat (1:2) (Cis-Diastereomer)

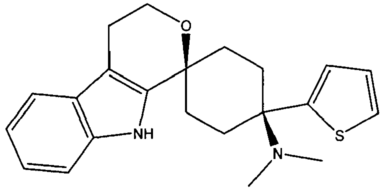

Das Keton E-4 (223 mg, 1 mmol) wurde zusammen mit Tryptophol (2, 161 mg, 1 mmol) in absolutem Dichlormethan (40 ml) vorgelegt. Anschließend erfolgte die Zugabe von Methansulfonsäure (0,071 ml, 1,1 mmol). Der Ansatz wurde 16 h bei RT gerührt, wobei das Methansulfonat des Spiroethers ausfiel. Der hellgraue Feststoff (ETHER-2*^{cis}*) wurde abgesaugt, mit Dichlormethan (2 × 10 ml) gewaschen und in einer Ausbeute von 25 % (117 mg) mit einem Schmelzpunkt von 132 °C gewonnen. Das Filtrat wurde mit 1N NaOH (20 ml) versetzt und 16 h bei RT gerührt. Die organische Phase wurde abgetrennt und die wäßrige Phase mit Dichlormethan (2 × 20 ml) extrahiert. Die organischen Phasen wurden vereinigt, getrocknet und eingeengt. Dabei wurde ein Substanzgemisch (274 mg) erhalten, das chromatographisch aufgetrennt wurde [Kieselgel G (20 g); Ethylacetat/Methanol 8 : 1]. Der trans-Spiroether wurde dabei in einer Ausbeute von 54 % (196 mg, Fp. 235-238 °C), der cis-Spiroether in einer Ausbeute von 10 % (38 mg) gewonnen.
¹H NMR (600 MHz, DMSO-*d*₆)
1,82 (m, 2 H) 1,98 (m, 2 H) 2,33 (m, 2 H) 2,36 (s, 6 H) 2,60 (s, 3 H) 2,61 (s, 3 H) 2,53 (m, 2 H) 2,70 (t, *J* = 5,23 Hz, 2 H) 3,96 (t, *J* = 5,23 Hz, 2 H) 6,94 (m, 1 H) 7,00 (m, 1 H) 7,21 (d, *J* = 8,29 Hz, 1 H) 7,34 (dd, *J* = 3,74 / 5,28 Hz, 1 H) 7,37 (d, *J* = 7,37 Hz, 1 H) 7,59 (d, *J* = 2,76 Hz, 1 H) 7,95 (d, *J* = 5,32 Hz, 1 H) 9,78 (m, 1 H) 10,74 (s, 1 H)

Geräte und Methoden für die HPLC-MS Analytik: *HPLC:* Waters Alliance 2795 mit PDA Waters 996; *MS:* ZQ 2000 MassLynx Single Quadrupol MS Detector; *Säule:* Waters Atlantis™ dC18, 3 µm, 2,1 x 30 mm; *Säulentemperatur:* 40°C, *Eluent A:* purified water + 0,1% Ameisensäure; *Eluent B*: Acetonitril (gradient grade) + 0,1% Ameisensäure; *Gradient:* 0% B zu 100% B in 8,8 min, 100% B für 0,4 min, 100% B zu 0% B in 0,01 min, 0% B für 0,8 min; *Fluß:* 1,0 mL/min; *Ionisation:* ES+, 25 V; *Make up:* 100 µL/min 70% Methanol + 0,2% Ameisensäure; *UV*: 200 - 400 nm.

### Untersuchung der pharmakologischen Eigenschaften der Beispielverbindungen

A) Vergleich der analgetischen Wirksamkeit (als ED₅₀, bzw. %MPE bei einer bestimmten Testdosierung) im Akutschmerzmodell (Tail-flick, Ratte/Maus) und in Mono-Neuropathieschmerzmodellen (Chung, Ratte; Bennett, Ratte) bzw. Poly-Neuropathieschmerzmodell (STZ-Polyneuropathie, Ratte).

Zur Beschreibung der überraschenden pharmakologischen Eigenschaften der erfindungsgemäßen Verbindungen werden in erster Linie die Ergebnisse aus dem Mononeuropathieschmerzmodell nach Chung bei der Ratte und dem Tail-flick Akutschmerzmodell bei der Ratte miteinander verglichen. Hierbei kann gezeigt werden, dass die erfindungsgemäßen Verbindungen bei einem Vielfachen einer im Chung-Modell analgetisch signifikant wirksamen Dosierung (beispielsweise ED₅₀ⁿ) keine signifikante anti-nociceptive Wirkung in Tail-flick-Modell bei der Ratte zeigt. Die Befunde aus weiteren Modellen zum Neuropathiesschmerz, wie Bennett-Modell bei der Ratte oder STZ-Polyneuropathie bei der Ratte, unterstreichen die generell sehr gute Wirksamkeit der Verbindungen bei unterschiedlichen Formen von neuropathischem Schmerz.

### Analgesieprüfung im Tail-Flick-Test an der Ratte

Versuchstiere: weibliche Sprague Dawley Ratten (crl: CD (SD) outbred; Züchter: Charles River, Sulzfeld, Deutschland); Körpergewicht: 130 - 190 g; die Tiere werden in Standardkäfigen (Typ IV Makrolone-Käfige, Fa. Ebeco, Castrop-Rauxel, Deutschland) besetzt mit jeweils maximal 8 Tieren unter einem 12:12h Licht-Dunkel Rhythmus mit Futter und Leitungswasser ad libitum gehalten.

Methodenbeschreibung: Die analgetische Wirksamkeit der Testverbindungen wurde im Brennstrahl (Tail-flick)-Test an der Ratte nach der Methode von D'Amour and Smith (J. Pharm. Exp. Ther. 72, 74 79 (1941)) untersucht. Die Tiere wurden einzeln in spezielle Testkäfige gesetzt und die Schwanzbasis einem focussierten Wärmestrahl einer Lampe (Tail-flick Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Wegziehlatenz) bei unbehandelten Tieren 2,5-5 Sekunden betrug. Vor Gabe einer Testverbindung wurden die Tiere innerhalb von 30 Minuten zweimal vorgetestet und der Mittelwert dieser Messungen als Vortestmittelwert berechnet. Die Schmerzmessung wurde in der Regel 5, 20, 40, 60, 90, 120, 180 und 240 min nach intravenöser Gabe von Testverbindung oder deren Vehikel durchgeführt. Die antinociceptive Wirkung wurde als Zunahme der Wegziehlatenzzeit nach folgender Formel bestimmt: (% MPE) = [(T₁ - T₀)/(T₂ - T₀)] x 100. Dabei sind: T₀: Kontroll-Latenzzeit vor Substanzapplikation, T₁: Latenzzeit nach Substanzapplikation, T₂: maximale Expositionszeit des Brennstrahls (12 Sekunden), MPE: maximal möglicher Effekt.

Bei antinociceptiv wirksamen Testverbindungen wurden zur Bestimmung der Dosisabhängigkeit 3 - 5 logarithmisch ansteigende Dosen, die jeweils die Schwellen- und die maximale Wirkdosis einschlossen, appliziert. Die halbmaximal wirksame Dosis (ED₅₀) mit entsprechenden 95% Vertrauensbereichen wurde über semilogarithmische Regressionsanalyse zum Zeitpunkt der maximalen Wirkung bestimmt.

Statistische Auswertung: Die Gruppengrößen betrugen üblicherweise n=10. Mittels Varianzanalyse mit Messwiederholung (repeated measures ANOVA) sowie einer post hoc Analyse nach Bonferroni wurde auf statistisch signifikante Unterschiede der %MPE-Daten zwischen den jeweiligen Dosisgruppen und der Vehikel-Kontrollgruppen getestet. Das Signifikanzniveau wurde auf p < 0.05 gesetzt.

### Tail-flick mit verringerter Brennstrahlintensität an der Ratte

Versuchstiere: männliche Sprague-Dawley Ratten (Züchter: Janvier, Le Genest St. Isle, Frankreich); Körpergewicht: 200 - 250 g; die Tiere werden in Standardkäfigen (Typ IV Makrolone-Käfige, Fa. Ebeco, Castrop-Rauxel, Deutschland) besetzt mit jeweils maximal 5 Tieren unter einem 12:12h Licht-Dunkel Rhythmus mit Futter und Leitungswasser ad libitum gehalten.

Methodenbeschreibung: Die modulatorische Wirksamkeit der Testsubstanzen auf akute, noxische thermische Reize wurde im Brennstrahl (Tail-flick)-Test an der Ratte nach der Methode von D'Amour und Smith (J. Pharm. Exp. Ther. 72, 74 79 (1941)) untersucht. Dazu wurden verwendet. Die Tiere wurden einzeln in spezielle Testkompartimente verbracht und die Schwanzbasis einem focussierten Brennstrahl eines Analgesiemeters (Modell 2011, Rhema Labortechnik, Hofheim, Deutschland) ausgesetzt. Die Brennstrahlintensität wurde so eingestellt, dass die Zeit vom Einschalten des Brennstrahls bis zum plötzlichen Wegziehen des Schwanzes (Wegziehlatenz) bei unbehandelten Tieren ca. 12-13 Sekunden betrug. Vor Gabe einer erfindungsgemäßen Substanz wurde im Abstand von fünf Minuten zweimal die Wegziehlatenz bestimmt und der Mittelwert als Kontroll-Latenzzeit definiert. Die Messung der Wegziehlatenz des Schwanzes wurde erstmals 10 Minuten nach intravenöser Gabe von Testverbindung oder deren Vehikel durchgeführt. Nach Abklingen des antinociceptiven Effekts (nach 2-4 Stunden) erfolgten die Messungen im Abstand von 30 Minuten bis maximal 6.5 Stunden nach Substanzapplikation. Die anti- oder pronociceptive Wirkung wurde als Zu- bzw. Abnahme der Wegziehlatenzzeit nach folgender Formel bestimmt: (% MPE) = [(T₁ - T₀)/(T₂ - T₀)] x 100. Dabei sind: T₀: Kontroll-Latenzzeit vor Substanzapplikation, T₁: Latenzzeit nach Substanzapplikation, T₂: maximale Expositionszeit des Brennstrahls (30 Sekunden), MPE: maximal möglicher Effekt. Bei antinociceptiv wirksamen Testverbindungen wurde zur Bestimmung der Dosisabhängigkeit 3 - 5 logarithmisch ansteigenden Dosen, die jeweils die Schwellen- und die maximale Wirkdosis einschlossen, appliziert. Die halbmaximal wirksame Dosis (ED₅₀) mit entsprechenden 95% Vertrauensbereichen wurde über semilogarithmische Regressionsanalyse zum Zeitpunkt der maximalen Wirkung bestimmt.

Statistische Auswertung: Die Gruppengrößen betrugen üblicherweise n=10. Mittels Varianzanalyse mit Messwiederholung (repeated measures ANOVA) sowie einer post hoc Analyse nach Bonferroni wurde auf statistisch signifikante Unterschiede der %MPE-Daten zwischen den jeweiligen Dosisgruppen und der Vehikel-Kontrollgruppen getestet. Das Signifikanzniveau wurde auf p < 0.05 gesetzt.

### Analgesieprüfung im Tail-Flick-Test an der Maus

Versuchstiere: männliche NMRI Mäuse (Züchter: Charles River, Sulzfeld, Deutschland); Körpergewicht: 20 - 25 g; die Tiere werden in Standardkäfigen (Typ III Makrolone-Käfige, Fa. Ebeco, Castrop-Rauxel, Deutschland) besetzt mit jeweils maximal 6 Tieren unter einem 12:12h Licht-Dunkel Rhythmus mit Futter und Leitungswasser ad libitum gehalten.

Methodenbeschreibung: Die analgetische Wirksamkeit der Testverbindung wurde im Brennstrahl (Tail-flick)-Test an der Maus nach der Methode von D'Amour and Smith (J. Pharm. Exp. Ther. 72, 74 79 (1941) untersucht. Die Tiere wurden einzeln in spezielle Testkäfige gesetzt und die Schwanzbasis einem focussierten Wärmestrahl einer elektrischen Lampe (Tail-flick Typ 55/12/10.fl, Labtec, Dr. Hess) ausgesetzt. Die Lampenintensität wurde so eingestellt, daß die Zeit vom Einschalten der Lampe bis zum plötzlichen Wegzucken des Schwanzes (Wegziehlatenz) bei unbehandelten Tieren 2,5-5 Sekunden betrug. Vor Gabe einer Testverbindung wurden die Tiere innerhalb von 30 Minuten zweimal vorgetestet und der Mittelwert dieser Messungen als Vortestmittelwert berechnet. Die Schmerzmessung wurde in der Regel 20, 40 und 60 min nach intravenöser Gabe von Testverbindung oder deren Vehikel durchgeführt. Die antinociceptive Wirkung wurde als Zunahme der Wegziehlatenzzeit nach folgender Formel bestimmt: (% MPE) = [(T₁ - T₀)/(T₂ - T₀)] x 100. Dabei sind: T₀: Kontroll-Latenzzeit vor Substanzapplikation, T₁: Latenzzeit nach Substanzapplikation, T₂: maximale Expositionszeit des Brennstrahls (12 Sekunden), MPE: maximal möglicher Effekt. Bei antinociceptiv wirksamen Testverbindungen wurden zur Bestimmung der Dosisabhängigkeit 3 - 5 logarithmisch ansteigende Dosen, die jeweils die Schwellen- und die maximale Wirkdosis einschlossen, appliziert. Die halbmaximal wirksame Dosis (ED₅₀) mit entsprechenden 95% Vertrauensbereichen wurde über semilogarithmische Regressionsanalyse zum Zeitpunkt der maximalen Wirkung bestimmt.

Statistische Auswertung: Die Gruppengrößen betrugen üblicherweise n=10. Mittels Varianzanalyse mit Messwiederholung (repeated measures ANOVA) sowie einer post hoc Analyse nach Bonferroni wurde auf statistisch signifikante Unterschiede der %MPE-Daten zwischen den jeweiligen Dosisgruppen und der Vehikel-Kontrollgruppen getestet. Das Signifikanzniveau wurde auf p < 0.05 gesetzt.

### Chung-Modell: Mononeuropathieschmerz nach spinaler Nervenligatur

Versuchstiere: Männliche Sprague Dawley Ratten (RjHan:SD outbred; Züchter: Janvier, Genest St. Isle, Frankreich) mit einem Körpergewicht von 140-160g wurden in Standardkäfigen (Typ IV Makrolone-Käfige, Fa. Ebeco, Castrop-Rauxel, Deutschland) besetzt mit jeweils maximal 8 Tieren unter einem 12:12h Licht-Dunkel Rhythmus mit Futter und Leitungswasser ad libitum gehalten. Zwischen der Lieferung der Tiere und der Operation wurde eine Pause von einer Woche eingehalten. Die Tiere wurden nach Operation über einen Zeitraum von 4-5 Wochen mehrfach getestet, wobei eine Auswaschzeit von mindestens einer Woche eingehalten wurde.

Modellbeschreibung: Unter Pentobarbital Narkose (Narcoren ®, 60 mg/kg i.p., Merial GmbH, Hallbergmoos, Deutschland), wurden die linken L5, L6 Spinalnerven exponiert, indem ein Stück des paravertebralen Muskels und ein Teil des linken spinalen Prozesses des L5 lumbalen Wirbelkörpers entfernt wurden. Die Spinalnerven L5 und L6 wurden vorsichtig isoliert und mit einer festen Ligatur abgebunden (NC-silk black,USP 5/0, metric 1, Braun Melsungen AG, Melsungen, Deutschland) (Kim and Chung 1992). Nach Ligatur wurden Muskel und benachbarte Gewebe zugenäht und die Wunde mittels Metallklammern geschlossen. Nach einer Woche Erholungszeit wurden die Tiere zur Messung der mechanischen Allodynie in Käfige mit einem Drahtboden gesetzt. An ipsi- und/oder kontralateraler Hinterpfote wurde die Wegziehschwelle mittels eines elektronischen von Frey Filamentes (Somedic AB, Malmö, Schweden) bestimmt. Der Median von fünf Stimulierungen ergab einen Datenpunkt. Die Tiere wurden 30 min vor und zu verschiedenen Zeiten nach Applikation von Testsubstanz- oder Vehikellösung getestet. Die Daten wurden als % maximal mögliche Wirkung (%MPE) aus den Vortesten der Einzeltiere (=0%MPE) und den Testwerten einer unabhängigen Sham-Kontrollgruppe (=100%MPE) bestimmt. Alternativ wurden die Wegziehschwellen in Gramm dargestellt. Bei analgetisch wirksamen Testverbindungen wurde zur Bestimmung der Dosisabhängigkeit 3 - 5 logarithmisch ansteigenden Dosen, die jeweils die Schwellen- und die maximale Wirkdosis einschlossen, appliziert. Die halbmaximal wirksame Dosis (ED₅₀) mit entsprechenden 95% Vertrauensbereichen wurde über semilogarithmische Regressionsanalyse zum Zeitpunkt der maximalen Wirkung bestimmt.

Statistische Auswertung: Die Gruppengrößen betrugen üblicherweise n=10. Mittels Varianzanalyse mit Messwiederholung (repeated measures ANOVA) sowie einer post hoc Analyse nach Bonferroni wurde auf statistisch signifikante Unterschiede der %MPE-Daten zwischen den jeweiligen Dosisgruppen und der Vehikel-Kontrollgruppen getestet. Das Signifikanzniveau wurde auf p < 0.05 gesetzt.

Referenz: Kim, S.H. and Chung, J.M., An experimental model for peripheral neuropathy produced by segmental spinal nerve ligation in the rat, Pain, 50 (1992) 355-363.

### Bennett Modell: Mononeuropathieschmerz an der Ratte

Versuchstiere: Männliche Sprague Dawley Ratten (RjHan:SD outbred; Züchter: Janvier, Genest St. Isle, Frankreich) mit einem Körpergewicht von 140-160g wurden in Standardkäfigen (Typ IV Makrolone-Käfige, Fa. Ebeco, Castrop-Rauxel, Deutschland) besetzt mit jeweils maximal 8 Tieren unter einem 12:12h Licht-Dunkel Rhythmus mit Futter und Leitungswasser ad libitum gehalten. Zwischen der Lieferung der Tiere und der Operation wurde eine Pause von einer Woche eingehalten. Die Tiere wurden nach Operation über einen Zeitraum von 4 Wochen mehrfach getestet, wobei eine Auswaschzeit von mindestens einer Woche eingehalten wurde.

Methodenbeschreibung: Die Untersuchung auf Wirksamkeit im neuropathischen Schmerz wurde im Bennett-Modell (chronic constriction injury; Bennett und Xie, 1988, Pain 33: 87-107) untersucht. Die Ratten werden unter Narcoren-Narkose mit vier losen Ligaturen des rechten nervus ischiaticus versehen. Die Tiere entwickeln an der vom geschädigten Nerv innervierten Pfote eine Überempfindlichkeit, die nach einer Erholungsphase von einer Woche über etwa vier Wochen mittels einer 4°C kalten Metallplatte quantifiziert wird (Kälte-Allodynie). Die Tiere werden für einen Zeitraum von 2 min. auf dieser Platte beobachtet und die Anzahl der Wegziehreaktionen der geschädigten Pfote wird gemessen.

Auswertung und Statistik: Bezogen auf den Vorwert vor Substanzapplikation wird die Substanzwirkung über einen Zeitraum von einer Stunde an vier Zeitpunkten (z.B. 15, 30, 45, 60 min. nach Applikation) bestimmt und die resultierend Fläche unter der Kurve (AUC) sowie die Hemmung der Kälte-Allodynie zu den einzelnen Meßpunkten in Prozent Wirkung zur Vehikelkontrolle (AUC) bzw. zum Ausgangswert (Einzelmeßpunkte) ausgedrückt. Die Gruppengröße beträgt n=10, die Signifikanz einer anti-allodynischen Wirkung (p<0.05) wird anhand einer Varianzanalyse mit wiederholter Messung und einer post hoc Analyse nach Bonferroni bestimmt.

### STZ- Modell: Polyneuropathieschmerz an der Ratte

Versuchstiere: Männliche Sprague-Dawley Ratten (Züchter: Janvier, Le Genest St. Isle, Frankreich); Körpergewicht: 140 - 160 g; die Tiere werden in Standardkäfigen (Typ IV Makrolone-Käfige, Fa. Ebeco, Castrop-Rauxel, Deutschland) besetzt mit jeweils maximal 8 Tieren unter einem 12:12h Licht-Dunkel Rhythmus mit Futter und Leitungswasser ad libitum gehalten.

Methodenbeschreibung: Zur Induktion eines Diabetes wurden männliche Sprague Dawley Ratten intraperitoneal mit Streptozotocin (STZ, 75 mg/kg) injiziert. Diabetische Ratten wiesen einen Blutglukosespiegel von mindestens 17 mM eine Woche nach STZ-Injektion auf. Kontrolltieren wurde eine Vehikellösung injiziert. Die Bestimmung der mechanischen nozizeptiven Reizschwelle (in Gramm) wurde mit einem Algesiometer im paw pressure Test nach Randall & Selitto (1957) durchgeführt. Dabei wurde auf die dorsale Oberfläche der Hinterpfote ein zunehmender Druckreiz ausgeübt und der Druck der schließlich zum reflektorischen Wegziehen der Pfote oder zu Vokalisation führte, registriert. Die Tests fanden drei Wochen nach Diabetesinduktion statt. Die mechanische nozizeptive Reizschwelle wurde vor sowie 15, 30, 45 und 60 Minuten nach Substanzgabe an diabetischen Tieren und an Kontrolltieren gemessen.

Referenzen: Randall LO, Selitto JJ. A method for measurement of analgesic activity on inflamed tissue. Arch. Int. Pharamcodyn. 1957; 111: 409-19
B) Vergleich des analgetischen wirksamen Dosisbereichs im Mono-Neuropathieschmerzmodellen (Chung, Ratte) mit dem Dosisbereich in dem opioidtypische Nebenwirkungen beobachtet werden.

Zur Beschreibung der überraschenden pharmakologischen Eigenschaften der erfindungsgemäßen Verbindungen werden in erster Linie die Ergebnisse aus dem Chung-Modell bei der Ratte (als Beispiel für die analgetische Wirksamkeit gegen Neuropathieschmerz) und dem Blutgasanalyse-Modell bei der Ratte (als Beispiel für Atemdepression als eine sehr schwerwiegende jedoch gut quantifizierbare opioidtypische Nebenwirkung) miteinander verglichen. Hierbei kann gezeigt werden, dass die erfindungsgemäßen Verbindungen bei einem Vielfachen einer im Chung-Modell analgetisch signifikant wirksamen Dosierung (beispielsweise ED₅₀ⁿ) keine signifikante Atemdepression bei der Ratte auslösen. Die Befunde aus weiteren Modellen zu opioidtypischen Nebenwirkungen, wie Kreislaufparameter beim Kaninchen, gastrointestinale Kohlepassage bei der Maus, RotaRod-Test bei der Maus, Jumping-Test bei der Maus sowie konditionierte Platzpräferenz bei der Ratte, unterstreichen die generell fehlenden bzw. sehr geringen opioidtypischen Nebenwirkungen der erfindungsgemäßen Verbindungen.

### Blutgasanalyse: Methode zur arteriellen pCO₂- und pO₂- Messung bei der Ratte

Die atemdepressorische Wirkung von Testsubstanzen wird nach i.v.-Gabe an instrumentierten, wachen Ratten untersucht. Testparameter ist die Veränderung von Kohlendioxid-Partialdruck (pCO₂) und Sauerstoff-Partialdruck (pO₂) im arteriellen Blut nach Substanzgabe.

Versuchstiere: männliche Sprague-Dawley Ratten (crl: CD (SD) outbred; Züchter: Charles River, Sulzfeld, Deutschland); Gewicht: 250-275g; die Tiere werden einzeln in Standardkäfigen (Typ II Makrolone-Käfige, Fa. Ebeco, Castrop-Rauxel, Deutschland) unter einem 12:12h Licht-Dunkel Rhythmus mit Futter und Leitungswasser ad libitum gehalten.

Methodenbeschreibung: Mindestens 6 Tage vor der Prüfsubstanzapplikation wird den Ratten unter Pentobarbitalnarkose jeweils ein PP-Katheter in die Arteria femoralis und in die Vena jugularis implantiert. Die Katheter werden mit Heparinlösung (4000 I.E.) gefüllt und mit einem Drahtstift verschlossen. Die Testsubstanz- bzw. Vehikelapplikation erfolgt über den Venenkatheter. Vor der Substanz- bzw. Vehikelapplikation und zu definierten Zeitpunkten nach Substanz- bzw. Vehikelapplikation wird jeweils der Arterienkatheter geöffnet und mit ca. 500 µl Heparinlösung gespült. Dann werden ca. 100 µl Blut aus dem Katheter entnommen und mittels einer heparinisierten Glaskapillare aufgenommen. Der Katheter wird nochmals mit Heparinlösung gespült und wieder verschlossen. Das arterielle Blut wird sofort mit Hilfe eines Blutgasanalysegerätes (ABL 5, Radiometer GmbH,Willich, Deutschland) vermessen. Nach einer Mindestauswaschzeit von einer Woche können die Tiere erneut in den Versuch genommen werden.

Versuchsauswertung und Statistik: Das Blutgasanalysegerätes liefert automatisch die Werte für pCO₂ und pO₂ des Blutes in mmHg. Substanzeffekte auf den Partialdruck werden als prozentuale Änderungen gegenüber den Vorwerten ohne Substanz- bzw. Vehikel berechnet. Zur statistischen Auswertung werden die Messwerte nach Substanzgabe und die zeitgleichen Messwerte nach Vehikelapplikation mittels Ein-Faktor Varianzanalyse (one-way ANOVA) sowie einer post hoc Analyse nach Dunnett verglichen. Das Signifikanzniveau wurde auf p < 0.05 gesetzt. Die Gruppengrößen betragen üblicherweise n=6.

### Kardiovaskuläre Parameter: Methode zur Messung von Blutdruck und Herzfrequenz beim wachen Kaninchen.

Die Wirkung von Testsubstanzen auf das Herz-Kreislaufsystem wird nach i.v.-Gabe an telemetrierten, wachen Kaninchen untersucht. Testparameter sind Änderung von Herzfrequenz und arteriellem Blutdruck nach Substanzgabe.

Versuchstiere: weibl. Kaninchen (New Zealand Whites; Züchter: Charles River, Kisslegg, Deutschland); Körpergewicht: ca. 3-5,5 kg; die Tiere werden in speziellen Kaninchenkäfigen (B x T x H = 885 x 775 x 600 mm; Fa. Ebeco, Castrop-Rauxel, Deutschland) in Einzelhaltung unter einem 12:12h Licht-Dunkel Rhythmus mit Futter und Leitungswasser ad libitum gehalten.

Versuchsvorbereitung: Mindestens 21 Tage vor Beginn der Experimente wird den Tieren unter Vollnarkose (Isoflurane 2-3%) eine Telemetrieeinheit (TL11 M2-D70-PCT der Fa. DSI, St. Paul, Minnesota, USA) zur Messung von Blutdruck und Elektrokardiogramm (EKG) implantiert. Dabei wird der Druckkatheter der Telemetrieeinheit in die *A. femoralis* eingeführt und die beiden Biopotentialelektroden subcutan in der Sternumregion bzw. im Bereich der oberen linken Thoraxwand befestigt. Die Sendereineheit wird In einer Hauttasche im linken Flankenbereich der Tiere vernäht. Die Aufnahme der Telemtriesignale erfolgt über Empfänger des Typs RMC-1 (Fa. DSI). Zur Datenerfassung, Datenspeicherung und Datenverarbeitung wird das Software Paket Po-Ne-Mah (Fa. DSI) verwendet.

Versuchsablauf: Die Substanz- bzw. Vehikelapplikation erfolgt über einen Venenkatheter (V. *auricularis*). Vor der Substanz- bzw. Vehikelapplikation und zu definierten Zeitpunkten nach Substanz- bzw. Vehikelapplikation werden Herzfrequenz und arterieller Blutdruck (systolischer, diastolischer und mittlerer Wert) mittels des kalibrierten Telemetriesystem direkt ermittelt und elektronisch gespeichert. Nach einer Mindestauswaschzeit von einer Woche können die Tiere erneut in den Versuch genommen werden.

Versuchsauswertung und Statistik: Aus den Messwerten für Blutdruck (in mmHg) und Herzfrequenz (in Schlägen pro min) zu den definierten Zeitpunkten werden jeweils die Mittelwerte von 10 aufeinanderfolegnden Herzschlägen ermittelt. Substanzeffekte auf die Testparameter werden als prozentuale Änderungen gegenüber den Vorwerten ohne Substanz- bzw. Vehikel berechnet. Zur statistischen Auswertung werden die Messwerte nach Substanzgabe und die zeitgleichen Messwerte nach Vehikelapplikation mittels Ein-Faktor Varianzanalyse (one-way ANOVA) sowie einer post hoc Analyse nach Dunnett verglichen. Das Signifikanzniveau wurde auf p < 0.05 gesetzt. Die Gruppengrößen betragen üblicherweise n=6.

### Kohlepassage-Test: Methode zur Messung der gastrointestinalen Transitgeschwindigkeit bei der Maus

Versuchstiere: männliche NMRI Mäuse (Züchter: Charles River, Sulzfeld, Deutschland), Körpergewicht: 30 - 35 g; die Tiere werden in Standardkäfigen (Typ IV Makrolone-Käfige, Fa. Ebeco, Castrop-Rauxel, Deutschland) besetzt mit jeweils maximal 18 Tieren unter einem 12:12h Licht-Dunkel Rhythmus mit Futter und Leitungswasser ad libitum gehalten.

Versuchsbeschreibung: Vor dem Versuch werden die Tiere 20 -24 h auf Drahtrost-Käfigeinlagen nüchtern gehalten.Als Markersubstanz der intestinalen Passagestrecke wird den Tiere eine Aktivkohlesuspension (10% Aktivkohle in 0,5% CMC Lösung; Applikationsvolumen: 0,1 ml/10g Körpergewicht) oral appliziert. Dannach wird die jeweilige Testsubstanz oder eine Vehikellösung intravenös appliziert. Zwei Stunden nach Applikation der Aktivkohlesuspension werden die Tiere durch CO₂-Begasung getötet. Anschließend wird der Intestinaltrakt vom Magen bis einschließlich Caecum entnommen und auf einer Glasplatte, die mit 0.9 % iger NaCl - Lösung benetzt wurde, ausgestreckt. Dann wird unmittelbar der Abstand Pylorus - Caecum, sowie die Passagestrecke der Kohlesuspension (weitester Punkt) gemessen.

Versuchsauswertung: Zur Bestimmung der relativen Hemmung des gastrointestinal Transits wird der Quotient Passagestrecke der Kohlesuspension (in cm)/Abstand Pylorus - Caecum (in cm) gebildet die Angabe erfolgt in %-Hemmung. Zur statistischen Auswertung werden die Messwerte nach Substanzgabe und die zeitgleichen Messwerte nach Vehikelapplikation mittels Ein-Faktor Varianzanalyse (one-way ANOVA) sowie einer post hoc Analyse nach Dunnett verglichen. Das Signifikanzniveau wurde auf p < 0.05 gesetzt. Die Gruppengrößen betragen üblicherweise n=10.

### Rota-Rod Test: Methode zur Untersuchung der Motorkoordination bei der Maus

Versuchstiere: männliche CD-1 Mäuse (Züchter: Charles River, Sulzfeld, Deutschland); Körpergewicht: 18 - 25 g; die Tiere werden in Standardkäfigen (Typ IV Makrolone-Käfige, Fa. Ebeco, Castrop-Rauxel, Deutschland) besetzt mit jeweils maximal 18 Tieren unter einem 12:12h Licht-Dunkel Rhythmus mit Futter und Leitungswasser ad libitum gehalten.

Methodenbeschreibung: Zur Beschreibung der Methode siehe: Kuribara H., Higuchi Y., Tadokoro S. (1977), Effects of central depressants on Rota-Rod and traction performance in mice. Japan. J. Pharmacol. 27, 117-126.

Statistische Auswertung: Zur statistischen Auswertung werden die Messwerte nach Substanzgabe und die zeitgleichen Messwerte nach Vehikelapplikation mittels Ein-Faktor Varianzanalyse (one-way ANOVA) sowie einer post hoc Analyse nach Dunnett verglichen. Das Signifikanzniveau wurde auf p < 0.05 gesetzt. Die Gruppengrößen betragen üblicherweise n=10.

### Jumping-Test: Methode zur Untersuchung des körperlichen Abhängigkeitspotentials an der Maus

Versuchstiere: männliche NMRI Mäuse (Züchter: Charles River, Sulzfeld, Deutschland); Körpergewicht: 20 - 24 g; die Tiere werden in Standardkäfigen (Typ III Makrolone-Käfige, Fa. Ebeco, Castrop-Rauxel, Deutschland) besetzt mit jeweils maximal 6 Tieren unter einem 12:12h Licht-Dunkel Rhythmus mit Futter und Leitungswasser ad libitum gehalten.

Methodenbeschreibung: Die Testsubstanzen werden insgesamt 7x über zwei Tage intraperitoneal appliziert. 5 Applikationen erfolgen am ersten Tag um 9:00, 10:00, 11:00, 13:00 und 15:00 Uhr und am zweiten Tag um 9:00 und 11:00 Uhr. Die ersten 3 Applikationen werden in aufsteigenden Dosierungen (Dosierungsschema) gegeben und dann weiter in der Dosierung der Dritten. Der Entzug wird 2 Stunden nach der letzten Substanzaplikation mit Naloxon 30 mg/kg (i.p.) präzipititert. Unmittelbar danach werden die Tiere einzeln in durchsichtige Beobachtungsboxen (Höhe 40 cm, Durchmesser 15 cm) gesetzt und die Sprungreaktionen über 15 Minuten in jeweils 5-Minuten- Perioden gezählt. Morphin wird in einer Dosierung als Vergleich/ Standard mitgeführt. Die Quantifizierung des Entzugs erfolgt über die Anzahl der Sprünge 0 bis 10 min. nach Naloxonapplikation. Die Anzahl der Tiere pro Gruppe mit mehr als 10 Sprüngen/ 10 min wird bestimmt und als "% positive Tiere" dokumentiert. Außerdem wird die durchschnittliche Sprungfrequenz in der Gruppe errechnet.

Statistische Auswertung: Die Auswertung der experimentellen Befunde im Hinblick auf statistisch signifikante Unterschiede zwischen den jeweiligen Dosisgruppen und der Vehikel-Kontrollgruppen erfolgt bevorzugt mittels Fisher's exact Test für den Parameter "% positive Tiere" sowie mittels Kruskal-Wallis Test für den Parameter "Sprungfrequenz" bevorzugt wie im experimentellen Teil beschrieben. Dabei wird das Signifikanzniveau jeweils auf p < 0.05 festgelegt. Die Gruppengrößen betragen üblicherweise n=12.

### Referenz: Saelens JK, Arch Int Pharmacodyn 190: 213-218, 1971

### Konditionierte Platzpräferenz: Methode zur Untersuchung der möglichen Induktion einer psychischen Abhängigkeit/Sucht an der Ratte

Methodenbeschreibung: Zur Untersuchung der Platzpräferenz siehe: Tzschentke, T.M., Bruckmann, W. and Friderichs, F. (2002) Lack of sensitization during place conditioning in rats is consistent with the low abuse potential of tramadol. Neuroscience Letters 329, 25-28.

Statistische Auswertung: Die Auswertung der experimentellen Befunde im Hinblick auf statistisch signifikante Unterschiede in der Präferenz der Tiere für den Wirkstoff bzw. das Vehikel erfolgt bevorzugt mittels gepaartem t-Test Dabei wird das Signifikanzniveau auf p < 0.05 festgelegt. Die Gruppengrößen betragen üblicherweise n=8.

**Tabelle 1a: Zusammenfassung der pharmakologischen Daten zu Beispiel AMD-6^{cis}**

| **Testsystem** | **Messparameter** | **Befund¹** | **Abstandsfaktor** |
|---|---|---|---|
| Bindung ORL1-Rezeptor | Bindungsaffinität | Ki = 0,030 µM | --- |
| Bindung µ-Opioidrezeptor | Bindungsaffinität | Ki = 0,138 µM | --- |
| Chung, Ratte | Hemmung des neuropathischen Schmerzes bei Mononeuropathie (Trennung von anti-allodynischer und anti-nociceptiver Wirkung) | ED₅₀ = 9 µg/kg i.v.; bis zur höchsten Testdosis (21.5 µg/kg i.v.): Keine anti-nociceptive Wirkung im gesunden Gewebe. | --- |
| Bennett, Ratte | Hemmung des neuropathischen Schmerzes bei Mononeuropathie | ED₅₀ = 7 µg/kg i.v. | --- |
| STZ, Ratte | Hemmung des neuropathischen Schmerzes bei diabetischer Polyneuropathie | ED₅₀ ca. 1 µg/kg i.v.; bis zur höchsten Testdosis (10 µg/kg i.v.): Keine anti-nociceptive Wirkung in nicht-neuropathischen Kontrolltieren. | --- |
| Tail-flick, Ratte | Hemmung von Akutschmerz (nociceptiver Schmerz) | NOEL: 1 mg/kg i.v. bzw. 4,64 mg/kg i.v. bei reduzierter Brennstrahlintensität | 220 - 1000x |
| Blutgasanalyse, Ratte | Atemdepression gemessen als Anstieg des arteriellen pCO₂ und Abfall des arteriellen pO₂ | NOEL: 1 mg/kg i.v. | 220x |
| Herz-Kreislauf, Kaninchen | Arterieller Blutdruck und Herzfrequenz | NOEL: 1 mg/kg i.v. | 220x |
| Kohlepassage, Maus | Gastrointestinaler Transit | NOEL: 3 mg/kg i.v. | 660x |
| RotaRod- Test, Maus | Motorkoordination | NOEL: ≥10 mg/kg i.v. | >2200x |
| Jumping-Test, Maus | Körperlicher Abhängigkeit / Entzugssymptome | NOEL: 10 mg/kg i.p. | 2200x |
| Platzpräferenz, Ratte | Psychische Abhängigkeit | NOEL: ≥13,8 mg/kg i.p. | >3000x |

| | | | |
|---|---|---|---|
| ¹) NOEL (= No Observed Effect Level) bezeichnet die obere befundfreie Dosis (d.h. Dosis ohne signifikanten Effekt) ²) Die Abstandsfaktoren wurden berechnet als Quotient aus NOEL und einer mittleren ED₅₀ⁿ aus den Neuropathiemodellen (hier: 4,5 µg/kg) | | | |

**Tabelle 1b: Zusammenfassung der pharmakologischen Daten zu Beispiel AMD-7^{cis}**

| **Testsystem** | **Messparameter** | **Befund¹** | **Abstandsfaktor²** |
|---|---|---|---|
| Bindung ORL1-Rezeptor | Bindungsaffinität | Ki = 0,070 µM | --- |
| Bindung µ-Opioidrezeptor | Bindungsaffinität | Ki = 0,450 µM | --- |
| Chung, Ratte | Hemmung des neuropathischen Schmerzes bei Mononeuropathie (Trennung von anti-allodynischer und anti-nociceptiver Wirkung) | ED₅₀ = 88 µg/kg i.v.; keine anti-nociceptive Wirkung im gesunden Gewebe. (Testdosis: 100 µg/kg i.v.) | --- |
| STZ, Maus | Hemmung des neuropathischen Schmerzes bei diabetischer Polyneuropathie | 68% MPE bei 100 µg/kg i.p.; keine anti-nociceptive Wirkung in nicht-neuropathischen Kontrolltieren. | --- |
| Tail-flick, Ratte | Hemmung von Akutschmerz (nociceptiver Schmerz) | NOEL: ≥10 mg/kg i.v. | >110x |
| Blutgasanalyse, Ratte | Atemdepression gemessen als Anstieg des arteriellen pCO₂ und Abfall des arteriellen pO₂ | NOEL: 1 mg/kg i.v. | 11x |
| Herz-Kreislauf, Kaninchen | Arterieller Blutdruck und Herzfrequenz | NOEL: ≥3 mg/kg i.v. | >34x |
| Kohlepassage, Maus | Gastrointestinaler Transit | NOEL: 1 mg/kg i.v. | 11x |
| RotaRod-Test, Maus | Motorkoordination | NOEL: 10 mg/kg i.v. | 110x |
| Jumping-Test, Maus | Körperlicher Abhängigkeit / Entzugssymptome | NOEL: ≥10 mg/kg i.p. | >110x |
| Platzpräferenz, Ratte | Psychische Abhängigkeit | NOEL: ≥20 mg/kg i.p. | >220x |

| | | | |
|---|---|---|---|
| ¹⁾ MPE (= Maximum Possible Effect) bezeichnet die maximal mögliche Effektgröße; NOEL (= No Observed Effect Level) bezeichnet die obere befundfreie Dosis (d.h. Dosis ohne signifikanten Effekt) ²⁾ Die Abstandsfaktoren wurden berechnet als Quotient aus NOEL und einer mittleren ED₅₀ⁿ aus den Neuropathiemodellen (hier: 88 pg/kg) | | | |

**Fazit:** Zur Veranschaulichung der überraschenden pharmakologischen Eigenschaften der erfindungsgemäß verwendeten Verbindungen wurden das Beispiel **AMD-6^{cis}** ausgewählt. Es handelt sich um hochaffine ORL1-Rezeptor- und µ-Opioidrezeptor-Liganden mit einem Verhältnis ORL1-Rezeptor-Affinität zu µ-Opioidrezeptor-Affinität von ca. 5 bzw. ca. 6. Das Beispiel **AMD-6^{cis}** belegt, dass die erfindungsgemäß verwendeten Verbindungen eine sehr hohe Wirksamkeit gegen neuropathischen Schmerz haben (hier: ED₅₀ⁿ zwischen 1 und 10 µg/kg i.v. bzw. 88 µg/kg i.v.). Dagegen wird im Akutschmerzmodell selbst bei Dosierungen, die 100 bis 1000 fach höher waren als die im Neuropathiemodell wirksamen Dosierungen, keine signifikante anti-nociceptive Wirkung beobachtet. Ebenso werden in Tiermodellen zur Untersuchung von Nebenwirkungen bei 11 bis mehr als 3000 fach höheren Dosierungen keine signifikanten opioidtypischen Nebenwirkungen (wie Atemdepression, Reduktion von Blutdruck und Herzfrequenz, Obstipation, zentralnervöse Effekte, körperliche Abhängigkeit, psychische Abhängigkeit/Sucht) beobachtet.

**Tabelle 2: Übersicht über ausgewählte pharmakologische bzw. pharmakokinetische Charakteristika weiterer Beispiele**

| **Verbindung** | **Ki (ORL₁) [µM]** | **Ki (µ) [µM]** | **Chung, Ratte** | **Tail-flick, Ratte** | **Blutgasanalyse, Ratte** | **Kohlepassage, Maus** | **RotaRod, Maus** | **t_{1/2}, Ratte, 100 µg/kg, i.v. // pharmakodynamische Wirkdauer (Dosis)** |
|---|---|---|---|---|---|---|---|---|
| **AMD-6*^{cis}*** | 0,030 | 0,138 | *ED₅₀* = 9 µg/kg i.v. | NOEL² = 1000 µg/kg i.v. | NOEL = 1000 µg/kg i.v. | NOEL = 3000 µg/kg i.v. | NOEL: ≥10000 µg/kg i.v. | 8 h // » 5 h (10 µg/kg i.v.) |
| **AMD-1*^{cis}*** | 0,018 | 0,032 | 18%MPE bei 100 µg/kg i.v. | NOEL > 100 µg/kg i.v. | NOEL = 1000 µg/kg i.v. | *Nicht durchgeführt* | *Nicht durchgeführt* | *nicht bestimmt* // *nicht bestimmt* |
| **AM D-2*^{cis}*** | 0,017 | 0,05 | 35%MPE bei 100 µg/kg i.v. | NOEL > 1000 µg/kg i.v. | *Nicht durchgeführt* | NOEL = 4600 µg/kg i.v. | *Nicht durchgeführt* | *nicht bestimmt* // ca. 3 h (100 µg/kg i.v.) |
| **AMD-3*^{cis}*** | 0,016 | 0,059 | 42%MPE bei 100 µg/kg i.v. | NOEL > 1000 µg/kg i.v. | *Nicht durchgeführt* | NOEL = 3000 µg/kg i.v. | NOEL: ≥10000 µg/kg i.v. | *nicht bestimmt* // ca. 3 h (100 µg/kg i.v.) |
| **AMD-4*^{cis}*** | 0,003 | 0,009 | 20%MPE bei 100 µg/kg i.v. | NOEL > 100 µg/kg i.v. | NOEL = 300 µg/kg i.v. | *Nicht durchgeführt* | *Nicht durchgeführt* | *nicht bestimmt* // 1 - 3 h (100 µg/kg i.v.) |
| **AMD-7*^{cis}*** | 0,070 | 0,450 | *ED₅₀ =* 88 µg/kg i.v. | NOEL ≥ 10000 µg/kg i.v. | NOEL = 1000 µg/kg i.v. | NOEL = 1000 µg/kg i.v. | NOEL = 10000 µg/kg i.v. | 3 h // ca. 3 h (100 µg/kg i.v.) |

| | | | | | | | | |
|---|---|---|---|---|---|---|---|---|
| ¹⁾ ORL1/µ-Affinitätsverhältnis definiert als 1/[K_{i(ORL1)}/K_{i(µ)}] ²⁾ NOEL (= No Observed Effect Level) bezeichnet die obere befundfreie Dosis (d.h. Dosis ohne signifikanten Effekt) | | | | | | | | |

**Fazit:** Die erfindungsgemäß verwendeten Verbindungen zeigen eine sehr gute Wirksamkeit gegen neuropathischen Schmerz. Überraschenderweise wurde dagegen im Akutschmerzmodell selbst bei Dosierungen, die ca. 10 fach bis mehr als 100 fach höher waren als die im Neuropathiemodell wirksamen Dosierungen, keine signifikante anti-nociceptive Wirkung beobachtet. Ebenso wurden überraschenderweise in Nebenwirkungs-Tiermodellen (z.B. Blutgasanalyse, gastrointestinale Kohlepassage und RotaRod Test) bei 10 fach bis mehr als 300 fach höheren Dosierungen keine signifikanten opioidtypischen Nebenwirkungen beobachtet.

**Tabelle 3: Vergleich von cis- und trans-Spiroamin**

| **Verbindung** | **Ki (ORL₁) [µM]** | **Ki (µ) [µM]** | **Chung, Ratte** | **Tail-flick, Ratte** | **Blutgasanalyse, Ratte** |
|---|---|---|---|---|---|
| **AMD-6*^{cis}*** | 0,030 | 0,138 | *ED₅₀* = 9 µg/kg i.v. | NOEL ² = 1000 µg/kg i.v. | NOEL = 1000 µg/kg i.v. |
| **AMD-6*^{trans}*** | 0,002 | 0,008 | NOEL ≥ 100 µg/kg i.v | *ED₅₀* = 640 µg/kg i.v. | NOEL = 300 µg/kg i.v. |
| **AMD-7^{cis}** | 0,070 | 0,450 | *ED₅₀ =* 88 µg/kg i.v. | NOEL² ≥ 10000 µg/kg i.v. | NOEL = 1000 µg/kg i.v. |
| **AMD-7^{trans}** | 0.001 | 0.001 | *Nicht durchgeführt* | 54%MPE bei 31.6 µg/kg i.v. | *Nicht durchgeführt* |
| **AMN-2*^{cis}*** | 0,012 | 0,031 | *ED₅₀* = 895 µg/kg i.v. | NOEL = 1000 µg/kg i.v. | *Nicht durchgeführt* |
| **AMN-2*^{trans}*** | 0,0004 | 0,0005 | 27%MPE bei 30 µg/kg i.v. | 60%MPE bei 100 µg/kg i.v. | *Nicht durchgeführt* |

| | | | | | |
|---|---|---|---|---|---|
| ¹⁾ ORL1/µ-Affinitätsverhältnis definiert als 1/[K_{i(ORL1)}/K_{i(µ)}] ²⁾ NOEL (= No Observed Effect Level) bezeichnet die obere befundfreie Dosis (d.h. Dosis ohne signifikanten Effekt) | | | | | |

**Fazit:** Überraschenderweise zeigen nur die cis-Spiroamine gemäß der allgemeinen Formel (I) (hier Beispiel **AMD-6^{cis}** und Vergleichsbeispiel **AMN-2^{cis})** gute Wirksamkeit gegen neuropathischen Schmerz bei gleichzeitig fehlender antinociceptiver Wirkung im Akutschmerz. Ebenso werden in den Nebenwirkungs-Tiermodellen (als Beispiel hier Blutgasanalyse) bei um ein Vielfaches höheren Dosierungen keine signifikanten opioidtypischen Nebenwirkungen beobachtet. Die jeweiligen trans-Spiroamine (hier Vergleichsbeispiel **AMD-6^{trans}** und Vergleichsbeispiel **AMN-2^{trans})** zeigen dagegen keinen Abstand zwischen Dosierungen, die gegen neuropathischen Schmerz bzw. gegen Akutschmerz wirksam sind. Ebenso wird kein Abstand zu Dosierungen bei denen opioidtypische Nebenwirkungen auftreten beobachtet (als Beispiel hier Blutgasanalyse). Dabei zeigen **AMD-5^{cis}** bzw. **AMD-6^{cis}** im Gesamtvergleich die größten Abstände bei höchstmöglicher analgetischer Wirkung.

**Tabelle 4: Vergleich von cis-Spiroaminen und cis-Spiroethern**

| **Verbindung** | **Ki(ORL₁)[µM]** | **Ki(µ)[µM]** | **Chung, Ratte** | **Tail-flick, Ratte bzw. Maus*** |
|---|---|---|---|---|
| **AMN-2*^{cis}*** | 0,012 | 0,031 | *ED₅₀* = 895 µg/kg i.v. | NOEL ² = 1000 µg/kg i.v. |
| **Ether-2*^{cis}*** | 0,031 | 0,092 | 17%MPE bei 100 µg/kg i.v | 78%MPE bei 1000 µg/kg i.V.* |
| **Ether-1 *^{cis}*** | 0,06 | 0,12 | 28%MPE bei 100 µg/kg i.v | 33%MPE bei 1000 µg/kg i.v. |

| | | | | |
|---|---|---|---|---|
| ¹⁾ ORL1/µ-Affinitätsverhältnis definiert als 1/[K_{i(ORL1)}/K_{i(µ)}] ²⁾ NOEL (= No Observed Effect Level) bezeichnet die obere befundfreie Dosis (d.h. Dosis ohne signifikanten Effekt) | | | | |

**Fazit:** Überraschenderweise zeigen bei erfindungsgemäßer Verwendung nur die cis-Spiroamine (hier Beispiel **AMN-2^{cis}**) gute Wirksamkeit gegen neuropathischen Schmerz bei gleichzeitig fehlender antinociceptiver Wirkung im Akutschmerz. Ebenso werden in den Nebenwirkungs-Tiermodellen (als Beispiel hier Blutgasanalyse) bei um ein Vielfaches höheren Dosierungen keine opioidtypischen Nebenwirkungen beobachtet. Cis-Spiroether (hier Vergleichsbeispiel **Ether-2^{cis}** und Vergleichsbeispiel **Ether-1^{cis}**) zeigen dagegen keine deutlichen Abstände zwischen Dosierungen, die gegen neuropathischen Schmerz bzw. gegen Akutschmerz wirksam sind.

**Tabelle 5: Vergleich von AMD-5^{cis} (freie Base) und AMD-6^{cis} (Citrat-Salz)**

| **Verbindung** | **Ki (ORL₁) [µM]** | **Ki(µ) [µM]** | **Chung, Ratte** | **Tail-flick, Ratte** |
|---|---|---|---|---|
| **AMD-5*^{cis}*** | 0,030 | 0,138 | *ED₅₀* = *17 µg*/*kg i.v.* | NOEL² = 30000 µg/kg i.p. |
| **AMD-6*^{cis}*** | 0,020 | 0,117 | *ED₅₀* = *9 µg*/*kg i.v.* | NOEL > 10000 µg/kg i.v. |

| | | | | |
|---|---|---|---|---|
| ¹⁾ ORL1/µ-Affinitätsverhältnis definiert als 1/[K_{i(ORL1)}/K_{i(µ)}] ²⁾ NOEL (= No Observed Effect Level) bezeichnet die obere befundfreie Dosis (d.h. Dosis ohne signifikanten Effekt) | | | | |

**Fazit:** Ein Vergleich von **AMD-5^{cis}** (freie Base) und **AMD-6^{cis}** (Citrat-Salz) ergab keine relevanten Unterschiede in pharmakologischen Eigenschaften von Base und Salz.

**Tabelle 6: Vergleich der Affinitäten gegenüber einzelnen Rezeptoren**

| | **ORL1** | | **µ-Opioid** | | **k-Opioid** | | **d-Opioid** | | **Schmerz, Ratte** | |
|---|---|---|---|---|---|---|---|---|---|---|
| | | | | | | | | | **Akut (tail flick)** | **neuropathisch (SNL [Chung])** |
| | Ki * | EC₅₀ ** | Ki | EC₅₀ | Ki | EC₅₀ | Ki | EC₅₀ | ED₅₀ rat [µg/kg] %MPE (@µg/kg) | |
| **AMD-3^{cis}** | 16 | 102/ 92% | 59 | 1112/ 82% | 160 | 874/ 42% | 6,7 | 41 / 92% | 0% (1000) 73% (10000) | 106 |
| **AMD-3^{trans}** | 14 | 16/ 81% | 12 | 13 / 66% | 49 | - / 55% | 8 | - / 87% | 0% (1000) | 20% (100) |
| **AMD-5^{cis}** | 30 | 76 / 106% | 138 | 300/ 63% | 768 | 1035 / 30% | 38 | 463 / 78% | 0% (1000) 58% (10000) | 9,2 |
| **AMD-6^{trans}** | 3 | 47 / 104% | 8 | 79 / 97% | 19 | 59 / 88% | 6 | 19 / 126% | 640 | 400 |
| **AMD-7^{cis}** | 70 | 50 / 90% | 450 | 49 / 94% | 542 | 1170 / 85 % | 791 | 2684 / 106 % | 0 % (10000) | 88 |
| **AMD-7^{trans}** | 1 | 16 / 90% | 1 | 3 / 88% | 4 | 29 / 64 % | 1 | 5/82 % | 54 % (31.6) | nicht durchgeführt |
| | * Radio-binding assay - Ki in nM | | | | | | | | | |
| | ** GTPgammaS assay - EC50 in nM and relative efficacy in % | | | | | | | | | |

| | | | | | | | | | | |
|---|---|---|---|---|---|---|---|---|---|---|
| Ki [nM] EC₅₀ [eff. %] | | | | | | | | | | |

In einem weiteren Aspekt betrifft die Erfindung Verbindung der allgemeinen Formel (III), 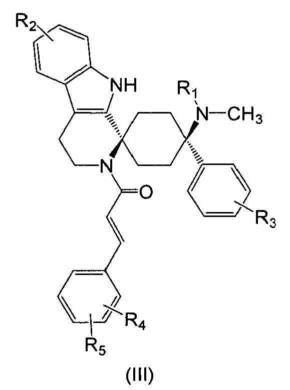 wobei die Verbindung als Hydrochlorid-, Citrat- oder Hemicitratsalz vorliegt.

In einer weiteren bevorzugten Ausführungsform der erfindungsgemäßen Verbindungen ist R₂ -H und/oder R₃ -F.

In einer weiteren bevorzugten Ausführungsform der erfindungsgemäßen Verbindungen sind R₄ und R₅ entweder beide -H oder beide -OCH₃ sind.

In einer weiteren bevorzugten Ausführungsform der erfindungsgemäßen Verbindungen sind diese ausgewählt aus der Gruppe bestehend aus
- (E)-1-((1s,4s)-4-(Dimethylamino)-4-phenyl-3',4'-dihydrospiro[cyclohexan-1,1'-spiro[cyclohexan-1,1'-pyrido[3,4-b]indol]-2'(9'H)-yl)methanon;
- (E)-1-((1s,4s)-4-(Dimethylamino)-4-(3-fluorophenyl)-3',4'-dihydrospiro[cyclohexan-1.1'-pyrido[3.4-b]indol]-2'(9'H)-yl)-3-phenylprop-2-en-1-on;
- (E)-1-((1s,4s)-4-(Dimethylamino)-6'-fluoro-4-(3-fluorophenyl)-3',4'-dihydrospiro-[cyclohexan-1,1'-pyrido[3,4-b]indol]-2'(9'H)-yl)-3-phenylprop-2-en-1-on;
- (E)-1-((1s,4s)-4-(Dimethylamino)-6'-fluoro-4-phenyl-3',4'-dihydrospiro[cyclohexan-1,1'-pyrido[3,4-b]indol]-2'(9'H)-yl)-3-phenylprop-2-en-1-on;
- (E)-1-((1s,4s)-4-(Dimethylamino)-4-(4-fluorophenyl)-3',4'-dihydrospiro[cyclohexan-11'-pyrido[3,4-b]indol]-2'(9'H)-yl)-3-phenylprop-2-en-1-on. jeweils in der Form als Hydrochlorid-, Citrat- oder Hemicitratsalz.

In einer weiteren bevorzugten Ausführungsform der Erfindung sind die erfindungsgemäßen Verbindungen Arzneistoffe.

Eine weitere bevorzugte Ausführungsform der Erfindung betrifft eine pharmazeutische Zusammensetzung, welche einen physiologisch verträglichen Träger und eine der erfindungsgemäßen Verbindungen enthält.

In einer bevorzugten Ausführungsform der erfindungsgemäßen pharmazeutischen Zusammensetzung ist diese:
- fest, flüssig oder pastös; und/oder
- enthält die erfindungsgemäße Verbindung in einer Menge von 0,001 bis 99 Gew.-% enthält, bezogen auf das Gesamtgewicht der Zusammensetzung.

Eine weitere bevorzugte Ausführungsform der Erfindung betrifft eine pharmazeutische Darreichungsform, welche eine der oben beschriebenen pharmazeutischen Zusammensetzungen enthält.
In einer weiteren Ausführungsform der Erfindung ist die oben genannte Darreichungsform konfektioniert zur höchstens einmal täglichen Verabreichung.
In einer weiteren bevorzugten Ausführungsform der Erfindung ist die erfindungsgemäße Darreichungsform zur systemischen Verabreichung konfektioniert.
In einer weiteren bevorzuten Ausführungsform der Erfindung ist die erfindungsgemäße Darreichungsform zur oralen Verabreichung konfektioniert.

In einer weiteren bevorzugten Ausführungsform der Erfindung enthält die erfindungsgemäße Darreichungsform eine der erfindungsgemäßen Verbindungen in einer Dosis im Bereich von 1,0 µg bis 10 mg enthält, bezogen auf das Molekulargewicht der freien Base.

## Patentansprüche

1. Verbindung der allgemeinen Formel (III), 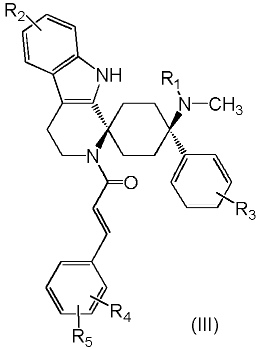 worin
R₁ -H oder CH₃ ist;
R₂ -H oder -Halogen ist;
R₃ -H oder -Halogen ist;
R₄ -H, -Halogen oder -OC₁₋₃-Alkyl ist;
R₅ -H, -Halogen oder -OC₁₋₃-Alkyl ist; und
wobei die Verbindung als Hydrochlorid-, Citrat- oder Hemicitratsalz vorliegt.

2. Verbindung nach Anspruch 1, wobei R₂ -H und/oder R₃ -F ist.

3. Verbindung nach Anspruch 1 oder 2, wobei R₄ und R₅ entweder beide -H oder beide - OCH₃ sind.

4. Verbindung nach einem der vorstehenden Ansprüche, welche ausgewählt ist aus der Gruppe bestehend aus
• (E)-1-((1s,4s)-4-(Dimethylamino)-4-phenyl-3',4'-dihydrospiro[cyclohexan-1,1'-pyrido[3,4-b]indol]-2'(9'H)-yl)-3-phenylprop-2-en-1-on;
• (E)-1-((1s,4s)-4-(Dimethylamino)-4-(3-fluorophenyl)-3',4'-dihydrospiro[cyclohexan-1,1'-pyrido[3,4-b]indol]-2'(9'H)-yl)-3-phenylprop-2-en-1-on;
• (E)-1-((1s,4s)-4-(Dimethylamino)-6'-fluoro-4-(3-fluorophenyl)-3',4'-dihydrospiro-[cyclohexan-1,1'-pyrido[3,4-b]indol]-2'(9'H)-yl)-3-phenylprop-2-en-1-on;
• (E)-1-((1s,4s)-4-(Dimethylamino)-6'-fluoro-4-phenyl-3',4'-dihydrospiro[cyclohexan-1,1'-pyrido[3,4-b]indol]-2'(9'H)-yl)-3-phenylprop-2-en-1-on;
• (E)-1-((1s,4s)-4-(Dimethylamino)-4-(4-fluorophenyl)-3',4'-dihydrospiro[cyclohexan-1,1'-pyrido[3,4-b]indol]-2'(9'H)-yl)-3-phenylprop-2-en-1-on,
jeweils in der Form als Hydrochlorid-, Citrat- oder Hemicitratsalz.

5. Verbindung gemäß einem der Ansprüche 1 bis 4 mit der Struktur 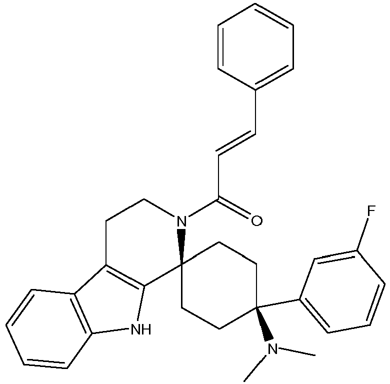 in Form des Hydrochlorid-, Citrat- oder Hemicitratsalzes.

6. Verbindung nach einem der Ansprüche 1 bis 4 zur Verwendung als Arzneistoff.

7. Pharmazeutische Zusammensetzung, welche einen physiologisch verträglichen Träger und eine Verbindung nach einem der Ansprüche 1 bis 5 enthält.

8. Zusammensetzung nach Anspruch 7, welche
- fest, flüssig oder pastös ist; und/oder
- die Verbindung nach einem der Ansprüche 1 bis 5 in einer Menge von 0,001 bis 99 Gew.-% enthält, bezogen auf das Gesamtgewicht der Zusammensetzung.

9. Pharmazeutische Darreichungsform, welche die pharmazeutische Zusammensetzung nach Anspruch 7 oder 8 enthält.

10. Darreichungsform nach Anspruch 9, welche zur höchstens einmal täglichen Verabreichung konfektioniert ist.

11. Darreichungsform nach Anspruch 9 oder 10, welche zur systemischen Verabreichung konfektioniert ist.

12. Darreichungsform nach Anspruch 11, welche zur oralen Verabreichung konfektioniert ist.

13. Darreichungsform nach Anspruch 12, welche die Verbindung nach einem der Ansprüche 1 bis 5 in einer Dosis im Bereich von 1,0 µg bis 10 mg enthält, bezogen auf das Molekulargewicht der freien Base.

14. Verbindung nach einem der Ansprüche 1 bis 5 zur Anwendung bei der Behandlung von neuropathischem und/oder chronischem Schmerz.

15. Verbindung zur Anwendung nach Anspruch 14, wobei die Anwendung höchstens einmal täglich erfolgt.

## Claims

1. Compound of the general formula (III), 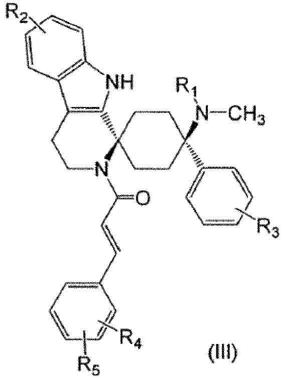 in which
R₁ is -H or CH₃;
R₂ is -H or -halogen;
R₃ is -H or -halogen;
R₄ is -H, -halogen or -OC₁₋₃-alkyl; and
R₅ is -H, -halogen or -OC₁₋₃-alkyl;
wherein the compound is present as hydrochloride, citrate or hemi-citrate salt.

2. Compound according to claim 1, wherein R₂ is -H and/or R₃ is -F.

3. Compound according to claim 1 or 2, wherein R₄ and R₅ are either both -H or both- OCH₃.

4. Compound according to one of the above claims, selected from the group consisting of
• (E)-1-((1s,4s)-4-(dimethylamino)-4-phenyl-3',4'-dihydrospiro[cyclohexane-1, 1'-pyrido[3,4-b]indole]-2'(9'H)-yl)-3-phenylprop-2-en-1-one;
• (E)-1-((1s,4s)-4-(dimethylamino)-4-(3-fluorophenyl)-3',4'-dihydrospiro[cyclohexane-1,1'-pyrido[3,4-b]indole]-2'(9'H)-yl)-3-phenylprop-2-en-1-one;
• (E)-1-((1s,4s)-4-(dimethylamino)-6'-fluoro-4-(3-fluorophenyl)-3',4'-dihydrospiro-[cyclohexane-1,1'-pyrido[3,4-b]indole]-2'(9'H)-yl)-3-phenylprop-2-en-1-one;
• (E)-1-((1s,4s)-4-(dimethylamino)-6'-fluoro-4-phenyl-3',4''-dihydrospiro[cyclohexane-1,1'-pyrido[3,4-b]indole]-2'(9'H)-yl)-3-phenylprop-2-en-1-one;
• (E)-1-((1s,4s)-4-(dimethylamino)-4-(4-fluorophenyl)-3',4'-dihydrospiro[cyclohexane-1,1'-pyrido[3,4-b]indole]-2'(9'H)-yl)-3-phenylprop-2-en-1-one,
in each case in the form of hydrochloride, citrate or hemi-citrate salt.

5. Compound according to one of claims 1 to 4 with the structure 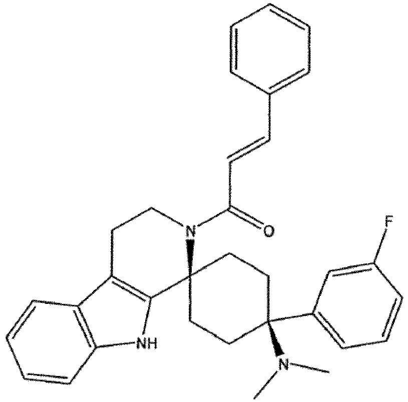 in the form of the hydrochloride, citrate or hemi-citrate salt.

6. Compound according to one of claims 1 to 4 for use as a medicament.

7. Pharmaceutical composition which contains a physiologically acceptable carrier and a compound according to one of claims 1 to 5.

8. Composition according to claim 7, which
- is solid, liquid or pasty; and/or
- contains the compound according to one of claims 1 to 5 in an amount from 0.001 to 99 wt. % relative to the total weight of the composition.

9. Pharmaceutical dosage form which contains the pharmaceutical composition according to claim 7 or 8.

10. Dosage form according to claim 9, which is prepared for administration no more than once daily.

11. Dosage form according to claim 9 or 10, which is prepared for systemic administration.

12. Dosage form according to claim 11, which is prepared for oral administration.

13. Dosage form according to claim 12, which contains the compound according to one of claims 1 to 5 in a dose within the range of 1.0 µg to 10 mg relative to the molecular weight of the free base.

14. Compound according to one of claims 1 to 5 for application in the treatment of neuropathic and/or chronic pain.

15. Compound for application according to claim 14, with application being effected no more than once daily.

## Revendications

1. Composé de formule générale (III) 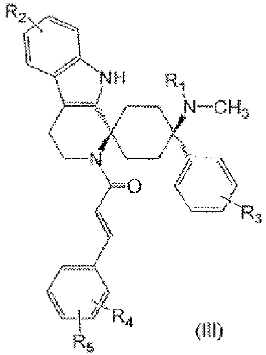 dans laquelle :
R₁ est -H ou un groupe CH₃ ;
R₂ est -H ou un groupe -halogéno ;
R₃ est -H ou un groupe -halogéno ;
R₄ est -H, un groupe -halogéno ou -O(alkyle en C₁ à C₃) ; et
R₅ est -H, un groupe -halogéno ou -O(alkyle en C₁ à C₃) ;
le composé étant présent en tant que sel chlorhydrate, sel citrate ou sel hémicitrate.

2. Composé selon la revendication 1, dans lequel R₂ est -H et/ou R₃ est -F.

3. Composé selon la revendication 1 ou 2, dans lequel R₄ et R₅ représentent soit tous les deux -H, soit tous les deux un groupe -OCH₃.

4. Composé selon l'une des revendications précédentes, qui est choisi dans le groupe constitué par les composés choisis dans le groupe constitué par :
• la (E)-1-((1s,4s)-4-(diméthylamino)-4-phényl-3',4'-dihydrospiro[cyclohexane-1,1'-pyrido[3,4-b]indole]-2'(9'H)-yl)-3-phénylprop-2-ène-1-one ;
• la (E)-1-((1s,4s)-4-(diméthylamino)-4-(3-fluorophényl)-3',4'-dihydrospiro[cyclohexane-1,1'-pyrido-[3,4-b]indole]-2' (9'H)-yl)-3-phénylprop-2-ène-1-one ;
• la (E)-1-((1s,4s)-4-(diméthylamino)-6'-fluoro-4-(3-fluorophényl)-3',4'-dihydrospiro[cyclohexane-1,1'-pyrido[3,4-b]indole]-2' (9'H)-yl)-3-phényl-prop-2-ène-1-one ;
• la (E)-1-((1s,4s)-4-(diméthylamino)-6'-fluoro-4-phényl-3',4'-dihydrospiro[cyclohexane-1,1'-pyrido-[3,4-b]indole]-2'(9'H)-yl)-3-phénylprop-2-ène-1-one ;
• la (E)-1-((1s,4s)-4-(diméthylamino)-4-(4-fluorophényl)-3',4'-dihydrospiro[cyclohexane-1,1'-pyrido-[3,4-b]indole]-2' (9'H)-yl)-3-phénylprop-2-ène-1-one ;
respectivement sous la forme d'un sel chlorhydrate, sel citrate ou sel hémicitrate.

5. Composé selon l'une des revendications 1 à 4, ayant la structure : 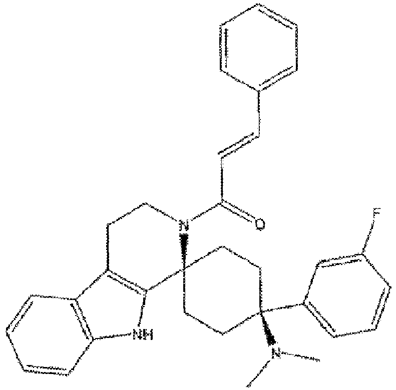 sous la forme du sel chlorhydrate, sel citrate ou sel hémicitrate.

6. Composé selon l'une des revendications 1 à 4, destiné à être utilisé en tant que médicament.

7. Composition pharmaceutique, qui contient un support physiologiquement compatible et un composé selon l'une des revendications 1 à 5.

8. Composition selon la revendication 7, qui :
- est solide, liquide ou pâteuse ; et/ou
- contient le composé selon l'une des revendications 1 à 5 en une quantité de 0,001 à 99 % en poids par rapport au poids total de la composition.

9. Forme galénique pharmaceutique, qui contient la composition pharmaceutique selon la revendication 7 ou 8.

10. Forme galénique selon la revendication 9, qui est formulée pour une administration au plus une fois par jour.

11. Forme galénique selon la revendication 9 ou 10, qui est formulée pour une administration systémique.

12. Forme galénique selon la revendication 11, qui est formulée pour une administration par voie orale.

13. Forme galénique selon la revendication 12, qui contient le composé selon l'une des revendications 1 à 5 selon une dose comprise dans la plage de 1,0 µg à 10 mg, par rapport à la masse moléculaire de la base libre.

14. Composé selon l'une des revendications 1 à 5, destiné à être utilisé lors du traitement d'une douleur neuropathique et/ou chronique.

15. Composé destiné à être utilisé selon la revendication 14, l'utilisation ayant lieu au plus une fois par jour.
